(19) **Europäisches Patentamt**
**European Patent Office**
**Office européen des brevets**

(11) **EP 3 107 556 B1**

(12) **FASCICULE DE BREVET EUROPEEN**

(45) Date de publication et mention de la délivrance du brevet:
**10.04.2019 Bulletin 2019/15**

(21) Numéro de dépôt: **15709276.8**

(22) Date de dépôt: **18.02.2015**

(51) Int Cl.:
*A61K 38/05* ^(2006.01)    *A61K 38/06* ^(2006.01)
*A61P 3/10* ^(2006.01)

(86) Numéro de dépôt international:
**PCT/FR2015/050397**

(87) Numéro de publication internationale:
**WO 2015/124867 (27.08.2015 Gazette 2015/34)**

(54) **COMPOSITIONS POUR LA PRÉVENTION ET/OU LE TRAITEMENT DE PATHOLOGIES LIÉES A L'ALPHA-GLUCOSIDASE**

ZUSAMMENSETZUNGEN ZUR VORBEUGUNG UND/ODER BEHANDLUNG PATHOLOGISCHER ZUSTÄNDE IM ZUSAMMENHANG MIT ALPHA-GLUCOSIDASE

COMPOSITIONS FOR PREVENTING AND/OR TREATING PATHOLOGICAL CONDITIONS ASSOCIATED WITH ALPHA-GLUCOSIDASE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.02.2014 FR 1451280**

(43) Date de publication de la demande:
**28.12.2016 Bulletin 2016/52**

(73) Titulaires:
• **Universite De La Rochelle**
  **17031 La Rochelle (FR)**
• **Université Blaise Pascal Clermont II**
  **63006 Clermont Ferrand Cedex 1 (FR)**

(72) Inventeurs:
• **MAUGARD, Thierry**
  **F-17220 La Jarne (FR)**
• **BORDENAVE-JUCHEREAU, Stéphanie**
  **F-17290 Le Thou (FR)**
• **PIOT, Jean-Marie**
  **F-17180 Perigny (FR)**
• **BEN-HENDA, Yesmine**
  **92700 Colombes (FR)**
• **SIRVENT, Pascal**
  **F-63122 Ceyrat (FR)**
• **PELTIER, Sébastien**
  **F-17450 Fouras (FR)**

(74) Mandataire: **Grosset-Fournier, Chantal Catherine et al**
  **Grosset-Fournier & Demachy**
  **54, rue Saint-Lazare**
  **75009 Paris (FR)**

(56) Documents cités:
**EP-A1- 1 661 909       EP-A1- 1 690 869**
**WO-A1-2006/084560      WO-A2-2006/005757**
**US-A1- 2004 229 934     US-A1- 2006 035 864**

• **CHU KWAN YI ET AL: "Angiotensin II in type 2 diabetes mellitus.", CURRENT PROTEIN & PEPTIDE SCIENCE FEB 2009, vol. 10, no. 1, février 2009 (2009-02), pages 75-84, XP009178203, ISSN: 1389-2037**

SEQUENCE LISTING

<110> UNIVERSITE DE LA ROCHELLE
UNIVERSITE BLAISE PASCAL-CLERMONT II

<120> COMPOSITIONS POUR LA PREVENTION ET/OU LE TRAITEMENT DE PATHOLOGIES LIEES A L'ALPHA-GLUCOSIDASE

<130> WOB 14 AF SGC APVA

<150> FR14/51280
<151> 2014-02-18

<160> 17

<170> PatentIn version 3.5

<210> 1
<211> 5
<212> PRT
<213> peptide synthétique

<400> 1

```
Ala Pro Phe Pro Glu
1               5
```

<210> 2
<211> 7
<212> PRT
<213> peptide synthétique

<400> 2

```
Ala Pro Phe Pro Glu Val Phe
1               5
```

<210> 3
<211> 7

<212> PRT
<213> peptide synthétique

<400> 3

```
                              Ser Ala Pro Leu Arg Val Tyr
                              1               5
```

<210> 4
<211> 7
<212> PRT
<213> peptide synthétique

<400> 4

```
                              Val Ala Pro Phe Pro Glu Val
                              1               5
```

<210> 5
<211> 6
<212> PRT
<213> peptide synthétique

<400> 5

```
                              Ala Val Pro Tyr Gln Arg
                              1               5
```

<210> 6
<211> 4
<212> PRT
<213> peptide synthétique

<400> 6

```
                              Val Ala Pro Gly
                              1
```

<210> 7
<211> 6
<212> PRT
<213> peptide synthétique

<400> 7

```
                              Val Gly Val Ala Pro Gly
                              1               5
```

<210> 8
<211> 178
<212> PRT
<213> Bos taurus

<400> 8

```
Met Lys Cys Leu Leu Leu Ala Leu Ala Leu Thr Cys Gly Ala Gln Ala
1               5                   10                  15

Leu Ile Val Thr Gln Thr Met Lys Gly Leu Asp Ile Gln Lys Val Ala
            20                  25                  30

Gly Thr Trp Tyr Ser Leu Ala Met Ala Ala Ser Asp Ile Ser Leu Leu
            35                  40                  45

Asp Ala Gln Ser Ala Pro Leu Arg Val Tyr Val Glu Glu Leu Lys Pro
    50                  55                  60

Thr Pro Glu Gly Asp Leu Glu Ile Leu Leu Gln Lys Trp Glu Asn Gly
65                  70                  75                  80

Glu Cys Ala Gln Lys Lys Ile Ile Ala Glu Lys Thr Lys Ile Pro Ala
                85                  90                  95

Val Phe Lys Ile Asp Ala Leu Asn Glu Asn Lys Val Leu Val Leu Asp
                100                 105                 110

Thr Asp Tyr Lys Lys Tyr Leu Leu Phe Cys Met Glu Asn Ser Ala Glu
            115                 120                 125

Pro Glu Gln Ser Leu Ala Cys Gln Cys Leu Val Arg Thr Pro Glu Val
    130                 135                 140

Asp Asp Glu Ala Leu Glu Lys Phe Asp Lys Ala Leu Lys Ala Leu Pro
145                 150                 155                 160

Met His Ile Arg Leu Ser Phe Asn Pro Thr Gln Leu Glu Glu Gln Cys
                165                 170                 175

His Ile
```

<210> 9
<211> 214
<212> PRT
<213> Bos taurus

<400> 9

```
Met Lys Leu Leu Ile Leu Thr Cys Leu Val Ala Val Ala Leu Ala Arg
1               5               10              15

Pro Lys His Pro Ile Lys His Gln Gly Leu Pro Gln Glu Val Leu Asn
            20              25              30

Glu Asn Leu Leu Arg Phe Phe Val Ala Pro Phe Pro Glu Val Phe Gly
        35              40              45

Lys Glu Lys Val Asn Glu Leu Ser Lys Asp Ile Gly Ser Glu Ser Thr
    50              55              60

Glu Asp Gln Ala Met Glu Asp Ile Lys Gln Met Glu Ala Glu Ser Ile
65              70              75              80

Ser Ser Ser Glu Glu Ile Val Pro Asn Ser Val Glu Gln Lys His Ile
            85              90              95

Gln Lys Glu Asp Val Pro Ser Glu Arg Tyr Leu Gly Tyr Leu Glu Gln
            100             105             110

Leu Leu Arg Leu Lys Lys Tyr Lys Val Pro Gln Leu Glu Ile Val Pro
        115             120             125

Asn Ser Ala Glu Glu Arg Leu His Ser Met Lys Glu Gly Ile His Ala


        130             135             140

Gln Gln Lys Glu Pro Met Ile Gly Val Asn Gln Glu Leu Ala Tyr Phe
145             150             155             160

Tyr Pro Glu Leu Phe Arg Gln Phe Tyr Gln Leu Asp Ala Tyr Pro Ser
            165             170             175

Gly Ala Trp Tyr Tyr Val Pro Leu Gly Thr Gln Tyr Thr Asp Ala Pro
            180             185             190

Ser Phe Ser Asp Ile Pro Asn Pro Ile Gly Ser Glu Asn Ser Glu Lys
            195             200             205

Thr Thr Met Pro Leu Trp
            210
```

<210> 10
<211> 224
<212> PRT
<213> Bos taurus

<400> 10

```
Met Lys Val Leu Ile Leu Ala Cys Leu Val Ala Leu Ala Leu Ala Arg
1               5               10              15

Glu Leu Glu Glu Leu Asn Val Pro Gly Glu Ile Val Glu Ser Leu Ser
            20              25              30

Ser Ser Glu Glu Ser Ile Thr Arg Ile Asn Lys Lys Ile Glu Lys Phe
            35              40              45

Gln Ser Glu Glu Gln Gln Gln Thr Glu Asp Glu Leu Gln Asp Lys Ile
        50              55              60

His Pro Phe Ala Gln Thr Gln Ser Leu Val Tyr Pro Phe Pro Gly Pro
65              70              75              80

Ile Pro Asn Ser Leu Pro Gln Asn Ile Pro Pro Leu Thr Gln Thr Pro
            85              90              95

Val Val Val Pro Pro Phe Leu Gln Pro Glu Val Met Gly Val Ser Lys
            100             105             110

Val Lys Glu Ala Met Ala Pro Lys His Lys Glu Met Pro Phe Pro Lys
        115             120             125

Tyr Pro Val Glu Pro Phe Thr Glu Ser Gln Ser Leu Thr Leu Thr Asp

        130             135                 140

Val Glu Asn Leu His Leu Pro Leu Pro Leu Leu Gln Ser Trp Met His
145             150             155             160

Gln Pro His Gln Pro Leu Pro Pro Thr Val Met Phe Pro Pro Gln Ser
            165             170             175

Val Leu Ser Leu Ser Gln Ser Lys Val Leu Pro Val Pro Gln Lys Ala
            180             185             190

Val Pro Tyr Pro Gln Arg Asp Met Pro Ile Gln Ala Phe Leu Leu Tyr
            195             200             205

Gln Glu Pro Val Leu Gly Pro Val Arg Gly Pro Phe Pro Ile Ile Val
        210             215             220
```

<210> 11
<211> 708

<212> PRT
<213> Bos taurus

<400> 11

```
Met Lys Leu Phe Val Pro Ala Leu Leu Ser Leu Gly Ala Leu Gly Leu
1               5                   10                  15

Cys Leu Ala Ala Pro Arg Lys Asn Val Arg Trp Cys Thr Ile Ser Gln
            20                  25                  30

Pro Glu Trp Phe Lys Cys Arg Arg Trp Gln Trp Arg Met Lys Lys Leu
            35                  40                  45

Gly Ala Pro Ser Ile Thr Cys Val Arg Arg Ala Phe Ala Leu Glu Cys
    50                  55                  60

Ile Arg Ala Ile Ala Glu Lys Lys Ala Asp Ala Val Thr Leu Asp Gly
65                  70                  75                  80

Gly Met Val Phe Glu Ala Gly Arg Asp Pro Tyr Lys Leu Arg Pro Val
            85                  90                  95

Ala Ala Glu Ile Tyr Gly Thr Lys Glu Ser Pro Gln Thr His Tyr Tyr
            100                 105                 110

Ala Val Ala Val Val Lys Lys Gly Ser Asn Phe Gln Leu Asp Gln Leu
        115                 120                 125

Gln Gly Arg Lys Ser Cys His Thr Gly Leu Gly Arg Ser Ala Gly Trp
```

```
          130                    135                         140


Ile Ile Pro Met Gly Ile Leu Arg Pro Tyr Leu Ser Trp Thr Glu Ser
145             150             155                 160


Leu Glu Pro Leu Gln Gly Ala Val Ala Lys Phe Phe Ser Ala Ser Cys
            165             170                 175


Val Pro Cys Ile Asp Arg Gln Ala Tyr Pro Asn Leu Cys Gln Leu Cys
            180             185                 190


Lys Gly Glu Gly Glu Asn Gln Cys Ala Cys Ser Ser Arg Glu Pro Tyr
            195             200             205


Phe Gly Tyr Ser Gly Ala Phe Lys Cys Leu Gln Asp Gly Ala Gly Asp
    210             215             220


Val Ala Phe Val Lys Glu Thr Thr Val Phe Glu Asn Leu Pro Glu Lys
225             230             235                 240


Ala Asp Arg Asp Gln Tyr Glu Leu Leu Cys Leu Asn Asn Ser Arg Ala
            245             250             255


Pro Val Asp Ala Phe Lys Glu Cys His Leu Ala Gln Val Pro Ser His
            260             265             270


Ala Val Val Ala Arg Ser Val Asp Gly Lys Glu Asp Leu Ile Trp Lys
            275             280             285


Leu Leu Ser Lys Ala Gln Glu Lys Phe Gly Lys Asn Lys Ser Arg Ser
    290             295             300


Phe Gln Leu Phe Gly Ser Pro Pro Gly Gln Arg Asp Leu Leu Phe Lys
305             310             315                 320


Asp Ser Ala Leu Gly Phe Leu Arg Ile Pro Ser Lys Val Asp Ser Ala
            325             330             335


Leu Tyr Leu Gly Ser Arg Tyr Leu Thr Thr Leu Lys Asn Leu Arg Glu
            340             345             350


Thr Ala Glu Glu Val Lys Ala Arg Tyr Thr Arg Val Val Trp Cys Ala
            355             360             365


Val Gly Pro Glu Glu Gln Lys Lys Cys Gln Gln Trp Ser Gln Gln Ser
    370             375             380
```

47

```
Gly Gln Asn Val Thr Cys Ala Thr Ala Ser Thr Thr Asp Asp Cys Ile
385             390             395             400

Val Leu Val Leu Lys Gly Glu Ala Asp Ala Leu Asn Leu Asp Gly Gly
                405             410             415

Tyr Ile Tyr Thr Ala Gly Lys Cys Gly Leu Val Pro Val Leu Ala Glu
            420             425             430

Asn Arg Lys Ser Ser Lys His Ser Ser Leu Asp Cys Val Leu Arg Pro
            435             440             445

Thr Glu Gly Tyr Leu Ala Val Ala Val Val Lys Lys Ala Asn Glu Gly
            450             455             460

Leu Thr Trp Asn Ser Leu Lys Asp Lys Lys Ser Cys His Thr Ala Val
465             470             475             480

Asp Arg Thr Ala Gly Trp Asn Ile Pro Met Gly Leu Ile Val Asn Gln
            485             490             495

Thr Gly Ser Cys Ala Phe Asp Glu Phe Phe Ser Gln Ser Cys Ala Pro
            500             505             510

Gly Ala Asp Pro Lys Ser Arg Leu Cys Ala Leu Cys Ala Gly Asp Asp
            515             520             525

Gln Gly Leu Asp Lys Cys Val Pro Asn Ser Lys Glu Lys Tyr Tyr Gly
            530             535             540

Tyr Thr Gly Ala Phe Arg Cys Leu Ala Glu Asp Val Gly Asp Val Ala
545             550             555             560

Phe Val Lys Asn Asp Thr Val Trp Glu Asn Thr Asn Gly Glu Ser Thr
            565             570             575

Ala Asp Trp Ala Lys Asn Leu Asn Arg Glu Asp Phe Arg Leu Leu Cys
            580             585             590

Leu Asp Gly Thr Arg Lys Pro Val Thr Glu Ala Gln Ser Cys His Leu
            595             600             605

Ala Val Ala Pro Asn His Ala Val Val Ser Arg Ser Asp Arg Ala Ala
            610             615             620

His Val Lys Gln Val Leu Leu His Gln Gln Ala Leu Phe Gly Lys Asn
625             630             635             640
```

48

```
Gly Lys Asn Cys Pro Asp Lys Phe Cys Leu Phe Lys Ser Glu Thr Lys
                645             650             655

Asn Leu Leu Phe Asn Asp Asn Thr Glu Cys Leu Ala Lys Leu Gly Gly
            660             665             670

Arg Pro Thr Tyr Glu Glu Tyr Leu Gly Thr Glu Tyr Val Thr Ala Ile
            675             680             685

Ala Asn Leu Lys Lys Cys Ser Thr Ser Pro Leu Leu Glu Ala Cys Ala
    690             695             700

Phe Leu Thr Arg
705
```

<210> 12
<211> 376
<212> PRT
<213> Salmo

<400> 12

```
Met Val Glu Asp Glu Val Ala Ala Leu Val Ile Asp Asn Gly Ser Gly
1               5               10              15

Met Cys Lys Ser Gly Phe Ala Gly Asp Asp Ala Pro Arg Ala Val Phe
            20              25              30

Pro Ser Ile Val Gly Arg Pro Arg His Val Gly Ile Met Val Gly Met
            35              40              45

Gly Gln Lys Asp Ser Tyr Val Gly Asp Glu Ala Gln Ser Lys Arg Gly
    50              55              60

Ile Leu Ser Leu Lys Tyr Pro Ile Asp His Gly Ile Val Thr Asn Trp
65              70              75              80

Asp Asp Met Glu Lys Ile Trp His His Thr Phe Tyr Asn Glu Leu Arg
            85              90              95

Val Ala Pro Glu Glu His Pro Val Leu Leu Thr Glu Ala Pro Leu Asn
            100             105             110

Pro Lys Asn Asn Arg Glu Lys Met Thr Gln Ile Met Phe Glu Thr Phe
            115             120             125

Asn Ser Pro Ala Met Tyr Val Ala Ile Gln Ala Val Leu Ser Leu Tyr
    130             135             140
```

```
Ala Ser Gly Arg Thr Thr Gly Ile Val Leu Asp Ser Gly Asp Gly Val
145             150             155             160

Thr His Thr Val Pro Ile Tyr Glu Gly Tyr Ala Leu Pro His Ala Val
                165             170             175

Leu Arg Leu Asp Leu Ala Gly Arg Asp Leu Thr Asp Tyr Leu Met Lys
            180             185             190

Val Leu Thr Glu Arg Gly Tyr Ser Phe Thr Thr Thr Ala Glu Arg Glu
        195             200             205

Ile Val Arg Asp Val Lys Glu Lys Leu Cys Tyr Val Ala Leu Asp Tyr
    210             215             220

Thr Asn Glu Leu Ala Val Ala Gly Ser Ser Ser Ser Leu Glu Lys Ser
225             230             235             240

Tyr Glu Leu Pro Asp Gly Gln Val Ile Thr Ile Gly Ser Glu Arg Phe
            245             250             255

Arg Cys Pro Glu Ala Leu Phe Gln Pro Ala Leu Ile Gly Met Glu Ala
            260             265             270

Val Gly Ile His Glu Thr Ala Tyr Asn Ser Ile Met Lys Cys Asp Val
        275             280             285

Asp Ile Arg Lys Asp Leu Tyr Ala Asn Thr Val Leu Ser Gly Gly Ser
    290             295             300

Thr Met Phe Ser Gly Ile Ala Asp Arg Met Gln Lys Glu Val Ser Ala
305             310             315             320

Leu Ala Pro Thr Thr Met Lys Ile Lys Ile Ile Ser Pro Pro Glu Arg
            325             330             335

Lys Tyr Ser Val Trp Ile Gly Gly Ser Ile Leu Ala Ser Leu Ser Thr
        340             345             350

Phe Gln Gln Met Trp Ile Ser Lys Met Glu Tyr Asp Glu Ser Gly Pro
        355             360             365

Ala Ile Val His Arg Lys Cys Phe
370             375
```

<210> 13
<211> 1352

<212> PRT
<213> Salmo

<400> 13

```
Met Leu Ser Phe Val Asp Asn Arg Ile Leu Leu Leu Ala Val Thr
1               5               10              15

Ser Leu Leu Ala Ser Cys Gln Ser Gly Pro Arg Gly Ala Lys Gly Pro
            20              25              30

Arg Gly Asp Arg Gly Pro Gln Gly Pro Asn Gly Arg Asp Gly Lys Ala
        35              40              45

Gly Leu Pro Gly Val Ala Gly Pro Pro Gly Pro Pro Gly Leu Gly Gly
    50              55              60

Asn Phe Ala Ala Gln Phe Asp Gly Gly Lys Gly Ser Asp Pro Gly Pro
65              70              75              80

Gly Pro Met Gly Leu Met Gly Ser Arg Gly Pro Asn Gly Pro Pro Gly
            85              90              95

Ser Pro Gly Pro Gln Gly Phe Thr Gly His Ala Gly Glu Pro Gly Glu
        100             105             110

Pro Gly Gln Thr Gly Ser Ile Gly Ala Arg Gly Pro Thr Gly Ser Ala
        115             120             125

Gly Lys Pro Gly Glu Asp Gly Asn Asn Gly Arg Pro Gly Lys Pro Gly
    130             135             140

Asp Arg Gly Gly Pro Gly Thr Gln Gly Ala Arg Gly Phe Pro Gly Thr
145             150             155             160

Pro Gly Leu Pro Gly Met Lys Gly His Arg Gly Tyr Asn Gly Leu Asp
            165             170             175

Gly Arg Lys Gly Glu Ser Gly Thr Ala Gly Ala Lys Gly Glu Thr Gly
        180             185             190

Ala His Gly Ala Asn Gly Thr Pro Gly Pro Ala Gly Ser Arg Gly Leu
    195             200             205

Asn Gly Glu Arg Gly Arg Ala Gly Pro Ala Gly Pro Ala Gly Ala Arg
    210             215             220

Gly Ala Asp Gly Ser Thr Gly Pro Ala Gly Pro Ala Gly Pro Leu Gly
225             230             235             240
```

52

Ala Ala Gly Pro Pro Gly Phe Pro Gly Ala Pro Gly Pro Lys Gly Glu
            245             250             255

Ile Gly Gly Ala Gly Ser Asn Gly Pro Ser Gly Pro Gln Gly Gly Arg
            260             265             270

Gly Glu Pro Gly Ile Asn Gly Ala Val Gly Pro Val Gly Pro Val Gly
            275             280             285

Asn Pro Gly Asn Asn Gly Ile Asn Gly Ala Lys Gly Ala Ala Gly Leu
        290             295             300

Pro Gly Val Ala Gly Ala Pro Gly Phe Pro Gly Pro Arg Gly Gly Pro
305             310             315             320

Gly Pro Gln Gly Pro Gln Gly Ser Thr Gly Ala Arg Gly Leu Gly Gly
            325             330             335

Asp Pro Gly Pro Ser Gly Gln Lys Gly Asp Ser Gly Ala Lys Gly Glu
            340             345             350

Pro Gly His Ser Gly Val Gln Gly Ala Ala Gly Pro Ala Gly Glu Glu
        355             360             365

Gly Lys Arg Gly Ser Thr Gly Glu Ala Gly Ala Thr Gly Pro Ala Gly
        370             375             380

Leu Arg Gly Ala Arg Gly Gly Ala Gly Thr Arg Gly Leu Pro Gly Leu
385             390             395             400

Glu Gly Arg Gly Gly Pro Ile Gly Met Pro Gly Ala Arg Gly Ala Thr
            405             410             415

Gly Pro Ala Gly Ile Arg Gly Ala Pro Gly Asp Ala Gly Arg Ala Gly
            420             425             430

Glu Ser Gly Leu Thr Gly Ala Arg Gly Leu Pro Gly Asn Ser Gly Gln
        435             440             445

Gly Gly Pro Pro Gly Lys Glu Gly Pro Ser Gly Ala Ala Gly Leu Asp
        450             455             460

Gly Arg Thr Gly Pro Pro Gly Pro Thr Gly Pro Arg Gly Gln Pro Gly
465             470             475             480

Asn Ile Gly Phe Pro Gly Pro Lys Gly Pro Gly Gly Glu Ala Gly Lys

```
                    485                     490                         495


        Gly Gly Asp Lys Gly Pro Thr Gly Ala Thr Gly Leu Arg Gly Gly Pro
                    500                     505                     510


        Gly Ala Asp Gly Asn Asn Gly Ala Pro Gly Pro Ala Gly Val Val Gly
                    515                     520                     525


        Asn Ala Gly Glu Lys Gly Glu Gln Gly Pro Ser Gly Ala Pro Gly Phe
                530                     535                     540


        Gln Gly Leu Pro Gly Pro Ala Gly Pro Ala Gly Glu Ala Gly Lys Ala
        545                     550                     555                     560


        Gly Asn Gln Gly Met Pro Gly Asp Gln Gly Leu Pro Gly Pro Ala Gly
                        565                     570                     575


        Val Lys Gly Glu Arg Gly Asn Ser Gly Pro Ala Gly Ser Ala Gly Ser
                    580                     585                     590


        Gln Gly Ala Ile Gly Ala Arg Gly Pro Ala Gly Thr Pro Gly Pro Asp
                    595                     600                     605


        Gly Gly Lys Gly Glu Pro Gly Ser Val Gly Ile Val Gly Ala Ala Gly
                610                     615                     620


        His Gln Gly Pro Gly Gly Met Pro Gly Glu Arg Gly Ala Gly Gly Thr
        625                     630                     635                     640


        Pro Gly Pro Lys Gly Glu Lys Gly Glu Gly Gly His Arg Gly Leu Glu
                        645                     650                     655


        Gly Asn Met Gly Arg Asp Gly Ala Arg Gly Ala Ala Gly Pro Ser Gly
                        660                     665                     670


        Pro Pro Gly Pro Ser Gly Ala Asn Gly Glu Lys Gly Glu Ser Gly Ser
                    675                     680                     685


        Phe Gly Pro Ala Gly Pro Ala Gly Leu Arg Gly Pro Ser Gly Glu Arg
                    690                     695                     700


        Gly Glu Gly Gly Pro Ala Gly Pro Pro Gly Phe Ala Gly Pro Pro Gly
        705                     710                     715                     720


        Ser Asp Gly Gln Ser Gly Pro Arg Gly Glu Lys Gly Pro Ala Gly Gly
                        725                     730                     735
```

54

```
Lys Gly Asp Val Gly Pro Ala Gly Pro Ala Gly Pro Ser Gly Gln Ser
            740                 745                 750

Gly Pro Ser Gly Ala Ser Gly Pro Ala Gly Pro Pro Gly Gly Arg Gly
            755                 760                 765

Asp Ala Gly Pro Ser Gly Leu Thr Gly Phe Pro Gly Ala Ala Gly Arg
            770                 775                 780

Val Gly Gly Pro Gly Pro Ala Gly Ile Ser Gly Pro Pro Gly Ser Ala
785                 790                 795                 800

Gly Pro Ala Gly Lys Asp Gly Pro Arg Gly Leu Arg Gly Asp Ala Gly
                805                 810                 815

Pro Gly Gly Pro Gln Gly Glu Gln Gly Val Val Gly Pro Ala Gly Ile
            820                 825                 830

Ala Gly Asp Lys Gly Pro Ser Gly Glu Gly Gly Pro Pro Gly Ala Pro
            835                 840                 845

Gly Thr Ala Gly Pro Gln Gly Val Leu Gly Pro Ser Gly Phe Val Gly
            850                 855                 860

Leu Pro Gly Ser Arg Gly Asp Lys Gly Leu Pro Gly Gly Pro Gly Ala
865                 870                 875                 880

Val Gly Glu Pro Gly Arg Leu Gly Pro Ala Gly Ala Ser Gly Pro Arg
                885                 890                 895

Gly Pro Ser Gly Asn Ile Gly Met Pro Gly Met Thr Gly Thr Gln Gly
            900                 905                 910

Glu Ala Gly Arg Glu Gly Asn Ser Gly Asn Asp Gly Pro Pro Gly Arg
            915                 920                 925

Pro Gly Ala Ala Gly Phe Lys Gly Asp Arg Gly Glu Pro Gly Ser Pro
            930                 935                 940

Gly Ala Leu Gly Ser Ser Gly Gln Pro Gly Pro Asn Gly Pro Ala Gly
945                 950                 955                 960

Ser Ala Gly Arg Pro Gly Asn Arg Gly Glu Ser Gly Pro Thr Gly Asn
                965                 970                 975

Gly Gly Pro Val Gly Ala Ala Gly Ala Arg Gly Ala Pro Gly Pro Ala
            980                 985                 990
```

```
Gly Pro Arg Gly Glu Lys Gly Gly  Ala Gly Glu Lys Gly  Asp Arg Gly
        995                  1000                1005


Met Lys Gly Leu Arg Gly His  Gly Gly Leu Gln Gly  Met Pro Gly
    1010              1015                1020


Pro Asn Gly Pro Ser Gly Glu  Thr Gly Ser Ala Gly  Ile Thr Gly
    1025              1030                1035


Pro Ala Gly Pro Arg Gly Pro  Ala Gly Pro His Gly  Pro Pro Gly
    1040              1045                1050


Lys Asp Gly Arg Ala Gly Gly  His Gly Ala Ile Gly  Pro Val Gly
    1055              1060                1065


His Arg Gly Pro Pro Gly His  Leu Gly Pro Ala Gly  Pro Pro Gly
    1070              1075                1080


Ser Pro Gly Leu Pro Gly Pro  Ala Gly Pro Ala Gly  Gly Gly Tyr
    1085              1090                1095


Asp Gln Ser Gly Gly Tyr Asp  Glu Tyr Arg Ala Asp  Gln Pro Ser
    1100              1105                1110


Leu Arg Ala Lys Asp Tyr Glu  Val Asp Ala Thr Ile  Lys Ser Leu
    1115              1120                1125


Asn Ser Gln Ile Glu Asn Leu  Leu Thr Pro Glu Gly  Ser Lys Lys
    1130              1135                1140


Asn Pro Ala Arg Thr Cys Arg  Asp Ile Arg Leu Ser  His Pro Glu
    1145              1150                1155


Trp Ser Ser Gly Phe Tyr Trp  Ile Gly Pro Asn Gln  Gly Cys Ile
    1160              1165                1170


Ala Asp Ala Ile Lys Ala Tyr  Cys Asp Phe Ser Thr  Gly His Thr
    1175              1180                1185


Cys Ile His Pro His Pro Glu  Ser Ile Ala Arg Lys  Asn Trp Tyr
    1190              1195                1200


Arg Ser Ser Glu Asn Lys Lys  His Val Trp Phe Gly  Glu Thr Ile
    1205              1210                1215


Asn Gly Gly Thr Glu Phe Ala  Tyr Asn Asp Glu Thr  Leu Ser Pro
    1220              1225                1230
```

```
Gln Ser  Met Ala Thr Gln Leu  Ala Phe Met Arg Leu  Leu Ala Asn
    1235             1240             1245

Gln Ala  Thr Gln Asn Ile Thr  Tyr His Cys Lys Asn  Ser Val Ala
    1250             1255             1260

Tyr Met  Asp Gly Glu Asn Gly  Asn Leu Lys Lys Ala  Val Leu Leu
    1265             1270             1275

Gln Gly  Ser Asn Asp Val Glu  Leu Arg Ala Glu Gly  Asn Ser Arg
    1280             1285             1290

Phe Thr  Phe Asn Val Leu Glu  Asp Gly Cys Thr Arg  His Thr Gly
    1295             1300             1305

Gln Trp  Ser Lys Thr Val Ile  Glu Tyr Arg Thr Asn  Lys Pro Ser
    1310             1315             1320

Arg Leu  Pro Ile Leu Asp Ile  Ala Pro Leu Asp Ile  Gly Glu Ala
    1325             1330             1335

Asp Gln  Glu Phe Gly Leu Asp  Ile Gly Pro Val Cys  Phe Lys
    1340             1345             1350
```

<210> 14
<211> 1306
<212> PRT
<213> Bos taurus

<400> 14

```
Gln Met Ala Gly Gly Phe Asp Glu Lys Ala Gly Gly Ala Gln Met Gly
1             5             10             15

Val Met Gln Gly Pro Met Gly Pro Met Gly Pro Arg Gly Pro Pro Gly
            20             25             30

Pro Ala Gly Ala Pro Gly Pro Gln Gly Phe Gln Gly Asn Pro Gly Glu
            35             40             45

Pro Gly Glu Pro Gly Val Ser Gly Pro Met Gly Pro Arg Gly Pro Pro
            50             55             60

Gly Pro Pro Gly Lys Pro Gly Asp Asp Gly Glu Ala Gly Lys Pro Gly
65             70             75             80

Lys Ser Gly Glu Arg Gly Pro Pro Gly Pro Gln Gly Ala Arg Gly Phe
                85             90             95
```

Pro Gly Thr Pro Gly Leu Pro Gly Val Lys Gly His Arg Gly Tyr Pro
100 105 110

Gly Leu Asp Gly Ala Lys Gly Glu Ala Gly Ala Pro Gly Val Lys Gly
115 120 125

Glu Ser Gly Ser Pro Gly Glu Asn Gly Ser Pro Gly Pro Met Gly Pro
130 135 140

Arg Gly Leu Pro Gly Glu Arg Gly Arg Thr Gly Pro Ala Gly Ala Ala
145 150 155 160

Gly Ala Arg Gly Asn Asp Gly Gln Pro Gly Pro Ala Gly Pro Pro Gly
165 170 175

Pro Val Gly Pro Ala Gly Gly Pro Gly Phe Pro Gly Ala Pro Gly Ala
180 185 190

Lys Gly Glu Ala Gly Pro Thr Gly Ala Arg Gly Pro Glu Gly Ala Gln
195 200 205

Gly Pro Arg Gly Glu Pro Gly Thr Pro Gly Ser Pro Gly Pro Ala Gly
210 215 220

Ala Ala Gly Asn Pro Gly Thr Asp Gly Ile Pro Gly Ala Lys Gly Ser
225 230 235 240

Ala Gly Ala Pro Gly Ile Ala Gly Ala Pro Gly Phe Pro Gly Pro Arg
245 250 255

Gly Pro Pro Gly Pro Gln Gly Ala Thr Gly Pro Leu Gly Pro Lys Gly
260 265 270

Gln Thr Gly Glu Pro Gly Ile Ala Gly Phe Lys Gly Glu Gln Gly Pro
275 280 285

Lys Gly Glu Pro Gly Pro Ala Gly Pro Gln Gly Ala Pro Gly Pro Ala
290 295 300

Gly Glu Glu Gly Lys Arg Gly Ala Arg Gly Glu Pro Gly Gly Ala Gly
305 310 315 320

Pro Ala Gly Pro Pro Gly Glu Arg Gly Ala Pro Gly Asn Arg Gly Phe
325 330 335

Pro Gly Gln Asp Gly Leu Ala Gly Pro Lys Gly Ala Pro Gly Glu Arg

58

<pre>
              340                    345                         350


    Gly Pro Ser Gly Leu Ala Gly Pro Lys Gly Ala Asn Gly Asp Pro Gly
            355                 360                 365


    Arg Pro Gly Glu Pro Gly Leu Pro Gly Ala Arg Gly Leu Thr Gly Arg
            370                 375                 380


    Pro Gly Asp Ala Gly Pro Gln Gly Lys Val Gly Pro Ser Gly Ala Pro
    385                 390                 395                 400


    Gly Glu Asp Gly Arg Pro Gly Pro Pro Gly Pro Gln Gly Ala Arg Gly
                    405                 410                 415


    Gln Pro Gly Val Met Gly Phe Pro Gly Pro Lys Gly Ala Asn Gly Glu
                420                 425                 430


    Pro Gly Lys Ala Gly Glu Lys Gly Leu Pro Gly Ala Pro Gly Leu Arg
            435                 440                 445


    Gly Leu Pro Gly Lys Asp Gly Glu Thr Gly Ala Ala Gly Pro Pro Gly
            450                 455                 460


    Pro Ala Gly Pro Ala Gly Glu Arg Gly Glu Gln Gly Ala Pro Gly Pro
    465                 470                 475                 480


    Ser Gly Phe Gly Gly Leu Pro Gly Pro Pro Gly Pro Pro Gly Glu Gly
                485                 490                 495


    Gly Lys Pro Gly Asp Gly Gly Val Pro Gly Glu Ala Gly Ala Pro Gly
                500                 505                 510


    Leu Val Gly Pro Arg Gly Glu Arg Gly Phe Pro Gly Glu Arg Gly Ser
            515                 520                 525


    Pro Gly Ser Gln Gly Leu Gln Gly Ala Arg Gly Leu Pro Gly Thr Pro
            530                 535                 540


    Gly Thr Asp Gly Pro Lys Gly Ala Ala Gly Pro Ala Gly Pro Pro Gly
    545                 550                 555                 560


    Ala Gln Gly Pro Pro Gly Leu Gln Gly Met Pro Gly Glu Arg Gly Ala
                565                 570                 575


    Ala Gly Ile Ala Gly Pro Lys Gly Asp Arg Gly Asp Val Gly Glu Lys
                580                 585                 590
</pre>

Gly Pro Glu Gly Ala Pro Gly Lys Asp Gly Gly Arg Gly Leu Thr Gly
595                 600                 605

Pro Ile Gly Pro Pro Gly Pro Ala Gly Ala Asn Gly Glu Lys Gly Glu
610                 615                 620

Val Gly Pro Pro Gly Pro Ala Gly Thr Ala Gly Ala Arg Gly Ala Pro
625                 630                 635                 640

Gly Glu Arg Gly Glu Thr Gly Pro Pro Gly Pro Ala Gly Phe Ala Gly
645                 650                 655

Pro Pro Gly Ala Asp Gly Gln Pro Gly Ala Lys Gly Glu Gln Gly Glu
660                 665                 670

Ala Gly Gln Lys Gly Asp Ala Gly Ala Pro Gly Pro Gln Gly Pro Ser
675                 680                 685

Gly Ala Pro Gly Pro Gln Gly Pro Thr Gly Val Thr Gly Pro Lys Gly
690                 695                 700

Ala Arg Gly Ala Gln Gly Pro Pro Gly Ala Thr Gly Phe Pro Gly Ala
705                 710                 715                 720

Ala Gly Arg Val Gly Pro Pro Gly Ser Asn Gly Asn Pro Gly Pro Pro
725                 730                 735

Gly Pro Pro Gly Pro Ser Gly Lys Asp Gly Pro Lys Gly Ala Arg Gly
740                 745                 750

Asp Ser Gly Pro Pro Gly Arg Ala Gly Asp Pro Gly Leu Gln Gly Pro
755                 760                 765

Ala Gly Pro Pro Gly Glu Lys Gly Glu Pro Gly Asp Asp Gly Pro Ser
770                 775                 780

Gly Pro Asp Gly Pro Pro Gly Pro Gln Gly Leu Ala Gly Gln Arg Gly
785                 790                 795                 800

Ile Val Gly Leu Pro Gly Gln Arg Gly Glu Arg Gly Phe Pro Gly Leu
805                 810                 815

Pro Gly Pro Ser Gly Glu Pro Gly Lys Gln Gly Ala Pro Gly Ala Ser
820                 825                 830

Gly Asp Arg Gly Pro Pro Gly Pro Val Gly Pro Pro Gly Leu Thr Gly
835                 840                 845

```
Pro Ala Gly Glu Pro Gly Arg Glu Gly Ser Pro Gly Ala Asp Gly Pro
    850             855             860

Pro Gly Arg Asp Gly Ala Ala Gly Val Lys Gly Asp Arg Gly Glu Thr
865             870             875             880

Gly Ala Val Gly Ala Pro Gly Ala Pro Gly Pro Gly Ser Pro Gly
            885             890             895

Pro Ala Gly Pro Ile Gly Lys Gln Gly Asp Arg Gly Glu Ala Gly Ala
        900             905             910

Gln Gly Pro Met Gly Pro Ala Gly Pro Ala Gly Ala Arg Gly Met Pro
        915             920             925

Gly Pro Gln Gly Pro Arg Gly Asp Lys Gly Glu Thr Gly Glu Ala Gly
    930             935             940

Glu Arg Gly Leu Lys Gly His Arg Gly Phe Thr Gly Leu Gln Gly Leu
945             950             955             960

Pro Gly Pro Pro Gly Pro Ser Gly Asp Gln Gly Ala Ser Gly Pro Ala
            965             970             975

Gly Pro Ser Gly Pro Arg Gly Pro Pro Gly Pro Val Gly Pro Ser Gly
            980             985             990

Lys Asp Gly Ala Asn Gly Ile Pro  Gly Pro Ile Gly Pro  Pro Gly Pro
        995             1000            1005

Arg Gly  Arg Ser Gly Glu Thr  Gly Pro Ala Gly Pro  Pro Gly Asn
    1010            1015            1020

Pro Gly  Pro Pro Gly Pro Pro  Gly Pro Pro Gly Pro  Gly Ile Asp
    1025            1030            1035

Met Ser  Ala Phe Ala Gly Leu  Gly Gln Arg Glu Lys  Gly Pro Asp
    1040            1045            1050

Pro Leu  Gln Tyr Met Arg Ala  Asp Glu Ala Ala Gly  Asn Leu Arg
    1055            1060            1065

Gln His  Asp Ala Glu Val Asp  Ala Thr Leu Lys Ser  Leu Asn Asn
    1070            1075            1080

Gln Ile  Glu Ser Leu Arg Ser  Pro Glu Gly Ser Arg  Lys Asn Pro
    1085            1090            1095
```

```
Ala Arg Thr Cys Arg Asp Leu Lys Leu Cys His Pro Glu Trp Lys
    1100              1105          1110

Ser Gly Asp Tyr Trp Ile Asp Pro Asn Gln Gly Cys Thr Leu Asp
    1115              1120          1125

Ala Met Lys Val Phe Cys Asn Met Glu Thr Gly Glu Thr Cys Val
    1130              1135          1140

Tyr Pro Asn Pro Ala Ser Val Pro Lys Lys Asn Trp Trp Ser Ser
    1145              1150          1155

Lys Ser Lys Asp Lys Lys His Ile Trp Phe Gly Glu Thr Ile Asn
    1160              1165          1170

Gly Gly Phe His Phe Ser Tyr Gly Asp Asp Asn Leu Ala Pro Asn
    1175              1180          1185

Thr Ala Asn Val Gln Met Thr Phe Leu Arg Leu Leu Ser Thr Glu
    1190              1195          1200

Gly Ser Gln Asn Ile Thr Tyr His Cys Lys Asn Ser Ile Ala Tyr
    1205              1210          1215

Leu Asp Glu Ala Ala Gly Asn Leu Lys Lys Ala Leu Leu Ile Gln
    1220              1225          1230

Gly Ser Asn Asp Val Glu Ile Arg Ala Glu Gly Asn Ser Arg Phe
    1235              1240          1245

Thr Tyr Thr Val Leu Lys Asp Gly Cys Thr Lys His Thr Gly Lys
    1250              1255          1260

Trp Gly Lys Thr Met Ile Glu Tyr Arg Ser Gln Lys Thr Ser Arg
    1265              1270          1275

Leu Pro Ile Ile Asp Ile Ala Pro Met Asp Ile Gly Gly Pro Glu
    1280              1285          1290

Gln Glu Phe Gly Val Asp Ile Gly Pro Val Cys Phe Leu
    1295              1300          1305
```

<210> 15
<211> 805
<212> PRT
<213> Bos taurus

<400> 15

```
Met Ala Gly Leu Thr Ala Ala Ala Arg Arg Pro Gly Val Leu Leu Leu
1               5                   10              15

Leu Leu Cys Ile Leu Gln Pro Ser Gln Pro Gly Gly Val Pro Gly Ala
        20                  25                  30

Val Pro Gly Gly Val Pro Gly Gly Val Phe Phe Pro Gly Ala Gly Leu
        35                  40                  45

Gly Gly Leu Gly Val Gly Ala Leu Gly Pro Gly Val Lys Pro Ala Lys
        50                  55                  60

Pro Gly Val Gly Gly Leu Ala Gly Pro Gly Leu Gly Ala Gly Leu Gly
65                  70                  75                  80

Ala Leu Pro Gly Ala Phe Pro Gly Ala Leu Val Pro Gly Gly Pro Ala
                85                  90                  95

Gly Ala Ala Ala Ala Tyr Lys Ala Ala Ala Lys Ala Gly Ala Ala Gly
        100                 105                 110

Leu Gly Val Gly Gly Ile Gly Gly Val Gly Gly Leu Gly Val Ser Thr
        115                 120                 125

Gly Ala Val Val Pro Gln Leu Gly Ala Gly Val Gly Ala Gly Val Lys
    130                 135                 140

Pro Gly Lys Val Pro Gly Val Gly Leu Pro Gly Val Tyr Pro Gly Gly
145                 150                 155                 160

Val Leu Pro Gly Ala Gly Ala Arg Phe Pro Gly Ile Gly Val Leu Pro
                165                 170                 175

Gly Val Pro Thr Gly Ala Gly Val Lys Pro Lys Ala Pro Gly Gly Gly
                180                 185                 190

Gly Ala Phe Ala Gly Ile Pro Gly Val Gly Pro Phe Gly Gly Gln Gln
                195                 200                 205

Pro Gly Val Pro Leu Gly Tyr Pro Ile Lys Ala Pro Lys Leu Pro Gly
        210                 215                 220

Gly Tyr Gly Leu Pro Tyr Ser Thr Gly Lys Leu Pro Tyr Gly Phe Gly
225                 230                 235                 240

Pro Gly Gly Val Ala Gly Ala Ala Gly Lys Ala Gly Tyr Pro Thr Gly
```

63

245                    250                         255

Thr Gly Val Gly Pro Gln Ala Ala Ala Ala Ala Ala Lys Ala Ala Ala
            260                 265                 270

Lys Leu Gly Ala Gly Gly Ala Gly Val Leu Pro Gly Val Gly Val Gly
            275                 280                 285

Gly Ala Gly Ile Pro Gly Ala Pro Gly Ala Ile Pro Gly Ile Gly Gly
            290                 295                 300

Ile Ala Gly Val Gly Ala Pro Asp Ala Ala Ala Ala Ala Ala Ala Ala
305                 310                 315                 320

Ala Lys Ala Ala Lys Phe Gly Ala Ala Gly Gly Phe Pro Gly Val Gly
            325                 330                 335

Val Pro Gly Val Gly Val Pro Gly Val Gly Val Pro Gly Val Gly Val
            340                 345                 350

Pro Gly Val Gly Val Pro Gly Val Gly Val Pro Gly Val Gly Val Pro
            355                 360                 365

Gly Val Gly Val Pro Gly Val Gly Val Pro Gly Val Gly Val Pro Gly
            370                 375                 380

Val Gly Val Pro Gly Ala Val Ser Pro Ala Ala Ala Ala Lys Ala Ala
385                 390                 395                 400

Ala Lys Ala Ala Lys Phe Gly Ala Arg Gly Gly Val Gly Val Gly Gly
            405                 410                 415

Ile Pro Thr Phe Gly Val Gly Pro Gly Gly Phe Pro Gly Ile Gly Asp
            420                 425                 430

Ala Ala Ala Ala Gln Ala Ala Ala Ala Ala Lys Ala Ala Lys Ile Gly
            435                 440                 445

Ala Gly Gly Val Gly Ala Leu Gly Gly Leu Val Pro Gly Ala Pro Gly
            450                 455                 460

Ala Ile Pro Gly Val Pro Gly Val Gly Gly Val Pro Gly Val Gly Ile
465                 470                 475                 480

Pro Ala Ala Ala Ala Ala Lys Ala Ala Ala Lys Ala Ala Gln Phe Gly
            485                 490                 495

Leu Gly Pro Gly Val Gly Val Ala Pro Gly Val Gly Val Val Pro Gly
500 505 510

Val Gly Val Val Pro Gly Val Gly Val Ala Pro Gly Ile Gly Leu Gly
515 520 525

Pro Gly Gly Val Ile Gly Ala Gly Val Pro Ala Ala Ala Lys Ser Ala
530 535 540

Ala Lys Ala Ala Ala Lys Ala Gln Phe Arg Ala Ala Ala Gly Leu Pro
545 550 555 560

Ala Gly Val Pro Gly Leu Gly Val Gly Val Gly Val Pro Gly Leu Gly
565 570 575

Val Gly Val Gly Val Pro Gly Leu Gly Val Gly Ala Gly Val Pro Gly
580 585 590

Leu Gly Ala Val Pro Gly Thr Leu Ala Ala Ala Lys Ala Ala Lys Phe
595 600 605

Gly Pro Gly Gly Val Gly Ala Leu Gly Gly Val Gly Asp Leu Gly Gly
610 615 620

Ala Gly Ile Pro Gly Gly Val Ala Gly Val Gly Pro Ala Ala Ala Ala
625 630 635 640

Ala Ala Ala Lys Ala Ala Val Gln Leu Val Pro Lys His Arg Asn Pro
645 650 655

His Ala Gly Leu Gly His Thr Ile Ser Trp Pro Pro Trp Pro Pro Phe
660 665 670

Pro Arg Pro Ile Ala Val Pro Tyr Val Arg Arg Leu Pro Pro Pro Pro
675 680 685

Tyr Trp Glu Gln Pro Ser Cys Ser Cys Gly Ile His Pro Pro Ile Cys
690 695 700

Pro Ser Val Arg Pro Ser Leu Ser Trp Phe Gly Arg Pro Ala Pro Leu
705 710 715 720

Ala Gly Trp Ala Pro Pro Pro Ser Thr Trp Leu Thr Cys His Gly Ser
725 730 735

Leu Gly Pro Ala Ser Thr Pro Ser His Thr Pro Leu Arg Arg Gly Pro
740 745 750

EP 3 107 556 B1

```
Glu Pro Leu Gly Val Lys Ser Cys Thr Ser Trp Gly Arg Arg Asn Leu
        755                 760             765

Arg Pro Asn Leu Asp Leu Pro Pro Arg Ser Thr Val Ser Pro Ser Pro
        770             775             780

Pro Arg Ala Thr Val Leu Gln Ser Ile Ser Pro Pro Pro Arg Pro Ser
785             790             795             800

Leu Cys Val Ser Leu
        805
```

<210> 16
<211> 705
<212> PRT
<213> Gallus gallus

<400> 16

```
Met Lys Leu Ile Leu Cys Thr Val Leu Ser Leu Gly Ile Ala Ala Val
1               5               10              15

Cys Phe Ala Ala Pro Pro Lys Ser Val Ile Arg Trp Cys Thr Ile Ser
            20              25              30

Ser Pro Glu Glu Lys Lys Cys Asn Asn Leu Arg Asp Leu Thr Gln Gln
        35              40              45

Glu Arg Ile Ser Leu Thr Cys Val Gln Lys Ala Thr Tyr Leu Asp Cys
        50              55              60

Ile Lys Ala Ile Ala Asn Asn Glu Ala Asp Ala Ile Ser Leu Asp Gly
65              70              75              80

Gly Gln Ala Phe Glu Ala Gly Leu Ala Pro Tyr Lys Leu Lys Pro Ile
            85              90              95

Ala Ala Glu Val Tyr Glu His Thr Glu Gly Ser Thr Thr Ser Tyr Tyr
            100             105             110

Ala Val Ala Val Val Lys Lys Gly Thr Glu Phe Thr Val Asn Asp Leu
            115             120             125

Gln Gly Lys Thr Ser Cys His Thr Gly Leu Gly Arg Ser Ala Gly Trp
        130             135             140

Asn Ile Pro Ile Gly Thr Leu Leu His Arg Gly Ala Ile Glu Trp Glu
145             150             155             160
```

66

```
Gly Ile Glu Ser Gly Ser Val Glu Gln Ala Val Ala Lys Phe Phe Ser
            165             170             175

Ala Ser Cys Val Pro Gly Ala Thr Ile Glu Gln Lys Leu Cys Arg Gln
            180             185             190

Cys Lys Gly Asp Pro Lys Thr Lys Cys Ala Arg Asn Ala Pro Tyr Ser
            195             200             205

Gly Tyr Ser Gly Ala Phe His Cys Leu Lys Asp Gly Lys Gly Asp Val
    210             215             220

Ala Phe Val Lys His Thr Thr Val Asn Glu Asn Ala Pro Asp Gln Lys
225             230             235             240

Asp Glu Tyr Glu Leu Leu Cys Leu Asp Gly Ser Arg Gln Pro Val Asp
            245             250             255

Asn Tyr Lys Thr Cys Asn Trp Ala Arg Val Ala Ala His Ala Val Val
            260             265             270

Ala Arg Asp Asp Asn Lys Val Glu Asp Ile Trp Ser Phe Leu Ser Lys
            275             280             285

Ala Gln Ser Asp Phe Gly Val Asp Thr Lys Ser Asp Phe His Leu Phe
            290             295             300

Gly Pro Pro Gly Lys Lys Asp Pro Val Leu Lys Asp Leu Leu Phe Lys
305             310             315             320

Asp Ser Ala Ile Met Leu Lys Arg Val Pro Ser Leu Met Asp Ser Gln
            325             330             335

Leu Tyr Leu Gly Phe Glu Tyr Tyr Ser Ala Ile Gln Ser Met Arg Lys
            340             345             350

Asp Gln Leu Thr Pro Ser Pro Arg Glu Asn Arg Ile Gln Trp Cys Ala
            355             360             365

Val Gly Lys Asp Glu Lys Ser Lys Cys Asp Arg Trp Ser Val Val Ser
    370             375             380

Asn Gly Asp Val Glu Cys Thr Val Val Asp Glu Thr Lys Asp Cys Ile
385             390             395             400

Ile Lys Ile Met Lys Gly Glu Ala Asp Ala Val Ala Leu Asp Gly Gly
            405             410             415
```

```
Leu Val Tyr Thr Ala Gly Val Cys Gly Leu Val Pro Val Met Ala Glu
        420             425         430

Arg Tyr Asp Asp Glu Ser Gln Cys Ser Lys Thr Asp Glu Arg Pro Ala
        435             440         445

Ser Tyr Phe Ala Val Ala Val Ala Arg Lys Asp Ser Asn Val Asn Trp
    450             455         460

Asn Asn Leu Lys Gly Lys Lys Ser Cys His Thr Ala Val Gly Arg Thr
465             470         475             480

Ala Gly Trp Val Ile Pro Met Gly Leu Ile His Asn Arg Thr Gly Thr
            485             490         495

Cys Asn Phe Asp Glu Tyr Phe Ser Glu Gly Cys Ala Pro Gly Ser Pro
        500             505         510

Pro Asn Ser Arg Leu Cys Gln Leu Cys Gln Gly Ser Gly Gly Ile Pro
        515             520         525

Pro Glu Lys Cys Val Ala Ser Ser His Glu Lys Tyr Phe Gly Tyr Thr
    530             535             540

Gly Ala Leu Arg Cys Leu Val Glu Lys Gly Asp Val Ala Phe Ile Gln
545             550             555             560

His Ser Thr Val Glu Glu Asn Thr Gly Gly Lys Asn Lys Ala Asp Trp
            565             570             575

Ala Lys Asn Leu Gln Met Asp Asp Phe Glu Leu Leu Cys Thr Asp Gly
        580             585             590

Arg Arg Ala Asn Val Met Asp Tyr Arg Glu Cys Asn Leu Ala Glu Val
        595             600             605

Pro Thr His Ala Val Val Val Arg Pro Glu Lys Ala Asn Lys Ile Arg
    610             615             620

Asp Leu Leu Glu Arg Gln Glu Lys Arg Phe Gly Val Asn Gly Ser Glu
625             630             635             640

Lys Ser Lys Phe Met Met Phe Glu Ser Gln Asn Lys Asp Leu Leu Phe
            645             650             655

Lys Asp Leu Thr Lys Cys Leu Phe Lys Val Arg Glu Gly Thr Thr Tyr
```

                    660                    665                    670

    Lys Glu Phe Leu Gly Asp Lys Phe Tyr Thr Val Ile Ser Ser Leu Lys
              675                    680                    685


    Thr Cys Asn Pro Ser Asp Ile Leu Gln Met Cys Ser Phe Leu Glu Gly
              690                    695                    700


    Lys
    705

<210> 17
<211> 517
<212> PRT
<213> Pisum sativum

<400> 17

```
Met Ala Lys Leu Leu Ala Leu Ser Leu Ser Phe Cys Phe Leu Leu Leu
1               5               10              15

Gly Gly Cys Phe Ala Leu Arg Glu Gln Pro Gln Gln Asn Glu Cys Gln
        20              25              30

Leu Glu Arg Leu Asp Ala Leu Glu Pro Asp Asn Arg Ile Glu Ser Glu
        35              40              45

Gly Gly Leu Ile Glu Thr Trp Asn Pro Asn Asn Lys Gln Phe Arg Cys
    50              55              60

Ala Gly Val Ala Leu Ser Arg Ala Thr Leu Gln Arg Asn Ala Leu Arg
65              70              75              80

Arg Pro Tyr Tyr Ser Asn Ala Pro Gln Glu Ile Phe Ile Gln Gln Gly
            85              90              95

Asn Gly Tyr Phe Gly Met Val Phe Pro Gly Cys Pro Glu Thr Phe Glu
        100             105             110

Glu Pro Gln Glu Ser Glu Gln Gly Glu Gly Arg Arg Tyr Arg Asp Arg
        115             120             125

His Gln Lys Val Asn Arg Phe Arg Glu Gly Asp Ile Ile Ala Val Pro
    130             135             140

Thr Gly Ile Val Phe Trp Met Tyr Asn Asp Gln Asp Thr Pro Val Ile
145             150             155             160

Ala Val Ser Leu Thr Asp Ile Arg Ser Ser Asn Asn Gln Leu Asp Gln
```

70

|  |  | 165 |  |  | 170 |  |  | 175 |  |
|---|---|---|---|---|---|---|---|---|---|

Met Pro Arg Arg Phe Tyr Leu Ala Gly Asn His Glu Gln Glu Phe Leu
            180               185               190

Gln Tyr Gln His Gln Gln Gly Gly Lys Gln Glu Gln Glu Asn Glu Gly
            195               200               205

Asn Asn Ile Phe Ser Gly Phe Lys Arg Asp Tyr Leu Glu Asp Ala Phe
            210               215               220

Asn Val Asn Arg His Ile Val Asp Arg Leu Gln Gly Arg Asn Glu Asp
225               230               235               240

Glu Glu Lys Gly Ala Ile Val Lys Val Lys Gly Gly Leu Ser Ile Ile
                245               250               255

Ser Pro Pro Glu Lys Gln Ala Arg His Gln Arg Gly Ser Arg Gln Glu
            260               265               270

Glu Asp Glu Asp Glu Glu Lys Gln Pro Arg His Gln Arg Gly Ser Arg
            275               280               285

Gln Glu Glu Glu Glu Asp Glu Asp Glu Glu Arg Gln Pro Arg His Gln
            290               295               300

Arg Arg Arg Gly Glu Glu Glu Glu Glu Asp Lys Lys Glu Arg Gly Gly
305               310               315               320

Ser Gln Lys Gly Lys Ser Arg Arg Gln Gly Asp Asn Gly Leu Glu Glu
                325               330               335

Thr Val Cys Thr Ala Lys Leu Arg Leu Asn Ile Gly Pro Ser Ser Ser
            340               345               350

Pro Asp Ile Tyr Asn Pro Glu Ala Gly Arg Ile Lys Thr Val Thr Ser
            355               360               365

Leu Asp Leu Pro Val Leu Arg Trp Leu Lys Leu Ser Ala Glu His Gly
            370               375               380

Ser Leu His Lys Asn Ala Met Phe Val Pro His Tyr Asn Leu Asn Ala
385               390               395               400

Asn Ser Ile Ile Tyr Ala Leu Lys Gly Arg Ala Arg Leu Gln Val Val
                405               410               415

71

```
Asn Cys Asn Gly Asn Thr Val Phe Asp Gly Glu Leu Glu Ala Gly Arg
        420             425             430

Ala Leu Thr Val Pro Gln Asn Tyr Ala Val Ala Ala Lys Ser Leu Ser
        435             440             445

Asp Arg Phe Ser Tyr Val Ala Phe Lys Thr Asn Asp Arg Ala Gly Ile
    450             455             460

Ala Arg Leu Ala Gly Thr Ser Ser Val Ile Asn Asn Leu Pro Leu Asp
465             470             475             480

Val Val Ala Ala Thr Phe Asn Leu Gln Arg Asn Glu Ala Arg Gln Leu
            485             490             495

Lys Ser Asn Asn Pro Phe Lys Phe Leu Val Pro Ala Arg Glu Ser Glu
        500             505             510

Asn Arg Ala Ser Ala
        515
```

**Description**

La présente invention concerne l'utilisation de compositions pour la prévention et/ou le traitement de pathologies liées à l'alpha-glucosidase.

La présente invention concerne l'utilisation de compositions pour la prévention et/ou le traitement du diabète de type 2.

Le diabète de type 2 est la forme la plus fréquente de diabète. Il s'agit d'une pathologie métabolique chronique, progressive, caractérisée par une hyperglycémie chronique, c'est-à-dire une concentration en sucre dans le sang (glycémie) anormalement élevée. Bien que la résistance à l'insuline et sa sécrétion inadéquate en réponse à un état métabolique donné constituent la principale cause, d'autres facteurs peuvent contribuer à un état d'hyperglycémie chronique, par exemple la sédentarité.

Le diabète de type 2 constitue un problème majeur de santé publique. Dans la majorité des pays industrialisés, la prévalence des cas connus de diabète se situe entre 6 et 7% pour les personnes de la tranche d'âge comprise de 45 à 64 ans, augmentant progressivement jusqu'à plus de 20% pour les personnes de 80 ans et plus.

Différentes classes de produits pour traiter et/ou prévenir le diabète de type 2 sont déjà connues. Des inhibiteurs d'enzyme ont notamment été proposés, en particulier des inhibiteurs de la dipeptidyl peptidase IV ou de l'alpha-glucosidase.

Bien que la dipeptidyl peptidase IV et l'alpha-glucosidase agissent toutes les deux pour diminuer la glycémie post-prandiale, ces deux enzymes ont des mécanismes d'action différents qui ne sont pas liés. Il n'est ainsi pas évident qu'un inhibiteur de l'alpha-glucosidase inhibe la dipeptidyl peptidase IV, et inversément.

En effet, la dipeptidyl peptidase IV (DPP-IV) est une glycoprotéine transmembranaire multifonctionnelle qui est exprimée dans la plupart des tissus, en particulier ceux impliqués dans la dégradation des peptides (notamment le rein, le tube digestif, le foie et les voies biliaires, l'utérus, la prostate et la peau). Cette glycoprotéine possède trois propriétés biologiques : elle lie la désaminase de l'adénosine (ADA), elle contribue à la liaison des cellules à la matrice extracellulaire, et c'est une peptidase.

En tant que peptidase, la dipeptidyl peptidase IV clive notamment des hormones telles que le Glucagon-Like Peptide 1 (GLP-1). GLP-1 est une incrétine, à savoir une hormone intestinale, sécrétée par les cellules L de l'iléon en réponse à un repas. L'un de ses rôles est de favoriser la sécrétion d'insuline pour diminuer la glycémie post-prandiale. Cependant, GLP-1 est rapidement dégradé dans le plasma par la DPP-IV et présente ainsi une courte demi-vie d'environ 1 ou 2 minutes.

L'inhibition de la dipeptidyl peptidase IV permet d'augmenter la demi-vie de circulation du GLP-1, qui est ainsi actif plus longtemps, ce qui se traduit par une sécrétion prolongée d'insuline pour diminuer la glycémie post-prandiale.

Des inhibiteurs de la dipeptidyl peptidase IV ont ainsi été proposés, notamment des peptides issus d'hydrolysats de protéines tels que décrits dans la demande internationale WO 2006/068480.

L'enzyme alpha-glucosidase peut être subdivisée en quatre enzymes intervenant dans le métabolisme du glucose (la maltase, la saccharrase, la glucoamylase et l'isomaltase). Toutes ces enzymes sont situées à la surface des villosités de l'intestin grêle et transforment les polysaccharides complexes en monosaccharides résorbables (le glucose).

En effet, les polysaccharides absorbés sont dégradés par l'amylase salivaire et pancréatique en disaccharides (saccharose, lactose ou maltose) puis en monosaccharides absorbables par les alpha-glucosidases ou les bêta-glucosidases (lactase et invertase).

La maltase et l'isomaltase catalysent l'hydrolyse du maltose et des dextrines en glucose.

La saccharase scinde le saccharose en une molécule de fructose et une de glucose.

La glucoamylase catalyse l'hydrolyse du maltotriose et des dextrines en glucose.

La lactase favorise la dissociation du lactose en glucose et galactose.

L'invertase est une bêta-fructofuranosidase, elle appartient à la famille des saccharases et à ce titre hydrolyse le saccharose.

Parmi les inhibiteurs de l'alpha-glucosidase connus, il existe des peptides bioactifs tels que les peptides Ile-Ile-Ser-Ile-Gly ; Ile-Ile-Ser-Ile-Gly-Arg ; Val-Phe-Ile-Lys-Ala-Ala ; Val-Phe-Ile-Lys-Ala-Ala-Ala et Val-Phe-Ile-Lys-Ala, tels que décrits dans la demande japonaise JPH 1029 2000, ou bien des composés chimiques tels que l'acarbose (commercialisé en France par Bayer AG sous le nom de Glucor et Glucobay® en Europe) et le miglitol (commercialisé sous le nom de Diastabol® en Europe).

L'acarbose et le miglitol inhibent tous deux l'alpha-glucosidase de façon compétitive et réversible, et notamment la saccharase (leur $IC_{50}$ envers la saccharase est respectivement de 0,5 $\mu$M et 0,19 $\mu$M). Ils inhibent le dernier stade de la digestion des sucres qui, ne pouvant être absorbés, continuent leur périple dans l'intestin et subissent la fermentation colique bactérienne en acides gras volatiles ou sont éliminés dans les selles. Ils permettent donc de ralentir la digestion des sucres et diminuer leur absorption, ce qui entraine une diminution de la glycémie post-prandiale à court terme et celle de l'hémoglobine glyquée à moyen-terme.

Ces deux composés présentent néanmoins des effets secondaires, dus à la stagnation et à la fermentation de sucres non digérés dans l'intestin, notamment des troubles digestifs tels que des douleurs abdominales, des ballonnements, des flatulences, et des diarrhées. De plus, ces composés ne permettent en moyenne de n'abaisser l'hémoglobine glyquée que de 0,5 à 1%, ce qui se traduit par une diminution de la glycémie plasmatique moyenne de 0,17 à 0,35 g/L.

En comparaison, des inhibiteurs de l'alpha-amylase (une enzyme responsable de l'hydrolyse des glucides à longue chaine) utilisés comme nutraceutique permettent une diminution plus importante de l'hémoglobine glyquée. Ainsi, la consommation de MealShape®, un extrait de cannelle commercialisé par la société DIALPHA, à la dose de 500 mg, deux prises, avant la consommation d'un repas riche en glucides (pain blanc), induit une diminution de la glycémie post-prandiale d'environ 20% (mesure sur 1 heure de l'aire sous la courbe, comparativement à un placebo chez des volontaires sains ; données DIALPHA rendues publiques mais non publiées dans un journal scientifique à comité de lecture à ce jour). MealShape® a fait l'objet de la demande internationale WO 2012/085266 A2. De même, la prise de 3000 mg de StarchLite® sous forme de poudre, dans des conditions similaires, semble induire une diminution de l'index glycémique de certains aliments d'environ 30% (Udani JK et al. Nutr J 2009, 8 :52). StarchLite® est un extrait de haricot blanc (*Phaseolus vulgaris*) commercialisé par la société Ingredia Nutritional.

MealShape® et StarchLite® ne sont néanmoins utilisés que dans le domaine nutraceutique ou alimentaire, et non dans le domaine thérapeutique. De plus, il est décrit dans la littérature que la consommation de doses importantes de cannelle ou d'extraits de cannelle peut se révéler toxique (la dose létale médiane ($DL_{50}$) est de 0,196 g/kg chez la souris).

Par ailleurs, les peptides bioactifs inhibiteurs d'enzymes sont également utilisés dans un domaine autre que la prévention et/ou le traitement du diabète de type 2. A ce sujet, des peptides VAP (un tripeptide Valine-Alanine-Proline) et AP (un dipeptide Alanine-Proline) sont notamment connus pour leur activité inhibitrice de l'enzyme de conversion de l'angiotensine (voir par exemple les articles Maruyama et al., Agric. Biol. Chem., 51 (6), 1581-1586, 1987 et Cheung et al., J. Biol. Chem. 1980, 255 :401-407 ou la demande internationale WO 2006/084560).

Il existe ainsi un réel besoin de fournir des molécules hypoglycémiantes pour le traitement et/ou la prévention du diabète de type 2 présentant moins d'effets secondaires que les traitements existants.

Il existe aussi un réel besoin de fournir des molécules hypoglycémiantes pour le traitement et/ou la prévention du diabète de type 2, plus efficaces que celles de l'art antérieur.

Il existe aussi un réel besoin de fournir des molécules hypoglycémiantes pouvant être utilisées dans le traitement et/ou la prévention du diabète de type 2, à la fois dans le domaine thérapeutique, mais également dans le domaine alimentaire et/ou nutraceutique.

La présente invention vise ainsi à fournir des molécules hypoglycémiantes plus efficaces et plus naturelles que celles de l'art antérieur.

La présente invention vise aussi à fournir des molécules hypoglycémiantes pouvant être utilisées à la fois dans le domaine thérapeutique, mais également dans le domaine alimentaire et/ou nutraceutique. La présente invention est définie dans les revendications.

Ainsi la présente invention a pour objet des compositions comprenant des peptides inhibiteurs de l'alpha-glucosidase pour la prévention et/ou le traitement de pathologies liées à l'alpha-glucosidase, notamment le diabète de type 2.

La présente invention a également pour objet une composition pharmaceutique comprenant de tels peptides.

La présente description a également pour objet l'utilisation de tels peptides pour la préparation d'une composition nutraceutique ou d'un complément alimentaire.

La présente invention a également pour objet une composition nutraceutique ou une composition alimentaire comprenant de tels peptides.

Enfin, la présente description a pour objet des compositions comprenant des hydrolysats d'au moins une protéine présentant dans sa séquence en acides aminés des motifs d'acides aminés inhibiteurs de l'alpha-glucosidase pour la prévention et/ou le traitement de pathologies liées à l'alpha-glucosidase, notamment le diabète de type 2.

La présente description a également pour objet une composition pharmaceutique comprenant de tels hydrolysats.

La présente description a également pour objet l'utilisation de tels hydrolysats pour la préparation d'une composition nutraceutique ou d'un complément alimentaire.

La présente description a également pour objet une composition nutraceutique ou une composition alimentaire comprenant de tels hydrolysats.

Dans un premier aspect, la présente description concerne ainsi une composition comprenant ou étant constituée d'au moins un peptide XAP, dans lequel X représente l'ensemble vide ou une valine, pour son utilisation dans la prévention et/ou le traitement de pathologies liées à l'alpha-glucosidase.

Dans un premier aspect, la présente invention concerne ainsi une composition comprenant ou étant constituée d'au moins un peptide AP pour son utilisation dans la prévention et/ou le traitement du diabète de type 2.

L'expression « X représente l'ensemble vide ou une valine » signifie que XAP peut être un tripeptide VAP ou un dipeptide AP.

Le tripeptide VAP est constitué de l'enchainement des trois acides aminés, dans l'ordre suivant : valine puis alanine et puis proline. Les acides aminés sont liés entre eux par des liaisons peptidiques. La proline est en position Cterminale.

Le dipeptide AP est constitué de l'enchainement des deux acides aminés, dans l'ordre suivant : alanine et puis proline. Les acides aminés sont liés entre eux par des liaisons peptidiques. La proline est en position Cterminale.

Lesdits peptides VAP et AP peuvent agir sur la glycémie post-prandiale, et peuvent permettre notamment de la diminuer.

Dans lesdits peptides VAP et AP, les acides aminés valine, alanine et proline peuvent être soit lévogyres, soit dextrogyres.

Les résidus acides aminés successifs des peptides selon l'invention sont de préférence tous constitués par leurs isomères lévogyres. Un ou plusieurs des résidus acides aminés des peptides susdits peuvent néanmoins également être sous forme dextrogyre. Il en résulte des produits moins biodégradables.

Dans un aspect particulier et préféré de l'invention, le peptide utilisé est le peptide AP.

Dans un aspect particulier de l'invention, l'alpha-glucosidase est une maltase ou une saccharase.

Les Inventeurs ont ainsi trouvé de façon surprenante que les peptides VAP ou AP présentent une très forte activité inhibitrice de l'alpha-glucosidase, en particulier la maltase, et présente notamment une concentration inhibitrice médiane de l'alpha-glucosidase, en particulier la maltase, d'environ 600 fois et d'environ 900 fois plus faible par rapport à celle des deux inhibiteurs de référence de l'alpha-glucosidase, à savoir l'acarbose et le miglitol.

Dans un aspect particulier, la présente description concerne ainsi une composition comprenant ou étant constituée d'au moins un peptide XAP, dans lequel X représente l'ensemble vide ou une valine, pour son utilisation en tant qu'inhibiteur de l'alpha-glucosidase.

En effet, l'invention concerne également les peptides XAP, dans lequel X représente l'ensemble vide ou une valine, pour leur utilisation en tant qu'inhibiteur de l'alpha-glucosidase.

L'activité inhibitrice des peptides VAP et AP peut, par exemple, être mesurée *in vitro* selon le protocole suivant :

- Solubiliser dans de l'eau déionisée contenant 10% de DMSO (diméthyl sulfoxide) les peptides à tester ou la molécule témoin (par exemple, en l'espèce les peptides VAP ou AP et l'acarbose ou le miglitol) ;
- Mélanger 20 μL d'alpha-glucosidase dans du tampon 0,1 mol/L phosphate de sodium (pH 6,8) à une concentration finale de 0,2 U/mL avec 8 μL de l'échantillon de peptide ou d'acarbose ou de miglitol à différentes concentrations (0,01 à 50 mmol/L). L'acarbose ou le miglitol, inhibiteurs synthétiques commerciaux, considérés comme inhibiteur référant de l'alpha-glucosidase, sont ici utilisés comme contrôle positif ;
- Incuber à 37°C pendant 20 minutes ;
- Ajouter 20 μL du substrat p-NPG (le p-nitro phenyl glucopyranoside) à 2,5 mM (préparé dans le même tampon précédemment cité) au mélange afin d'initier la réaction ;
- Incuber pendant 30 minutes à 37°C ;
- Mettre fin à la réaction par l'ajout de 80 μL d'une solution de carbonate de sodium ($Na_2CO_3$) à 0,3M ;
- Mesurer la quantité de produit formé (le p-nitro phenyl (p-NP) de couleur jaune) par spectrophotométrie (absorbance à 410 nm, VersaMax™, Microplate Reader. De préférence, le test est réalisé en microplaque de 96 puits et est réalisés en triplicata.
- Calculer le pourcentage d'inhibition selon l'équation ci-dessous :

$$\% \, d'inhibition = \left[1 - \frac{(DO \, test \, echantillon - DO \, blanc \, test)}{(DO \, test \, controle - DO \, blanc \, controle)}\right] *100$$

dans laquelle :

- DO test échantillon correspond à la densité optique obtenue pour le mélange « échantillon + enzyme + substrat »
- DO blanc test correspond à la densité optique obtenue pour le mélange « échantillon + tampon »
- DO test contrôle correspond à la densité optique obtenue pour le mélange « tampon + enzyme + substrat »
- DO blanc contrôle correspond à la densité optique obtenue pour le tampon.

L'activité inhibitrice des peptides VAP et AP peut, par exemple, être mesurée *in vivo* selon le protocole suivant : L'activité inhibitrice des peptides AP et VAP et l'effet des peptides AP et VAP sur la réponse glycémique, peuvent être mesurés lors d'un test oral de tolérance au saccharose et au maltose *in vivo* chez la souris db/db.

Des souris âgées de 4 semaines sont utilisées et chaque souris est son propre témoin.

Cinq tests oraux de tolérance au saccharose et/ou maltose (4 g/kg) sont réalisés sur chaque souris avec un minimum de 72 h d'intervalle. L'ordre est déterminé de façon à annihiler un effet potentiellement confondant d'une modification de la composition corporelle des souris durant les 3 semaines d'étude.

Les 5 tests sont les suivants :

- Un test contrôle (comprenant une solution saline à 0,9%) ;
- Un test avec le peptide AP (à une concentration de 500 mg/kg) ;
- Un test avec le peptide VAP (à une concentration de 500 mg/kg) ;
- Un test avec le peptide AP (à une concentration de 500 mg/kg) et avec le peptide VAP (à une concentration de 500 mg/kg) ;
- Un test avec l'acarbose (à une concentration de 10 mg/kg). Ce dernier test avec l'acarbose est facultatif pour déterminer l'activité inhibitrice des peptides AP et VAP.

Le saccharose ou le maltose et les produits tests sont dilués dans une solution saline 0,9%, puis administrés directement par voie gastrique.

Cinq minutes après l'administration par voie gastrique, un premier prélèvement sanguin à la queue est réalisé afin de déterminer la glycémie (t = 0) ; puis 6 autres prélèvements sont effectués après 15, 30, 45, 60, 90 et 120 minutes.

Le critère d'évaluation principal est la mesure de l'aire sous la courbe glycémique sur les 2 heures suivant l'administration du saccharose ou du maltose (AUC, aera under the curve, 0 - 120 minutes, en g*min/L).

L'alpha-glucosidase pouvant être utilisée aux fins des protocoles mentionnés ci-dessus peut par exemple être l'alpha-glucosidase recombinante de *Saccharomyces cerevisiae* qui est une maltase.

Tout autre protocole jugé équivalent ou plus approprié par l'homme du métier pourra être utilisé.

Dans un aspect de la description, les pathologies liées à l'alpha-glucosidase sont par exemple le diabète de type 2, la mucoviscidose (voir la demande internationale WO2005046672 A2), l'hépatite C (voir la demande internationale WO2006096769), et l'adipose (voir le brevet EP0638317 B1).

Dans un aspect plus particulier de l'invention, la pathologie liée à l'alpha-glucosidase est le diabète de type 2.

Dans un aspect particulier de l'invention, lesdits peptides sont utilisés chez des patients qui présentent ou risquent de présenter un pré-diabète, notamment un pré-diabète de type 2. Lesdits patients ne souffrent pas obligatoirement de maladies cardiovasculaires, notamment d'hypertension artérielle, de maladie coronarienne ou d'insuffisance cardiaque chronique. En effet, les patients qui présentent un pré-diabète, notamment un pré-diabète de type 2, ne sont pas nécessairement atteints d'hypertension-artérielle (HTA).

Le pré-diabète correspond à un état où les taux de glycémie sont plus élevés que la normale sans être suffisamment élevé pour définir un diabète. Le pré-diabète peut faire partie d'un concept médical appelé syndrome métabolique. La définition du syndrome métabolique fait intervenir différentes données dont l'obésité de type abdominale, une anomalie des paramètres lipidiques, une hypertension artérielle... Le syndrome métabolique est lui-même un facteur de risque d'apparition de pathologies cardiovasculaires. Toutes les personnes présentant un pré-diabète ne développeront pas forcément un diabète. Mais ce risque est multiplié par 10 par rapport à un sujet sain lorsque l'on a un syndrome métabolique.

Le pré-diabète se définit par une glycémie à jeun supérieure ou égale à 1g/l (notamment comprise entre 1 g/l et 1,26 g/l et ceci quelque soit les normes du laboratoire), ou une intolérance au glucose.

Un diabète est diagnostiqué lorsque la glycémie à jeun est égale ou supérieure à 1.26 g/l, lors de 2 prélèvements successifs ou lorsque la glycémie est égale ou supérieure à 2 g/l à n'importe quel moment de la journée.

Dans un autre aspect particulier de la description, ladite composition pour son utilisation dans la prévention et/ou le traitement de pathologies liées à l'alpha-glucosidase comprend au moins un peptide VAP et/ou au moins un peptide AP. Cela signifie que la composition selon la présente invention comprend au moins un peptide VAP ou au moins un peptide AP, ou que ladite composition comprend au moins un peptide VAP et au moins un peptide AP.

Dans un autre aspect particulier de l'invention, ladite composition pour son utilisation dans la prévention et/ou le traitement du diabète de type 2 comprend au moins un peptide AP ou au moins un peptide AP et VAP.

Dans un autre aspect particulier de la description, ladite composition pour son utilisation dans la prévention et/ou le traitement de pathologies liées à l'alpha-glucosidase comprend ou est constituée d'au moins un peptide XAP, dans lequel X représente l'ensemble vide ou une valine, en association avec au moins un peptide de type APX'.

Dans un autre aspect particulier de l'invention, ladite composition pour son utilisation dans la prévention et/ou le traitement du diabète de type 2 comprend ou est constituée d'au moins un peptide AP en association avec au moins un peptide de type APX'.

L'expression « peptide de type APX' » correspond à un peptide dans lequel A est un acide aminé alanine, P est un acide aminé proline, et X' correspond à un acide aminé ou à un groupe d'acides aminés choisi parmi les 20 acides aminés universellement distribués chez les êtres vivants (Alanine ; Arginine ; Asparagine ; Aspartate ou Acide aspartique ; Cystéine ; Glutamate ou Acide glutamique ; Glutamine ; Glycine ; Histidine ; Isoleucine ; Leucine ; Lysine ; Méthionine ; Phénylalanine ; Proline ; Sérine ; Thréonine ; Tryptophane ; Tyrosine ; Valine). Les acides aminés sont liés entre eux par des liaisons peptidiques.

Dans un aspect particulier de l'invention, le peptide de type APX' est choisi parmi APFPE (SEQ ID NO : 1) ou APFPEVF (SEQ ID NO : 2).

Ainsi, dans un aspect particulier de la description, ladite composition pour son utilisation dans la prévention et/ou le traitement de pathologies liées à l'alpha-glucosidase comprend ou est constituée d'au moins un peptide XAP, dans lequel X représente l'ensemble vide ou une valine, en association avec au moins un peptide APFPE et/ou APFPEVF.

Ainsi, dans un aspect particulier de l'invention, ladite composition pour son utilisation dans la prévention et/ou le traitement du diabète de type 2 comprend ou est constituée d'au moins un peptide AP en association avec au moins un peptide APFPE et/ou APFPEVF.

Ainsi, dans un aspect particulier, la composition pour son utilisation dans la prévention et/ou le traitement de pathologies liées à l'alpha-glucosidase comprend ou est constituée au moins des associations de peptides suivantes :

- AP + APFPE (SEQ ID NO : 1), ou
- AP + APFPEVF (SEQ ID NO : 2), ou
- VAP + APFPE(SEQ ID NO : 1), ou
- VAP + APFPEVF (SEQ ID NO : 2), ou
- AP + APFPE (SEQ ID NO : 1) + APFPEVF (SEQ ID NO : 2), ou
- VAP + APFPE (SEQ ID NO : 1) + APFPEVF (SEQ ID NO : 2), ou
- AP + VAP + APFPE (SEQ ID NO : 1) + APFPEVF (SEQ ID NO : 2), ou
- AP + VAP + APFPE (SEQ ID NO : 1), ou
- AP + VAP + APFPEVF (SEQ ID NO : 2).

Dans un autre aspect de la description, ladite composition pour son utilisation dans la prévention et/ou le traitement de pathologies liées à l'alpha-glucosidase comprend ou est constituée d'environ 0,06 mg/kg à environ 40,0 mg/kg de peptide XAP.

Dans un autre aspect de l'invention, ladite composition pour son utilisation dans la prévention et/ou le traitement du diabète de type 2 comprend ou est constituée d'environ 0,06 mg/kg à environ 40,0 mg/kg de peptide AP.

Dans un autre aspect de la description, ladite composition pour son utilisation dans la prévention et/ou le traitement de pathologies liées à l'alpha-glucosidase comprend ou est constituée d'environ 0,06 mg/kg à environ 30,0 mg/kg de peptide XAP ou d'environ >30,0 mg/kg à 40,0 mg/kg de peptide XAP, et dans un mode avantageux environ 0,06 mg/kg à environ 0,099 mg/kg.

Dans un autre aspect de l'invention, ladite composition pour son utilisation dans la prévention et/ou le traitement du diabète de type 2 comprend ou est constituée d'environ 0,06 mg/kg à environ 30,0 mg/kg de peptide AP ou d'environ >30,0 mg/kg à 40,0 mg/kg de peptide AP, et dans un mode avantageux environ 0,06 mg/kg à environ 0,099 mg/kg.

Les doses ont été établies en considérant un patient adulte de 75 kg.

L'expression « environ 0,06 mg/kg à environ 30,0 mg/kg » doit s'entendre comme pouvant couvrir des valeurs quelque peu inférieures à 0,06 mg/kg, par exemple 0,059 mg/kg, ou bien quelque peu supérieures à 30,0 mg/kg, par exemple 30,1 mg/kg, notamment pour un patient d'un poids différent de 75 kg.

L'expression « environ >30,0 mg/kg à environ 40,0 mg/kg » doit s'entendre comme pouvant couvrir des valeurs quelque peu supérieures à 40,0 mg/kg, par exemple 40,1 mg/kg, notamment pour un patient d'un poids différent de 75 kg.

L'expression « environ >30,0 mg/kg signifie des valeurs strictement supérieures à 30,0 mg/kg.

L'expression « environ 0,06 mg/kg à environ 30,0 mg/kg » signifie aussi que la composition selon la présente invention peut comprendre toutes les valeurs de 0,06 mg/kg à 30,0 mg/kg, notamment de 0,06 mg/kg à 0,08 mg/kg ; de 0,06 mg/kg à 0,09 mg/kg ; de 0,06 mg/kg à 0,1 mg/kg ; de 0,06 mg/kg à 0,5 mg/kg ; de 0,06 mg/kg à 1,0 mg/kg de 0,06 mg/kg à 2,0 mg/kg ; de 0,06 mg/kg à 5,0 mg/kg ; de 0,06 mg/kg à 10,0 mg/kg ; de 0,06 mg/kg à 15,0 mg/kg ; de 0,06 mg/kg à 20,0 mg/kg ; de 0,06 mg/kg à 25,0 mg/kg ; de 0,06 mg/kg à 30,0 mg/kg, de 0,2 mg/kg à 0,4 mg/kg ; de 0,2 à 0,5 mg/kg ; de 0,6 mg/kg à 0,9 mg/kg ; de 0,6 mg/kg à 1 mg/kg ; de 2 mg/kg à 3 mg/kg ou 4 mg/kg ou 5 mg/kg ou 6 mg/kg ou 7 mg/kg ou 8 mg/kg ou 9 mg/kg ou 10 mg/kg ou 11 mg/kg ou 12 mg/kg ou 13 mg/kg ou 14 mg/kg ou 15 mg/kg ou 16 mg/kg ou 17 mg/kg ou 18 mg/kg ou 19 mg/kg ou 20 mg/kg ou 21 mg/kg ou 22 mg/kg ou 23 mg/kg ou 24 mg/kg ou 25 mg/kg ou 26 mg/kg ou 27 ou 28 mg/kg ou 29 mg/kg ou 30 mg/kg.

L'expression « environ >30,0 mg/kg à environ 40,0 mg/kg » signifie aussi que la composition selon la présente invention peut comprendre toutes les valeurs supérieures à 30,0 mg/kg à environ 40,0 mg/kg, notamment 31 mg/kg, 32 mg/kg, 33 mg/kg, 34 mg/kg, 35 mg/kg, 36 mg/kg, 37 mg/kg, 38 mg/kg, 39 mg/kg et 40 mg/kg.

Dans un aspect, la description concerne ainsi une composition pour son utilisation dans la prévention et/ou le traitement de pathologies liées à l'alpha-glucosidase, comprenant ou étant constituée d'environ 0,06 mg/kg à environ 30,0 mg/kg de peptide VAP ou d'environ >30,0 mg/kg à 40,0 mg/kg de peptide VAP, et/ou comprenant ou étant constituée d'environ 0,06 mg/kg à environ 30,0 mg/kg de peptide AP ou d'environ >30,0 mg/kg à 40,0 mg/kg de peptide AP, et/ou comprenant ou étant constituée d'environ 0,06 mg/kg à environ 30,0 mg/kg de peptides VAP et AP ou d'environ >30,0 mg/kg à 40,0 mg/kg de peptides VAP et AP.

Dans un aspect, l'invention concerne ainsi une composition pour son utilisation dans la prévention et/ou le traitement du diabète de type 2, comprenant ou étant constituée d'environ 0,06 mg/kg à environ 30,0 mg/kg de peptide AP ou d'environ >30,0 mg/kg à 40,0 mg/kg de peptide AP, et/ou comprenant ou étant constituée d'environ 0,06 mg/kg à environ 30,0 mg/kg de peptides VAP et AP ou d'environ >30,0 mg/kg à 40,0 mg/kg de peptides VAP et AP.

Dans un aspect particulier, ladite composition pour son utilisation dans la prévention et/ou le traitement de pathologies liées à l'alpha-glucosidase comprend aussi ou est constituée d'environ 0,06 mg/kg à environ 0,099 mg/kg de peptide VAP, et/ou ladite composition pour son utilisation dans la prévention et/ou le traitement de pathologies liées à l'alpha-glucosidase comprend aussi ou est constituée d'environ 0,06 mg/kg à environ 0,099 mg/kg de peptide AP, et/ou ladite composition pour son utilisation dans la prévention et/ou le traitement de pathologies liées à l'alpha-glucosidase comprend aussi ou est constituée d'environ 0,06 mg/kg à environ 0,099 mg/kg de peptides VAP et AP.

Dans un autre aspect particulier, ladite composition pour son utilisation dans la prévention et/ou le traitement de pathologies liées à l'alpha-glucosidase comprend aussi ou est constituée d'environ 0,1 mg/kg à environ 30,0 mg/kg de peptide VAP ou d'environ >30,0 mg/kg à 40,0 mg/kg de peptide VAP, et/ou ladite composition pour son utilisation dans la prévention et/ou le traitement de pathologies liées à l'alpha-glucosidase comprend aussi ou est constituée d'environ 0,1 mg/kg à environ 30,0 mg/kg de peptide AP ou d'environ >30,0 mg/kg à 40,0 mg/kg de peptide AP, et/ou ladite composition pour son utilisation dans la prévention et/ou le traitement de pathologies liées à l'alpha-glucosidase comprend aussi ou est constituée d'environ environ 0,1 mg/kg à environ 30,0 mg/kg de peptides VAP et AP ou d'environ >30,0 mg/kg à 40,0 mg/kg de peptides VAP et AP.

Dans un aspect particulier de la description, ladite composition pour son utilisation dans la prévention et/ou le traitement de pathologies liées à l'alpha-glucosidase comprend ainsi ou est constituée d'environ 0,06 mg/kg à environ 30,0 mg/kg de peptide VAP notamment environ 0,06 mg/kg à environ 0,099 mg/kg ou notamment environ 0,1 mg/kg à environ 30,0 mg/kg, ou ladite composition pour son utilisation dans la prévention et/ou le traitement de pathologies liées à l'alpha-glucosidase comprend ou est constituée d'environ >30,0 mg/kg à 40,0 mg/kg de peptide VAP.

Dans un autre aspect particulier de la description ladite composition pour son utilisation dans la prévention et/ou le traitement de pathologies liées à l'alpha-glucosidase comprend ainsi ou est constituée d'environ 0,06 mg/kg à environ 30,0 mg/kg de peptide AP, notamment environ 0,06 mg/kg à environ 0,099 mg/kg ou notamment environ 0,1 mg/kg à environ 30,0 mg/kg, ou ladite composition pour son utilisation dans la prévention et/ou le traitement de pathologies liées à l'alpha-glucosidase comprend ou est constituée d'environ >30,0 mg/kg à 40,0 mg/kg de peptide AP.

Dans un autre aspect particulier de l'invention, ladite composition pour son utilisation dans la prévention et/ou le traitement du diabète de type 2 comprend ainsi ou est constituée d'environ 0,06 mg/kg à environ 30,0 mg/kg de peptide AP, notamment environ 0,06 mg/kg à environ 0,099 mg/kg ou notamment environ 0,1 mg/kg à environ 30,0 mg/kg, ou ladite composition pour son utilisation dans la prévention et/ou le traitement du diabète de type 2 comprend ou est constituée d'environ >30,0 mg/kg à 40,0 mg/kg de peptide AP.

Dans un autre aspect particulier de la description, ladite composition pour son utilisation dans la prévention et/ou le traitement de pathologies liées à l'alpha-glucosidase comprend ainsi ou est constituée d'environ 0,06 mg/kg à environ 30,0 mg/kg de peptides VAP et AP, notamment environ 0,06 mg/kg à environ 0,099 mg/kg ou notamment environ 0,1 mg/kg à environ 30,0 mg/kg, ou ladite composition pour son utilisation dans la prévention et/ou le traitement de pathologies liées à l'alpha-glucosidase comprend ou est constituée d'environ >30,0 mg/kg à 40,0 mg/kg de peptides VAP et AP.

Dans un autre aspect particulier de l'invention, ladite composition pour son utilisation dans la prévention et/ou le traitement du diabète de type 2 comprend ainsi ou est constituée d'environ 0,06 mg/kg à environ 30,0 mg/kg de peptides VAP et AP, notamment environ 0,06 mg/kg à environ 0,099 mg/kg ou notamment environ 0,1 mg/kg à environ 30,0 mg/kg, ou ladite composition pour son utilisation dans la prévention et/ou le traitement du diabète de type 2 comprend ou est constituée d'environ >30,0 mg/kg à 40,0 mg/kg de peptides VAP et AP.

Dans encore un autre aspect de la description, ladite composition pour son utilisation dans la prévention et/ou le traitement de pathologies liées à l'alpha-glucosidase comprend aussi ou est constituée d'environ 0,06 mg/kg à environ 30,0 mg/kg de peptide VAP, notamment environ 0,06 mg/kg à environ 0,099 mg/kg ou notamment environ 0,1 mg/kg à environ 30,0 mg/kg, et ladite composition pour son utilisation dans la prévention et/ou le traitement de pathologies liées à l'alpha-glucosidase comprend aussi ou est constituée d'environ 0,06 mg/kg à environ 30,0 mg/kg de peptide AP, notamment environ 0,06 mg/kg à environ 0,099 mg/kg ou environ 0,1 mg/kg à environ 30,0 mg/kg.

Dans encore un autre aspect de l'invention, ladite composition pour son utilisation dans la prévention et/ou le traitement du diabète de type 2 comprend aussi ou est constituée d'environ 0,06 mg/kg à environ 30,0 mg/kg de peptide VAP, notamment environ 0,06 mg/kg à environ 0,099 mg/kg ou notamment environ 0,1 mg/kg à environ 30,0 mg/kg, et ladite composition pour son utilisation dans la prévention et/ou le traitement du diabète de type 2 comprend aussi ou est constituée d'environ 0,06 mg/kg à environ 30,0 mg/kg de peptide AP, notamment environ 0,06 mg/kg à environ 0,099 mg/kg ou environ 0,1 mg/kg à environ 30,0 mg/kg.

Dans encore un autre aspect de la description, ladite composition pour son utilisation dans la prévention et/ou le traitement de pathologies liées à l'alpha-glucosidase comprend aussi ou est constituée d'environ >30,0 mg/kg à 40,0 mg/kg de peptide VAP, et ladite composition pour son utilisation dans la prévention et/ou le traitement de pathologies liées à l'alpha-glucosidase comprend aussi ou est constituée d'environ >30,0 mg/kg à 40,0 mg/kg mg/kg de peptide AP.

Dans encore un autre aspect de l'invention, ladite composition pour son utilisation dans la prévention et/ou le traitement du diabète de type 2 comprend aussi ou est constituée d'environ >30,0 mg/kg à 40,0 mg/kg de peptide VAP, et ladite composition pour son utilisation dans la prévention et/ou le traitement de du diabète de type 2 comprend aussi ou est constituée d'environ >30,0 mg/kg à 40,0 mg/kg mg/kg de peptide AP.

Dans un autre aspect particulier, ladite composition pour son utilisation dans la prévention et/ou le traitement de pathologies liées à l'alpha-glucosidase comprend aussi ou est constituée de 13,3 mg/kg de peptide XAP, notamment de peptide AP.

Dans un autre aspect, ladite composition pour son utilisation dans la prévention et/ou le traitement de pathologies liées à l'alpha-glucosidase est sous forme unitaire et comprend ou est constituée d'une quantité de peptide XAP d'environ 5 mg à 3000 mg.

Dans un autre aspect, ladite composition pour son utilisation dans la prévention et/ou le traitement de pathologies liées à l'alpha-glucosidase est sous forme unitaire et comprend ou est constituée d'une quantité de peptide XAP d'environ 5 mg à 2250 mg ou d'environ >2250 mg à 3000 mg.

Dans un autre aspect, ladite composition pour son utilisation dans la prévention et/ou le traitement de pathologies liées à l'alpha-glucosidase comprend ou est constituée d'une quantité de peptide VAP d'environ 5 mg à environ 2250 mg ou d'environ >2250 mg à 3000 mg, et/ou comprend ou est constituée d'une quantité de peptide AP d'environ 5 mg à environ 2250 mg ou d'environ >2250 mg à 3000 mg, et/ou comprend ou est constituée d'une quantité de peptides VAP et AP d'environ 5 mg à environ 2250 mg ou d'environ >2250 mg à 3000 mg.

L'expression « d'environ 5 mg à environ 2250 mg » signifie que les doses peuvent être quelque peu inférieures à 5 mg, par exemple 4,9 mg, et quelque peu supérieures à 2250 mg, par exemple 2250,1 mg. Cette expression signifie aussi que toutes les valeurs de 5 mg à 2250 mg sont comprises, par exemple de 5 mg à 10 mg ; de 10 mg à 15 mg : de 15 mg à 20 mg ; de 20 mg à 25 mg ; de 25 mg à 30 mg ; de 30 mg à 35 mg ; de 35 mg à 40 mg ; de 45 mg à 50 mg ; de 50 mg à 55 mg ; de 55 mg à 60 mg ; de 60 mg à 65 mg ; de 65 mg à 70 mg ; de 70 mg à 75 mg ; de 75 mg à 80 mg ; de 80 mg à 85 mg ; de 85 mg à 90 mg ou de 95 mg à 100 mg, mais aussi de 5 mg à 100 mg ; à 150 mg ; à 200 mg ; à 250 mg ; à 300 mg ; à 350 mg ; à 400 mg ; à 450 mg ; à 500 mg ; à 550 mg ; à 600 mg ; à 650 mg ; à 700 mg ; à 750 mg ; à 800 mg ; à 850 mg ; à 900 mg ; à 950 mg ; à 1000 mg ; à 1050 mg ; à 1100 mg ; à 1150 mg ; à 1200 mg ; à 1250 mg ; à 1300 mg ; à 1350 mg ; à 1400 mg ; à 1450 mg ; à 1500 mg ; à 1550 mg ; à 1600 mg ; à 1650 mg ; à 1700 mg ; à 1750 mg ; à 1800 mg ; à 1850 mg ; à 1900 mg ; à 1950 mg ; à 2000 mg ; à 2050 mg ; à 2100 mg ; à 2150 mg ; à 2200 mg.

Dans un aspect particulier, ladite composition pour son utilisation dans la prévention et/ou le traitement de pathologies liées à l'alpha-glucosidase comprend ou est constituée d'une quantité de 1000 mg de peptide XAP, notamment de peptide AP.

L'expression « d'environ >2250 mg à 3000 mg » signifie que les doses peuvent être quelque peu supérieures à 3000 mg, par exemple 3000,1 mg. Cette expression signifie aussi que toutes les valeurs de >2250 mg à 3000 mg sont comprises, notamment 2260 mg ; 2270 mg ; 2280 mg et 2290 mg.

L'expression « d'environ >2250 mg » signifie des valeurs strictement supérieures à 2250 mg.

Dans un autre aspect, ladite composition pour son utilisation dans la prévention et/ou le traitement de pathologies liées à l'alpha-glucosidase comprend aussi ou est constituée d'une quantité de peptide VAP d'environ 5 mg à environ 7,4 mg, et/ou comprend ou est constituée d'une quantité de peptide AP d'environ 5 mg à environ 7,4 mg, et/ou comprend ou est constituée d'une quantité de peptides VAP et AP d'environ 5 mg à environ 7,4 mg.

L'expression « d'environ 5 mg à environ 7,4 mg » doit être comprise comme pouvant indiquer l'une des valeurs suivantes : 5,0 ; 5,1 ; 5,2 ; 5,3 ; 5,4 ; 5,5 ; 5,6 ; 5,7 ; 5,8 ; 5,9 ; 6,0 ; 6,1 ; 6,2 ; 6,3 ; 6,4 ; 6,5 ; 6,6 ; 6,7 ; 6,8 ; 6,9 ; 7,0 ; 7,1 ; 7,2 ; 7,3 ou 7,4.

Dans encore un autre aspect, ladite composition pour son utilisation dans la prévention et/ou le traitement de pathologies liées à l'alpha-glucosidase comprend ou est constituée d'aussi une quantité de peptide VAP d'environ 7,5 mg à environ 2250 mg ou d'environ >2250 mg à 3000 mg, et/ou comprend ou est constituée d'une quantité de peptide AP d'environ 7,5 mg à environ 2250 mg ou d'environ >2250 mg à 3000 mg, et/ou comprenant ou est constituée d'une quantité de peptides VAP et AP d'environ 7,5 mg à environ 2250 mg ou d'environ >2250 mg à 3000 mg.

Dans un aspect particulier de la description, ladite composition pour son utilisation dans la prévention et/ou le traitement de pathologies liées à l'alpha-glucosidase comprend ainsi ou est constituée d'une quantité de peptide VAP d'environ 5 mg à environ 2250 mg, notamment d'environ 5 mg à environ 7,4 mg ou notamment d'environ 7,5 mg à environ 2250 mg ou une quantité de peptide VAP d'environ >2250 mg à 3000 mg.

Dans un autre aspect particulier de la description, ladite composition pour son utilisation dans la prévention et/ou le traitement de pathologies liées à l'alpha-glucosidase comprend ainsi ou est constituée d'une quantité de peptide AP d'environ 5 mg à environ 2250 mg, notamment environ 5 mg à environ 7,4 mg ou notamment environ 7,5 mg à environ 2250 mg ou une quantité de peptide AP d'environ >2250 mg à 3000 mg.

Dans un autre aspect particulier de l'invention, ladite composition pour son utilisation dans la prévention et/ou le traitement du diabète de type 2 comprend ainsi ou est constituée d'une quantité de peptide AP d'environ 5 mg à environ 2250 mg, notamment environ 5 mg à environ 7,4 mg ou notamment environ 7,5 mg à environ 2250 mg ou une quantité de peptide AP d'environ >2250 mg à 3000 mg.

Dans un autre aspect particulier de la description, ladite composition pour son utilisation dans la prévention et/ou le traitement de pathologies liées à l'alpha-glucosidase comprend ainsi ou est constituée d'une quantité de peptides VAP et AP d'environ 5 mg à environ 2250 mg, notamment environ 5 mg à environ 7,4 mg ou notamment environ 7,5 mg à environ 2250 mg.

Dans un autre aspect particulier de l'invention, ladite composition pour son utilisation dans la prévention et/ou le traitement du diabète de type 2 comprend ainsi ou est constituée d'une quantité de peptides VAP et AP d'environ 5 mg à environ 2250 mg, notamment environ 5 mg à environ 7,4 mg ou notamment environ 7,5 mg à environ 2250 mg.

Dans un autre aspect particulier de la description, ladite composition pour son utilisation dans la prévention et/ou le traitement de pathologies liées à l'alpha-glucosidase comprend ainsi ou est constituée d'une quantité de peptides VAP et AP d'environ >2250 mg à 3000 mg.

Dans un autre aspect particulier de l'invention, ladite composition pour son utilisation dans la prévention et/ou le traitement du diabète de type 2 comprend ainsi ou est constituée d'une quantité de peptides VAP et AP d'environ >2250 mg à 3000 mg.

Dans un autre aspect particulier de la description, ladite composition pour son utilisation dans la prévention et/ou le traitement de pathologies liées à l'alpha-glucosidase comprend ainsi ou est constituée d'une quantité de peptide VAP d'environ 5 mg à environ 2250 mg, notamment environ 5 mg à environ 7,4 mg ou notamment environ 7,5 mg à environ 2250 mg, et comprend ou est constituée d'une quantité de peptide AP d'environ 5 mg à environ 2250 mg, notamment environ 5 mg à environ 7,4 mg ou notamment environ 7,5 mg à environ 2250 mg.

Dans un autre aspect particulier de l'invention, ladite composition pour son utilisation dans la prévention et/ou le traitement du diabète de type 2 comprend ainsi ou est constituée d'une quantité de peptide VAP d'environ 5 mg à environ 2250 mg, notamment environ 5 mg à environ 7,4 mg ou notamment environ 7,5 mg à environ 2250 mg, et comprend ou est constituée d'une quantité de peptide AP d'environ 5 mg à environ 2250 mg, notamment environ 5 mg à environ 7,4 mg ou notamment environ 7,5 mg à environ 2250 mg.

Dans un autre aspect particulier de la description, ladite composition pour son utilisation dans la prévention et/ou le traitement de pathologies liées à l'alpha-glucosidase comprend ainsi ou est constituée d'une quantité de peptide VAP d'environ >2250 mg à 3000 mg, et comprend ou est constituée d'une quantité de peptide AP d'environ >2250 mg à 3000 mg.

Dans un autre aspect particulier de l'invention, ladite composition pour son utilisation dans la prévention et/ou le traitement du diabète de type 2 comprend ainsi ou est constituée d'une quantité de peptide VAP d'environ >2250 mg à 3000 mg, et comprend ou est constituée d'une quantité de peptide AP d'environ >2250 mg à 3000 mg.

Dans encore un autre aspect, la description concerne la composition pour son utilisation dans la prévention et/ou le traitement de pathologies liées à l'alpha-glucosidase telle que précédemment mentionnée, pour son utilisation par administration orale d'une quantité de peptide XAP d'environ 0,06 mg/kg à 40,0 mg/kg, trois fois par jour au début du repas.

Dans encore un autre aspect, l'invention concerne la composition pour son utilisation dans la prévention et/ou le traitement du diabète de type 2 telle que précédemment mentionnée, pour son utilisation par administration orale d'une quantité de peptide AP d'environ 0,06 mg/kg à 40,0 mg/kg, trois fois par jour au début du repas.

Dans encore un autre aspect, la description concerne la composition pour son utilisation dans la prévention et/ou le traitement de pathologies liées à l'alpha-glucosidase telle que précédemment mentionnée, pour son utilisation par administration orale d'une quantité de peptide XAP d'environ 0,06 mg/kg à environ 30,0mg/kg ou d'environ >30,0 mg/kg à 40 mg/kg, trois fois par jour au début du repas.

Dans encore un autre aspect, l'invention concerne la composition pour son utilisation dans la prévention et/ou le traitement du diabète de type 2 telle que précédemment mentionnée, pour son utilisation par administration orale d'une quantité de peptide AP d'environ 0,06 mg/kg à environ 30,0mg/kg ou d'environ >30,0 mg/kg à 40 mg/kg, trois fois par jour au début du repas.

L'expression « environ 0,06 mg/kg à environ 30,0 mg/kg de peptide XAP » signifie aussi que la composition selon la présente invention peut comprendre toutes les valeurs de 0,06 mg/kg à 30,0 mg/kg, notamment de 0,06 mg/kg à 0,08 mg/kg ; de 0,06 mg/kg à 0,09 mg/kg ; de 0,06 mg/kg à 0,1 mg/kg ; de 0,06 mg/kg à 0,5 mg/kg ; de 0,06 mg/kg à 1,0 mg/kg de 0,06 mg/kg à 2,0 mg/kg ; de 0,06 mg/kg à 5,0 mg/kg ; de 0,06 mg/kg à 10,0 mg/kg ; de 0,06 mg/kg à 15,0 mg/kg ; de 0,06 mg/kg à 20,0 mg/kg ; de 0,06 mg/kg à 25,0 mg/kg ; de 0,06 mg/kg à 30,0 mg/kg.

L'expression « d'environ >30,0 mg/kg à 40 mg/kg » signifie aussi que la composition selon la présente invention peut comprendre toutes les valeurs de >30,0 mg/kg à 40 mg/kg, notamment de 31 mg/kg à 40 mg/kg ; de 32 mg/kg à 40 mg/kg ; de 33 mg/kg à 40 mg/kg ; de 33 mg/kg à 40 mg/kg ; de 34 mg/kg à 40 mg/kg ; de 35 mg/kg à 40 mg/kg ; de 36 mg/kg à 40 mg/kg ; de 37 mg/kg à 40 mg/kg ; de 38 mg/kg à 40 mg/kg ; de 39 mg/kg à 40 mg/kg.

Dans un aspect, ladite composition pour son utilisation dans la prévention et/ou le traitement de pathologies liées à l'alpha-glucosidase comprend ainsi ou est constituée d'une quantité de peptide VAP d'environ 0,06 mg/kg à environ 30,0 mg/kg ou d'environ >30,0 mg/kg à 40 mg/kg, et/ou une quantité de peptide AP d'environ 0,06 mg/kg à environ 30,0 mg/kg ou d'environ >30,0 mg/kg à 40 mg/kg, et/ou une quantité de peptides VAP et AP d'environ 0,06 mg/kg à environ 30,0 mg/kg ou d'environ >30,0 mg/kg à 40 mg/kg, pour une administration orale trois fois par jour au début du repas.

Dans un aspect particulier, ladite composition pour son utilisation dans la prévention et/ou le traitement de pathologies liées à l'alpha-glucosidase comprend ainsi ou est constituée d'une quantité de peptide VAP d'environ 0,06 mg/kg à environ 0,099 mg/kg, et/ou une quantité de peptide AP d'environ 0,06 mg/kg à environ 0,099 mg/kg, et/ou une quantité de peptides VAP et AP d'environ 0,06 mg/kg à environ 0,099 mg/kg, pour une administration orale trois fois par jour au début du repas.

Dans encore un aspect particulier, ladite composition pour son utilisation dans la prévention et/ou le traitement de pathologies liées à l'alpha-glucosidase comprend ainsi ou est constituée d'une quantité de peptide VAP d'environ 0,1 mg/kg à environ 30,0 mg/kg ou d'environ >30,0 mg/kg à 40 mg/kg, et/ou une quantité de peptide AP d'environ 0,1 mg/kg à environ 30,0 mg/kg ou d'environ >30,0 mg/kg à 40 mg/kg, et/ou une quantité de peptides VAP et AP d'environ 0,1 mg/kg à environ 30,0 mg/kg ou d'environ >30,0 mg/kg à 40 mg/kg, pour une administration orale trois fois par jour au début du repas.

Dans un aspect particulier de la description, ladite composition pour son utilisation dans la prévention et/ou le traitement de pathologies comprend ou est constituée d'une quantité de peptide VAP d'environ 0,06 mg/kg à environ 30,0 mg/kg, notamment environ 0,06 mg/kg à environ 0,099 mg/kg ou notamment environ 0,1 mg/kg à environ 30,0 mg/kg ou d'environ >30,0 mg/kg à 40 mg/kg, pour une administration orale trois fois par jour au début du repas.

Dans un autre aspect particulier, ladite composition pour son utilisation dans la prévention et/ou le traitement de pathologies comprend ou est constituée d'une quantité de peptide AP d'environ 0,06 mg/kg à environ 30,0 mg/kg, notamment environ 0,06 mg/kg à environ 0,099 mg/kg ou notamment environ 0,1 mg/kg à environ 30,0 mg/kg ou d'environ >30,0 mg/kg à 40 mg/kg, pour une administration orale trois fois par jour au début du repas.

Dans un autre aspect particulier, ladite composition pour son utilisation dans la prévention et/ou le traitement de pathologies comprend ou est constituée d'une quantité de peptides VAP et AP d'environ 0,06 mg/kg à environ 30,0 mg/kg, notamment environ 0,06 mg/kg à environ 0,099 mg/kg ou notamment environ 0,1 mg/kg à environ 30,0 mg/kg ou d'environ >30,0 mg/kg à 40 mg/kg, pour une administration orale trois fois par jour au début du repas.

Dans encore un autre aspect particulier, ladite composition pour son utilisation dans la prévention et/ou le traitement de pathologies comprend ou est constituée d'une quantité de peptide VAP d'environ 0,06 mg/kg à environ 30,0 mg/kg, notamment environ 0,06 mg/kg à environ 0,099 mg/kg ou notamment environ 0,1 mg/kg à environ 30,0 mg/kg et une quantité de peptide AP d'environ 0,06 mg/kg à environ 30,0 mg/kg, notamment environ 0,06 mg/kg à environ 0,099 mg/kg ou notamment environ 0,1 mg/kg à environ 30,0 mg/kg, pour une administration orale trois fois par jour au début du repas.

Dans encore un autre aspect particulier, ladite composition pour son utilisation dans la prévention et/ou le traitement de pathologies comprend ou est constituée d'une quantité d'environ >30,0 mg/kg à 40 mg/kg de peptide VAP et une quantité de peptide AP d'environ >30,0 mg/kg à 40 mg/kg, pour une administration orale trois fois par jour au début du repas.

La présente description concerne également une composition pharmaceutique comprenant un peptide XAP, dans lequel X représente l'ensemble vide ou une valine, ladite composition comprenant ou étant constituée d'une quantité de peptide XAP d'environ 0,06 mg/kg à environ 0,099 mg/kg, notamment environ 0,066 mg/kg, en association avec un véhicule pharmaceutiquement acceptable.

La présente invention concerne également une composition pharmaceutique comprenant un peptide AP, ladite composition comprenant ou étant constituée d'une quantité de peptide AP d'environ 0,06 mg/kg à environ 0,099 mg/kg, notamment environ 0,066 mg/kg, en association avec un véhicule pharmaceutiquement acceptable.

Les doses ont été établies en considérant un patient adulte de 75 kg.

L'expression « d'environ 0,06 mg/kg à environ 0,099 mg/kg » signifie que les doses peuvent être quelque peu inférieures à 0,06 mg/kg ou quelque peu supérieures à 0,099 mg/kg par exemple 0,059 mg/kg ou bien 0,0999 mg/kg.

L'expression « d'environ 0,06 mg/kg à environ 0,099 mg/kg » peut aussi indiquer les valeurs suivantes : de 0,06 mg/kg à 0,07 mg/kg ; de 0,06 mg/kg à 0,08 mg/kg ; de 0,06 mg/kg à 0,09 mg/kg ; de 0,07 mg/kg à 0,08 mg/kg ; de 0,08 mg/kg à 0,09 mg/kg ou de 0,07 mg/kg à 0,09 mg/kg.

La présente description concerne également une composition pharmaceutique telle que mentionnée ci-dessus, ladite composition étant dépourvue de vitamine B dans un aspect particulier, ladite composition apparaissant avantageusement meilleure sans vitamine B.

Dans un autre aspect particulier de la description, ladite composition pharmaceutique contient le peptide VAP et est dépourvue de vitamine B.

Dans un autre aspect particulier de l'invention, ladite composition pharmaceutique contient le peptide AP et de la vitamine B.

La présente description concerne également une composition pharmaceutique telle que mentionnée ci-dessus, ladite composition comprenant ou étant constituée d'une quantité de peptide VAP d'environ 0,06 mg/kg à environ 0,099 mg/kg, et/ou une quantité de peptide AP d' environ 0,06 mg/kg à environ 0,099 mg/kg, et/ou une quantité de peptides VAP et AP d' environ 0,06 mg/kg à environ 0,099 mg/kg.

La présente invention concerne également une composition pharmaceutique telle que mentionnée ci-dessus, ladite composition comprenant ou étant constituée d'une quantité de peptide AP d' environ 0,06 mg/kg à environ 0,099 mg/kg, et/ou une quantité de peptides VAP et AP d'environ 0,06 mg/kg à environ 0,099 mg/kg.

Dans un aspect particulier de la description, ladite composition pharmaceutique comprend ou est constituée d'une quantité de peptide VAP d'environ 0,06 mg/kg à environ 0,099 mg/kg.

Dans un autre aspect particulier de l'invention, ladite composition pharmaceutique comprend ou est constituée d'une quantité de peptide AP d'environ 0,06 mg/kg à environ 0,099 mg/kg.

Dans un autre aspect particulier de l'invention, ladite composition pharmaceutique comprend ou est constituée d'une quantité de peptides VAP et AP d'environ 0,06 mg/kg à environ 0,099 mg/kg.

Dans encore un autre aspect particulier, ladite composition pharmaceutique comprend ou est constituée d'une quantité de peptide VAP d'environ 0,06 mg/kg à environ 0,099 mg/kg, et une quantité de peptide AP d'environ 0,06 mg/kg à environ 0,099 mg/kg.

Dans encore un autre aspect de la description, ladite composition pharmaceutique comprend un peptide XAP, dans lequel X représente l'ensemble vide ou une valine, et est sous forme unitaire et comprend ou est constituée d'une quantité de peptide XAP d'environ 5 mg à environ 7,4 mg, en association avec un véhicule pharmaceutiquement acceptable.

Dans encore un autre aspect de l'invention, ladite composition pharmaceutique comprend un peptide AP, et est sous forme unitaire et comprend ou est constituée d'une quantité de peptide AP d'environ 5 mg à environ 7,4 mg, en association avec un véhicule pharmaceutiquement acceptable.

Dans un aspect particulier de la description, ladite composition pharmaceutique est dépourvue de vitamine B, ladite composition apparaissant avantageusement meilleure sans vitamine B.

Dans un autre aspect particulier de la description, ladite composition pharmaceutique contient le peptide VAP et est dépourvue de vitamine B.

L'expression « d'environ 5 mg à environ 7,4 mg » doit être comprise comme pouvant indiquer l'une des valeurs suivantes : 5,0 ; 5,1 ; 5,2 ; 5,3 ; 5,4 ; 5,5 ; 5,6 ; 5,7 ; 5,8 ; 5,9 ; 6,0 ; 6,1 ; 6,2 ; 6,3 ; 6,4 ; 6,5 ; 6,6 ; 6,7 ; 6,8 ; 6,9 ; 7,0 ; 7,1 ; 7,2 ; 7,3 ou 7,4.

Dans un aspect particulier, ladite composition pharmaceutique comprend ou est constituée d'une quantité de peptide VAP d'environ 5 mg à environ 7,4 mg, et/ou ladite composition pharmaceutique comprend ou est constituée d'une quantité de peptide AP d'environ 5 mg à environ 7,4 mg, et/ou ladite composition pharmaceutique comprend ou est constituée d'une quantité de peptides VAP et AP d'environ 5 mg à environ 7,4 mg. Cela signifie que dans un aspect particulier de la description ladite composition pharmaceutique comprend ou est constituée d'une quantité de peptide VAP d'environ 5 mg à environ 7,4 mg ou une quantité de peptide AP d'environ 5 mg à environ 7,4 mg ou une quantité de peptides VAP et AP d'environ 5 mg à environ 7,4 mg, ou bien une quantité de peptide VAP d'environ 5 mg à environ 7,4 mg et une quantité de peptide AP d'environ 5 mg à environ 7,4 mg.

Cela signifie que dans un aspect particulier de l'invention ladite composition pharmaceutique comprend ou est constituée d'une quantité de peptide AP d'environ 5 mg à environ 7,4 mg ou une quantité de peptides VAP et AP d'environ 5 mg à environ 7,4 mg, ou bien une quantité de peptide VAP d'environ 5 mg à environ 7,4 mg et une quantité de peptide AP d'environ 5 mg à environ 7,4 mg.

Dans un aspect particulier, ladite composition pharmaceutique peut aussi être en association avec au moins un peptide de type APX'.

Ladite composition pharmaceutique peut être administrée sous forme de comprimés, de gélules, de poudres, de pastilles, de pilules, de granulés ou toute autre forme administrable par voie orale, et être sous forme de sachets de poudre, d'ampoules de liquide, de flacons munis de compte-gouttes et les autres formes analogues de préparations liquides ou de poudre.

De préférence, la composition pharmaceutique est sous forme de comprimés et pourra être avalée avec un peu d'eau au début des repas ou bien croquée lors des premières bouchées de nourriture.

Dans un autre aspect de l'invention, ladite composition pharmaceutique peut comprendre un ou plusieurs inhibiteurs de l'amylase et/ou un ou plusieurs inhibiteurs de lipase.

En plus de l'aspect thérapeutique mentionné ci-dessus, les peptides XAP selon la présente invention, peuvent également être utilisés dans le domaine nutraceutique (en tant que complément alimentaire par exemple) ou dans le domaine alimentaire (aliments fonctionnels).

Le terme nutraceutique fait référence à l'ingrédient actif présent à l'état naturel ou sous forme synthétique dans un aliment qui procure un effet bénéfique pour la santé. Par exemple le yoghourt Danacol® de Danone contient des stérols végétaux non présents naturellement dans le yoghourt. Ce sont des actifs qui ont été ajoutés. Un aliment de consommation courante (par exemple une boisson, un produit laitier, des céréales, un biscuit) peut contenir un ingrédient actif nutraceutique et peut être considéré comme un aliment fonctionnel s'il a été démontré qu'il exerce un effet bénéfique sur une ou plusieurs fonctions cibles de l'organisme, au-delà des effets nutritionnels de base.

Un aliment peut être rendu fonctionnel selon 5 approches : en éliminant un composant connu ou identifié pour ses effets nocifs, en augmentant la concentration d'un composant naturel dans un aliment, en ajoutant un composant normalement absent de la majorité des aliments (mais dont les effets bénéfiques sont prouvés), en remplaçant un composant ou en améliorant la biodisponibilité des composants alimentaires.

Les compléments alimentaires sont des denrées alimentaires dont le but est de compléter le régime alimentaire normal et qui constituent une source concentrée de nutriments ou d'autres substances ayant un effet nutritionnel ou physiologique seuls ou combinés. De telles denrées sont destinées à être prises en unités mesurées en faible quantités.

Dans un autre aspect, la description concerne aussi l'utilisation d'au moins un peptide XAP, dans lequel X représente l'ensemble vide ou une valine, pour la préparation d'une composition nutraceutique ou d'un complément alimentaire.

Dans un autre aspect, l'invention concerne aussi l'utilisation d'au moins un peptide AP, pour la préparation d'une composition nutraceutique ou d'un complément alimentaire.

Dans un aspect particulier, la description concerne l'utilisation d'au moins un peptide VAP.

Dans un aspect particulier, l'invention concerne l'utilisation d'au moins un peptide AP.

Dans un aspect particulier, l'invention concerne l'utilisation d'au moins un peptide VAP et l'utilisation d'au moins un peptide AP.

Dans encore un autre aspect, la description concerne ainsi une composition alimentaire ou nutraceutique pour inhiber l'alpha-glucosidase, et notamment la maltase, ladite composition comprenant au moins un peptide XAP, dans lequel X représente l'ensemble vide ou une valine, ladite composition comprenant une quantité de peptide XAP d'environ 0,06 mg/kg à environ 14 mg/kg.

Dans encore un autre aspect, l'invention concerne ainsi une composition alimentaire ou nutraceutique pour inhiber l'alpha-glucosidase, ladite composition comprenant au moins un peptide AP, ladite composition comprenant une quantité de peptide AP d'environ 0,06 mg/kg à environ 14 mg/kg.

Dans encore un autre aspect, la description concerne ainsi une composition alimentaire ou nutraceutique pour inhiber l'alpha-glucosidase, et notamment la maltase, ladite composition comprenant au moins un peptide XAP, dans lequel X représente l'ensemble vide ou une valine, ladite composition comprenant une quantité de peptide XAP d'environ 0,06 mg/kg à <0,6 mg/kg ou d'environ 0,6 mg/kg à environ 14 mg/kg.

Dans encore un autre aspect, l'invention concerne ainsi une composition alimentaire ou nutraceutique pour inhiber l'alpha-glucosidase, ladite composition comprenant au moins un peptide AP, ladite composition comprenant une quantité de peptide AP d'environ 0,06 mg/kg à <0,6 mg/kg ou d'environ 0,6 mg/kg à environ 14 mg/kg.

Dans un aspect particulier, ladite composition alimentaire ou nutraceutique est dépourvue de vitamine B dans un aspect particulier, ladite composition apparaissant avantageusement meilleure sans vitamine B.

Dans un autre aspect particulier de la description, ladite composition alimentaire ou nutraceutique contient le peptide VAP et est dépourvue de vitamine B.

Dans un autre aspect particulier de l'invention, ladite composition alimentaire ou nutraceutique contient le peptide AP et de la vitamine B.

Dans un autre aspect particulier de la description, la composition alimentaire ou nutraceutique comprend une quantité de peptide VAP d'environ 0,06 mg/kg à <0,6 mg/kg ou d'environ 0,6 mg/kg à environ 14 mg/kg, et/ou une quantité de peptide AP d'environ 0,06 mg/kg à <0,6 mg/kg ou d'environ 0,6 mg/kg à environ 14 mg/kg, et/ou une quantité de peptides VAP et AP d'environ 0,06 mg/kg à <0,6 mg/kg ou d'environ 0,6 mg/kg à environ 14 mg/kg.

Dans un autre aspect particulier de l'invention, la composition alimentaire ou nutraceutique comprend une quantité de peptide AP d'environ 0,06 mg/kg à <0,6 mg/kg ou d'environ 0,6 mg/kg à environ 14 mg/kg, et/ou une quantité de peptides VAP et AP d'environ 0,06 mg/kg à <0,6 mg/kg ou d'environ 0,6 mg/kg à environ 14 mg/kg.

L'expression « d'environ 0,06 mg/kg à <0,6 mg/kg » doit s'entendre comme pouvant couvrir des valeurs quelques peu inférieures à 0,06 mg/kg, par exemple 0,059 mg/kg.

L'expression « <0,6 mg/kg » signifie des valeurs strictement inférieures à 0,6 mg/kg.

L'expression « d'environ 0,06 mg/kg à <0,6 mg/kg » peut aussi indiquer les valeurs suivantes : de 0,06 mg/kg à 0,58 mg/kg ; de 0,08 mg/kg à 0,58 mg/kg ; de 0,1 mg/kg à 0,58 mg/kg ; de 0,12 mg/kg à 0,58 mg/kg ; de 0,13 mg/kg à 0,58 mg/kg ; de 0,15 à 0,58 mg/kg ; de 0,20 mg/kg à 0,58 mg/kg ; de 0,25 mg/kg à 0,58 mg/kg ; de 0,30 mg/kg à 0,58 mg/kg ; de 0,35 mg/kg à 0,58 mg/kg ; de 0,40 mg/kg à 0,58 mg/kg ; de 0,45 mg/kg à 0,58 mg/kg ; de 0,50 mg/kg à 0,58 mg/kg ; de 0,55 mg/kg à 0,58 mg/kg.

L'expression « d'environ 0,6 mg/kg à environ 14 mg/kg » doit s'entendre comme pouvant couvrir des valeurs quelques peu inférieures à 0,6 mg/kg, par exemple 0,59 mg/kg, ou bien des valeurs quelques peu supérieures à 14 mg/kg, par exemple 14,1 mg/kg

L'expression « d'environ 0,6 mg/kg à environ 14 mg/kg » peut aussi indiquer les valeurs suivantes : 0,6 mg/kg ; 0,8 mg/kg ; 1,0 mg/kg ; 1,2 mg/kg ; 1,4 mg/kg ; 1,6 mg/kg ; 1,8 mg/kg ; 2,0 mg/kg ; 2,2 mg/kg ; 2,4 mg/kg ; 2,6 mg/kg ; 2,8 mg/kg ; 3,0 mg/kg ; 3,2 mg/kg ; 3,4 mg/kg ; 3,6 mg/kg ; 3,8 mg/kg ; 3,0 mg/kg ; 3,2 mg/kg ; 3,4 mg/kg ; 3,6 mg/kg ; 3,8 mg/kg ; 4,0 mg/kg ; 4,2 mg/kg ; 4,4 mg/kg ; 4,6 mg/kg ; 4,8 mg/kg ; 5,0 mg/kg ; 5,2 mg/kg ; 5,4 mg/kg ; 5,6 mg/kg ; 5,8 mg/kg ; 6,0 mg/kg ; 6,2 mg/kg ; 6,4 mg/kg ; 6,6 mg/kg ; 6,8 mg/kg ; 7,0 mg/kg ; 7,2 mg/kg ; 7,4 mg/kg; 7,6 mg/kg ; 7,8 mg/kg ; 8,0 mg/kg ; 8,2 mg/kg ; 8,4 mg/kg ; 8,6 mg/kg ; 8,8 mg/kg ; 9,0 mg/kg ; 9,2 mg/kg ; 9,4 mg/kg ; 9,6 mg/kg ; 9,8 mg/kg ; 10,0 mg/kg ; 10,2 mg/kg ; 10,4 mg/kg ; 10,6 mg/kg ; 10,8 mg/kg ; 11,2 mg/kg ; 11,4 mg/kg ; 11,6 mg/kg ; 11,8 mg/kg ; 12,2 mg/kg ; 12,4 mg/kg ; 12,6 mg/kg ; 12,8 mg/kg ; 13,0 mg/kg ; 13,2 mg/kg ; 13,4 mg/kg ; 13,6 mg/kg ; 13,8 mg/kg et 14,0 mg/kg, ou encore de 0,6 mg/kg à 0,9 mg/kg ; de 2 mg/kg à 3 mg/kg ou 4 mg/kg ou 5 mg/kg ou 6 mg/kg ou 7 mg/kg ou 8 mg/kg ou 9 mg/kg ou 10 mg/kg ou 11 mg/kg ou 12 mg/kg ou 13 mg/kg ou 14 mg/kg.

Dans encore un autre aspect particulier, ladite composition alimentaire ou nutraceutique comprend une quantité de peptide VAP d'environ 0,06 mg/kg à <0,6 mg/kg ou d'environ 0,6 mg/kg à environ 14 mg/kg.

Dans encore un autre aspect particulier, ladite composition alimentaire ou nutraceutique comprend une quantité de peptide AP d'environ 0,06 mg/kg à <0,6 mg/kg ou d'environ 0,6 mg/kg à environ 14 mg/kg.

Dans encore un autre aspect particulier, ladite composition alimentaire ou nutraceutique comprend une quantité de peptides VAP et AP d'environ 0,06 mg/kg à <0,6 mg/kg ou d'environ 0,6 mg/kg à environ 14 mg/kg.

Dans encore un autre aspect, ladite composition alimentaire ou nutraceutique comprend une quantité de peptide VAP d'environ 0,06 mg/kg à <0,6 mg/kg ou d'environ 0,6 mg/kg à environ 14 mg/kg, et une quantité de peptide AP d'environ 0,06 mg/kg à <0,6 mg/kg ou d'environ 0,6 mg/kg à environ 14 mg/kg.

Dans un aspect particulier, ladite composition alimentaire ou nutraceutique peut aussi être en association avec au moins un peptide de type APX'.

Dans un aspect particulier, ladite composition alimentaire ou nutraceutique peut être administrée sous forme de comprimés, de gélules, de poudres, de pastilles, de pilules, de granulés ou toute autre forme administrable par voie orale, et être sous forme de sachets de poudre, d'ampoules de liquide, de flacons munis de compte-gouttes et les autres formes analogues de préparations liquides ou de poudre

De préférence, ladite composition alimentaire ou nutraceutique est sous forme de comprimés et pourra être avalée avec un peu d'eau au début des repas ou bien croquée lors des premières bouchées de nourriture.

Dans encore un autre aspect, la description concerne aussi une composition alimentaire ou nutraceutique pour inhiber l'alpha-glucosidase, et notamment la maltase, ladite composition comprenant au moins un peptide XAP, dans lequel X représente l'ensemble vide ou une valine, ladite composition étant sous forme unitaire et comprenant une quantité de peptide XAP d'environ 5 mg à environ 1000 mg.

Dans encore un autre aspect, l'invention concerne aussi une composition alimentaire ou nutraceutique pour inhiber l'alpha-glucosidase, ladite composition comprenant au moins un peptide AP, ladite composition étant sous forme unitaire et comprenant une quantité de peptide AP d'environ 5 mg à environ 1000 mg.

Dans encore un autre aspect, la description concerne aussi une composition alimentaire ou nutraceutique pour inhiber l'alpha-glucosidase, et notamment la maltase, ladite composition comprenant au moins un peptide XAP, dans lequel X représente l'ensemble vide ou une valine, ladite composition étant sous forme unitaire et comprenant une quantité de peptide XAP d'environ 5 mg à <50 mg ou d'environ 50 mg à environ 1000 mg.

Dans encore un autre aspect, l'invention concerne aussi une composition alimentaire ou nutraceutique pour inhiber l'alpha-glucosidase, ladite composition comprenant au moins un peptide AP, ladite composition étant sous forme unitaire et comprenant une quantité de peptide AP d'environ 5 mg à <50 mg ou d'environ 50 mg à environ 1000 mg.

Dans un aspect particulier, ladite composition alimentaire ou nutraceutique est dépourvue de vitamine B dans un aspect particulier, ladite composition apparaissant avantageusement meilleure sans vitamine B.

Dans un autre aspect particulier de la description, ladite composition alimentaire ou nutraceutique contient le peptide VAP et est dépourvue de vitamine B.

Dans un autre aspect particulier de l'invention, ladite composition alimentaire ou nutraceutique contient le peptide AP et de la vitamine B.

L'expression « d'environ 5 mg à <50 mg » doit s'entendre comme pouvant couvrir des valeurs quelque peu inférieures à 5 mg, par exemple 4,9 mg.

L'expression « <50 mg » signifie des valeurs strictement inférieures à 50 mg.

L'expression « d'environ 5 mg à <50 mg » peut indiquer toutes les valeurs de 10 mg à <50 mg, par exemple de de 5 mg à 49 mg ; de 10 mg à 49 mg ; de 15 mg à 49 mg ; de 20 mg à 49 mg ; de 25 mg à 49 mg ; de 30 mg à 49 mg ; de 35 mg à 49 mg ; de 40 à 49 mg ; de 45 mg à 49 mg.

L'expression « d'environ 50 mg à environ 1000 mg » doit s'entendre comme pouvant couvrir des valeurs quelque peu inférieures à 50 mg, par exemple 49,9 mg, ou bien des valeurs quelques peu supérieures à 1000 mg, par exemple 1000,01 mg.

L'expression « d'environ 50 mg à environ 1000 mg » peut indiquer toutes les valeurs de 50 mg à 1000 mg, par exemple de 50 mg à 100 mg ; de 100 mg à 200 mg ; de 200 mg à 300 mg ; de 300 mg à 400 mg ; de 400 mg à 500 mg ; de 500 mg à 600 mg ; de 600 mg à 700 mg ; de 800 mg à 900 mg ou de 900 mg à 1000 mg.

Dans un autre aspect, la description concerne aussi une composition alimentaire ou nutraceutique pour inhiber l'alpha-glucosidase, et notamment la maltase, comprenant une quantité de peptide VAP d'environ 5 mg à <50 mg ou d'environ 50 mg à environ 1000 mg, et/ou une quantité de peptide AP d'environ 5 mg à <50 mg ou d'environ 50 mg à environ 1000 mg, et/ou une quantité de peptides VAP et AP d'environ 5 mg à <50 mg ou d'environ 50 mg à environ 1000 mg. Cela signifie ainsi que ladite composition alimentaire ou nutraceutique pour inhiber l'alpha-glucosidase comprend une quantité de peptide VAP d'environ 5 mg à <50 mg ou d'environ 50 mg à environ 1000 mg ou une quantité de peptide AP d'environ 5 mg à <50 mg ou d'environ 50 mg à environ 1000 mg ou une quantité de peptides VAP et AP d'environ 5 mg à <50 mg ou d'environ 50 mg à environ 1000 mg, ou bien une quantité de peptide VAP d'environ 5 mg à <50 mg ou d'environ 50 mg à environ 1000 mg et une quantité de peptide AP d'environ 5 mg à <50 mg ou d'environ 50 mg à environ 1000 mg.

Dans un autre aspect, l'invention concerne aussi une composition alimentaire ou nutraceutique pour inhiber l'alpha-glucosidase, comprenant une quantité de peptide AP d'environ 5 mg à <50 mg ou d'environ 50 mg à environ 1000 mg, et/ou une quantité de peptides VAP et AP d'environ 5 mg à <50 mg ou d'environ 50 mg à environ 1000 mg. Cela signifie ainsi que ladite composition alimentaire ou nutraceutique pour inhiber l'alpha-glucosidase comprend une quantité de peptide AP d'environ 5 mg à <50 mg ou d'environ 50 mg à environ 1000 mg ou une quantité de peptides VAP et AP d'environ 5 mg à <50 mg ou d'environ 50 mg à environ 1000 mg, ou bien une quantité de peptide VAP d'environ 5 mg à <50 mg ou d'environ 50 mg à environ 1000 mg et une quantité de peptide AP d'environ 5 mg à <50 mg ou d'environ 50 mg à environ 1000 mg.

Dans un autre mode de réalisation, la description concerne l'utilisation d'une composition contenant tout peptide inhibiteur de l'alpha-glucosidase, et notamment tout peptide inhibiteur de la maltase, présentant un $IC_{50}$ maximale vis de l'alpha-glucosidase, et notamment la maltase, de 12,50 mM pour la prévention et/ou le traitement de pathologies liées à l'alpha-glucosidase. L'$IC_{50}$ représente la concentration nécessaire de peptide pour inhiber 50 % de l'activité de l'enzyme.

Dans encore un autre aspect, la description concerne aussi l'utilisation d'un hydrolysat d'au moins une protéine comportant ou étant constituée d'au moins 0,05% de motifs XAP, à la place du peptide XAP tel que précédemment mentionné.

Dans un aspect particulier, ledit hydrolysat n'est pas utilisé en combinaison avec un sensibilisateur d'insuline. Un « sensibilisateur d'insuline » signifie toute molécule qui permet de diminuer le taux sanguins de glucose, à l'exception des peptides XAP et/ou APX'. Un sensibilisateur d'insuline peut par exemple être le chromium, le vanadium, la vitamine B (notamment la niacine), ou encore des herbes ou des extraits de plantes, de préférence des feuilles de *Banaba*, des baies de ginseng, la cannelle et certains composés dans les raisins. Les composés suivants sont également considérés comme des « sensibilisateur d'insuline » : l'acide corosolique, le ptérostilbène, le polymère méthylhydroxy chalcone (MHCP) et les ginsensosides. Des exemples préférés de "sensibilisateurs d'insuline" sont le biguanides (tels que la metformine (Glucophage®), les thiazolidinediones (tels que le Pioglitazone (Actos ®) et Rosiglitazone (Avandia®).

Dans encore un autre aspect, la description concerne aussi l'utilisation d'un hydrolysat d'au moins une protéine comportant ou étant constituée d'au moins 5% de motifs XAP, à la place du peptide XAP tel que précédemment mentionné.

La description concerne ainsi l'utilisation d'un hydrolysat d'au moins une protéine comportant ou étant constituée d'au moins 0,05% à <5% de motifs XAP ou l'utilisation d'un hydrolysat d'au moins une protéine comportant ou étant constituée d'au moins 5% de motifs XAP, à la place du peptide XAP tel que précédemment mentionné.

Dans un autre aspect, la description concerne aussi l'utilisation d'un hydrolysat d'au moins une protéine comportant ou étant constituée d'au moins 0,05% à <5% de motifs XAP ou l'utilisation d'un hydrolysat d'au moins une protéine comportant ou étant constituée d'au moins 5% de motifs XAP, en tant qu'inhibiteur de l'alpha-glucosidase.

Dans un autre aspect particulier, la description concerne ainsi une composition comprenant ou consistant en au moins un hydrolysat d'au moins une protéine, ladite protéine comportant ou étant constituée d'au moins 0,05% à <5% ou d'au moins 5% de motifs XAP, dans lequel X représente l'ensemble vide ou une valine, pour son utilisation dans la prévention et/ou le traitement de pathologies liées à l'alpha-glucosidase.

L'expression « au moins un hydrolysat d'au moins une protéine » signifie que l'hydrolysat peut être issu d'une protéine unique ou d'un mélange de protéines.

Dans ledit « mélange de protéines », les protéines peuvent être de sources différentes ou bien de la même source. Un mélange de protéines de sources différentes peut par exemple être un mélange de protéines de saumon et de protéines de carpe. Un mélange de protéines de même source peut par exemple être un mélange de protéines de saumon.

L'expression « <5% de motifs XAP » signifie des valeurs strictement inférieures à 5% de motifs XAP, notamment 4,9%.

L'expression « ladite protéine comportant ou étant constituée d'au moins 0,05% à <5% de motifs XAP » signifie que la séquence totale en acides aminés de ladite protéine comprend ou est constituée au minimum de 0,05% de motifs XAP, jusqu'à une valeur inférieure à 5%, de motifs XAP, par rapport à la totalité des acides aminés constituant la séquence.

L'expression « au minimum de 0,05% de motifs XAP » signifie que ladite protéine peut comprendre ou être constituée de 0,05% à 100% de motifs XAP, et notamment 0,05 % ; 0,5 % ; 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% et 100%.

L'expression « ladite protéine comportant ou étant constituée d'au moins 5% de motifs XAP » signifie que la séquence totale en acides aminés de ladite protéine comprend ou est constituée au minimum de 5% de motifs XAP par rapport à la totalité des acides aminés constituant la séquence.

L'expression « au minimum de 5% de motifs XAP » signifie que ladite protéine peut comprendre ou être constituée de 5% à 100% de motifs XAP, et notamment 5%, 10%, 15%, 20%, 25%, 30%, 35%, 40%, 45%, 50%, 55%, 60%, 65%, 70%, 75%, 80%, 85%, 90%, 95%, 100%.

Lesdits motifs XAP peuvent être répartis sur toute la longueur de la séquence, ou bien être localisés sur une partie seulement de la séquence. Dans ce dernier cas, la séquence de ladite protéine comprend ainsi une succession de motifs XAP, par exemple XAP-XAP-XAP-XAP ou bien une succession de motifs XAP intercalés par des acide aminés (AA) autres que la valine, l'alanine ou la proline, par exemple AA-XAP-XAP-AA-XAP-XAP-XAP-AA.

Etant entendu, comme cela a été précédemment mentionné, que le terme « XAP » doit s'entendre comme le tripeptide VAP ou le dipetide AP, ladite protéine comportant ou étant constituée d'au moins 0,05% à <5% ou d'au moins 5% de motifs XAP, peut être constituée d'au moins 0,05% à <5% ou d'au moins 5% de motifs VAP ou d'au moins 0,05% à <5% ou d'au moins 5% de peptide AP ou d'au moins 0,05% à <5% ou d'au moins 5% de motifs VAP et AP, ou bien d'au moins 0,05% à <5% ou d'au moins 5% de motifs VAP et d'au moins 0,05% à <5% ou d'au moins 5% de motifs AP.

Lorsque ladite protéine comprend au moins 0,05% à <5% ou d'au moins 5% de motifs VAP et AP, la séquence de cette dernière comprend des motifs VAP et des motifs AP, la somme des pourcentages des motifs VAP et AP par rapport à la totalité des acides aminés constituant la séquence devant être au minimum de 0,05% à <5% ou de 5%.

Lorsque ladite protéine comprend au moins 0,05% à <5% ou au moins 5% de motifs VAP et AP, les motifs VAP et AP peuvent être répartis sur toute la longueur de la séquence, ou bien être localisés sur une partie seulement de la séquence. Lesdits motifs VAP peuvent être répartis séparément des motifs AP, ou bien les motifs VAP peuvent être regroupés avec les motifs AP.

Dans un aspect de la description, ladite composition comprenant ou consistant en au moins un hydrolysat est utilisée dans la prévention et/ou le traitement de pathologies liées à l'alpha-glucosidase, notamment le diabète de type 2.

Dans un autre aspect de la description, l'hydrolysat est utilisé en tant qu'inhibiteur de l'alpha-glucosidase.

Dans un autre aspect, dans ladite composition comprenant ou consistant en au moins un hydrolysat, ladite protéine comprend ou est constituée d'au moins 0,05% à <5% ou d'au moins 5% de motifs VAP et/ou comprend ou est constituée d'au moins 0,05% à <5% ou d'au moins 5% de motifs AP. Cela signifie que dans ladite composition comprenant ou consistant en au moins un hydrolysat, ladite protéine comprend ou est constituée d'au moins 0,05% à <5% ou d'au moins 5% de motifs VAP, ou ladite protéine comprend ou est constituée d'au moins 0,05% à <5% ou d'au moins 5% de motifs AP. Cela signifie aussi que dans ladite composition comprenant ou consistant en au moins un hydrolysat, ladite protéine comprend ou est constituée d'au moins 0,05% à <5% ou d'au moins 5% de motifs VAP et ladite protéine comprend ou est constituée d'au moins 0,05% à <5% ou d'au moins 5% de motifs AP.

Dans un aspect de la description, dans ladite composition comprenant ou consistant en au moins un hydrolysat, ladite protéine comprend ou est constituée d'au moins 0,05% à <5% ou d'au moins 5% de motifs VAP et AP.

Dans un autre aspect particulier, la description concerne ainsi une composition pour son utilisation dans la prévention et/ou le traitement de pathologies liées à l'alpha-glucosidase comprenant ou consistant en au moins un hydrolysat d'au moins une protéine, ladite protéine comportant ou étant constituée d'au moins 0,05% à <5% ou d'au moins 5% de motifs XAP, dans lequel X représente l'ensemble vide ou une valine, en association avec au moins un peptide de type APX'.

Dans un autre aspect particulier, la description concerne ainsi une composition pour son utilisation dans la prévention et/ou le traitement de pathologies liées à l'alpha-glucosidase comprenant ou consistant en au moins un hydrolysat d'au moins une protéine, ladite protéine comportant ou étant constituée d'au moins 0,05% à <5% ou d'au moins 5% de motifs XAP, dans lequel X représente l'ensemble vide ou une valine, en association avec au moins un peptide APFPE (SEQ ID NO : 1) et/ou APFPEVF (SEQ ID NO : 2).

Ainsi, dans un aspect particulier de la description, la composition pour son utilisation dans la prévention et/ou le traitement de pathologies liées à l'alpha-glucosidase comprend ou est constituée au moins des associations suivantes :

- un hydrolysat d'au moins une protéine, ladite protéine comportant ou étant constituée d'au moins 0,05% à <5% ou d'au moins 5% de motifs AP + le peptide APFPE (SEQ ID NO : 1), ou

- un hydrolysat d'au moins une protéine, ladite protéine comportant ou étant constituée d'au moins 0,05% à <5% ou d'au moins 5% de motifs AP + le peptide APFPEVF (SEQ ID NO : 2), ou
- un hydrolysat d'au moins une protéine, ladite protéine comportant ou étant constituée d'au moins 0,05% à <5% ou d'au moins 5% de motifs VAP + le peptide APFPE (SEQ ID NO : 1), ou
- un hydrolysat d'au moins une protéine, ladite protéine comportant ou étant constituée d'au moins 0,05% à <5% ou d'au moins 5% de motifs VAP + le peptide APFPEVF (SEQ ID NO : 2), ou
- un hydrolysat d'au moins une protéine, ladite protéine comportant ou étant constituée d'au moins 0,05% à <5% ou d'au moins 5% de motifs AP + le peptide APFPE (SEQ ID NO : 1) + le peptide APFPEVF (SEQ ID NO : 2), ou
- un hydrolysat d'au moins une protéine, ladite protéine comportant ou étant constituée d'au moins 0,05% à <5% ou d'au moins 5% de motifs VAP + le peptide APFPE (SEQ ID NO : 1) + le peptide APFPEVF (SEQ ID NO : 2), ou
- un hydrolysat d'au moins une protéine, ladite protéine comportant ou étant constituée d'au moins 0,05% à <5% ou d'au moins 5% de motifs AP + d'un hydrolysat d'au moins une protéine, ladite protéine comportant ou étant constituée d'au moins 0,05% à <5% ou d'au moins 5% de motifs VAP + le peptide APFPE (SEQ ID NO : 1) + le peptide APFPEVF (SEQ ID NO : 2), ou
- un hydrolysat d'au moins une protéine, ladite protéine comportant ou étant constituée d'au moins 0,05% à <5% ou d'au moins 5% de motifs AP + d'un hydrolysat d'au moins une protéine, ladite protéine comportant ou étant constituée d'au moins 0,05% à <5% ou d'au moins 5% de motifs VAP + le peptide APFPE (SEQ ID NO : 1), ou
- un hydrolysat d'au moins une protéine, ladite protéine comportant ou étant constituée d'au moins 0,05% à <5% ou d'au moins 5% de motifs AP + d'un hydrolysat d'au moins une protéine, ladite protéine comportant ou étant constituée d'au moins 0,05% à <5% ou d'au moins 5% de motifs VAP + le peptide APFPEVF (SEQ ID NO : 2).

Dans un autre aspect particulier, la présente la description concerne aussi une composition pharmaceutique comprenant ou consistant en au moins un hydrolysat d'au moins une protéine, ladite protéine comportant ou étant constituée d'au moins 0,05% à <5% ou d'au moins 5% de motifs XAP, dans lequel X représente l'ensemble vide ou une valine.

Dans un autre aspect particulier, ladite composition pharmaceutique comprenant ou consistant en au moins un hydrolysat peut également comprendre au moins un peptide de type APX'.

Dans un aspect particulier, ladite composition pharmaceutique comprenant ou consistant en au moins un hydrolysat peut être administrée sous forme de comprimés, de gélules, de poudres, de pastilles, de pilules, de granulés ou toute autre forme administrable par voie orale, et être sous forme de sachets de poudre, d'ampoules de liquide, de flacons munis de compte-gouttes et les autres formes analogues de préparations liquides ou de poudre.

Dans un aspect particulier de la description, l'hydrolysat peut également être incorporé dans une matrice alimentaire. Une matrice alimentaire signifie des boissons, yaourts, confiserie, céréales, soupes, sauces jus fruits et légumes, matières grasses, assaisonnements, pain).

De préférence, ladite composition pharmaceutique comprenant ou consistant en au moins un hydrolysat est sous forme de comprimés et pourra être avalée avec un peu d'eau au début des repas ou bien croquée lors des premières bouchées de nourriture.

En plus de l'aspect thérapeutique mentionné ci-dessus, les hydrolysats d'au moins une protéine comportant ou étant constituée d'au moins 0,05% à <5% ou d'au moins 5% de motifs XAP selon la présente description, peuvent également être utilisés dans le domaine nutraceutique ou dans le domaine alimentaire (en tant que complément alimentaire par exemple)

La description concerne ainsi l'utilisation d'au moins un hydrolysat d'au moins une protéine, ladite protéine comportant ou étant constituée d'au moins 0,05% à <5% ou d'au moins 5% de motifs XAP, dans lequel X représente l'ensemble vide ou une valine, pour la préparation d'une composition nutraceutique ou d'un complément alimentaire.

Dans un aspect particulier, dans ladite utilisation d'au moins un hydrolysat d'au moins une protéine, ladite protéine comprend ou est constituée d'au moins 0,05% à <5% ou d'au moins 5% de motifs VAP.

Dans un aspect particulier, dans ladite utilisation d'au moins un hydrolysat d'au moins une protéine, ladite protéine comprend ou est constituée d'au moins 0,05% à <5% ou d'au moins 5% de motifs AP.

Dans un aspect particulier, dans ladite utilisation d'au moins un hydrolysat d'au moins une protéine, ladite protéine comprend ou est constituée d'au moins 0,05% à <5% ou d'au moins 5% de motifs VAP et AP.

Dans un aspect particulier, dans ladite utilisation d'au moins un hydrolysat d'au moins une protéine, ladite protéine comprend ou étant constituée d'au moins 0,05% à <5% ou d'au moins 5% de motifs VAP et ladite protéine comprend ou est constituée d'au moins 0,05% à <5% ou d'au moins 5% de motifs AP.

Dans un autre aspect particulier, la description concerne une composition alimentaire ou nutraceutique comprenant ou consistant en au moins un hydrolysat d'au moins une protéine, ladite protéine comportant ou étant constituée d'au moins 0,05% à <5% ou d'au moins 5% de motifs XAP, dans lequel X représente l'ensemble vide ou une valine.

Dans un autre aspect particulier, ladite composition alimentaire ou nutraceutique comprenant ou consistant en au moins un hydrolysat peut également comprendre au moins un peptide de type APX'.

Dans un aspect particulier, ladite composition alimentaire ou nutraceutique comprenant ou consistant en au moins un hydrolysat peut être administrée sous forme de comprimés, de gélules, de poudres, de pastilles, de pilules, de granulés ou toute autre forme administrable par voie orale, et être sous forme de sachets de poudre, d'ampoules de liquide, de flacons munis de compte-gouttes et les autres formes analogues de préparations liquides ou de poudre.

De préférence, ladite composition alimentaire ou nutraceutique comprenant ou consistant en au moins un hydrolysat est sous forme de comprimés et pourra être avalée avec un peu d'eau au début des repas ou bien croquée lors des premières bouchées de nourriture.

Dans un autre aspect, la description concerne aussi un procédé de préparation d'un hydrolysat d'au moins une protéine comportant ou étant constituée d'au moins 0,05% à <5% ou d'au moins 5% de motifs XAP, dans lequel X représente l'ensemble vide ou une valine.

Ledit hydrolysat peut être obtenu par voir chimique ou par voie enzymatique.

L'hydrolyse chimique est réalisée à l'aide d'un acide fort, par exemple HCl en quantité 3M, de 12 heures à 24 heures.

Ladite hydrolyse chimique est également réalisée dans des conditions strictes de pH, plus particulièrement à pH 2.

Il est à noter toutefois qu'une telle hydrolyse chimique peut altérer la qualité de l'hydrolysat obtenu. L'hydrolyse par voie enzymatique est ainsi préférée.

Dans un aspect, la description concerne ainsi un procédé de préparation d'un hydrolysat d'au moins une protéine comportant ou étant constituée d'au moins 0,05% à <5% ou d'au moins 5% de motifs XAP, dans lequel X représente l'ensemble vide ou une valine, comprenant les étapes suivantes :

- La dissolution d'au moins une protéine comportant ou étant constituée d'au moins 0,05% à <5% ou d'au moins 5% de motifs XAP dans de l'eau afin d'obtenir une solution aqueuse ;
- L'ajout d'au moins une enzyme à ladite solution aqueuse en quantité appropriée pour hydrolyser ladite protéine.

« L'ajout d'au moins une enzyme » signifie que l'hydrolysat peut être réalisé par un mélange d'enzymes ou par une succession d'hydrolyse.

Une « succession d'hydrolyse » signifie au moins deux hydrolyses (l'enzyme A puis l'enzyme B par exemple, l'enzyme A servant à réaliser l'hydrolyse maximale).

Dans un aspect, la description concerne ainsi un procédé de préparation d'un hydrolysat d'au moins une protéine comportant ou étant constituée d'au moins 0,05% à <5% ou d'au moins 5% de motifs XAP, dans lequel X représente l'ensemble vide ou une valine, comprenant les étapes suivantes :

- La dissolution d'au moins une protéine comportant ou étant constituée d'au moins 0,05% à <5% ou d'au moins 5% de motifs XAP dans de l'eau afin d'obtenir une solution aqueuse ;
- L'ajout d'une enzyme à ladite solution aqueuse en quantité appropriée pour hydrolyser ladite protéine.

Dans un aspect particulier de la description, la dissolution d'au moins une protéine dans de l'eau consiste en un broyage d'au moins une source de protéines.

Cela signifie donc que la matière première de départ dudit procédé de préparation d'un hydrolysat peut être soit une protéine, notamment une protéine purifiée, commercialement disponible, soit un broyat d'au moins une source de protéine.

Des protéines purifiées sont par exemple commercialement disponibles chez BNLfood (Belovo) (http://www.bnlfood.com/) ; Setalg (http://www.setalg.fr/) ; Copalis (http://www.copalis.fr/fr/) ; Vegan (http://www.veganproteins.com/) ; (Solabia (http://www.solabia.fr)...

Un « broyat d'au moins une source de protéine » peut par exemple être une farine animale, par exemple une farine de poisson, ou bien un broyat de chair de poisson. Ladite source de protéine peut par exemple être d'origine végétale, d'origine marine ou d'origine animale, voire issus d'insectes.

Lorsque la matière première de départ dudit procédé de préparation d'un hydrolysat est sous forme de poudre (une farine de poisson par exemple), alors une simple dissolution est nécessaire.

Lorsque la matière première de départ dudit procédé de préparation d'un hydrolysat sont des co-produits riches en protéines (par exemple des filets de poissons, un tourteau, des algues, etc...), alors il est nécessaire de faire un broyage.

Dans un aspect particulier, la description concerne aussi un procédé de préparation d'un hydrolysat tel que mentionné ci-dessus, ledit procédé comprenant les étapes suivantes :

- La dissolution d'au moins une protéine comportant ou étant constituée d'au moins 0,05% à <5% ou d'au moins 5% de motifs XAP dans de l'eau afin d'obtenir une solution aqueuse ;

- L'incubation de ladite solution aqueuse pendant 15 minutes à 90°C ;
- L'ajout d'au moins une enzyme à ladite solution aqueuse en quantité appropriée pour hydrolyser ladite protéine ;
- L'incubation de la solution aqueuse obtenue à l'étape précédente pendant une période comprise de 6 à 24 heures, à une température comprise de 35°C à 55°C.

Dans un autre aspect particulier, la description concerne aussi un procédé de préparation d'un hydrolysat tel que mentionné ci-dessus, ledit procédé comprenant les étapes suivantes :

- La dissolution d'au moins une protéine comportant ou étant constituée d'au moins 0,05% à <5% ou d'au moins 5% de motifs XAP dans de l'eau afin d'obtenir une solution aqueuse ;
- L'incubation de ladite solution aqueuse pendant 15 minutes à 90°C ;
- L'ajout d'une enzyme à ladite solution aqueuse en quantité appropriée pour hydrolyser ladite protéine ;
- L'incubation de la solution aqueuse obtenue à l'étape précédente pendant une période comprise de 6 à 24 heures, à une température comprise de 35°C à 55°C.

L'incubation mentionnée ci-dessus vise à dénaturer les protéines et favoriser la protéolyse.

L'expression « une période comprise de 6 à 24 heures » doit s'entendre comme toutes les heures de 6 à 24 heures, à savoir 6 heures, 7 heures, 8 heures, 9 heures, 10 heures, 11 heures, 12 heures, 13 heures, 14 heures, 15 heures, 16 heures, 17 heures, 18 heures, 19 heures, 20 heures, 21 heures, 22 heures, 23 heures, et 24 heures. Cela signifie aussi 6 heures et 30 minutes, 7 heures et 30 minutes, 8 heures et 30 minutes, 9 heures et 30 minutes, 10 heures et 30 minutes, 11 heures et 30 minutes, 12 heures et 30 minutes, 13 heures et 30 minutes, 14 heures et 30 minutes, 15 heures et 30 minutes, 16 heures et 30 minutes, 17 heures et 30 minutes ; 18 heures et 30 minutes ; 19 heures et 30 minutes ; 20 heures et 30 minutes ; 21 heures et 30 minutes ; 22 heures et 30 minutes ; 23 heures et 30 minutes et 24 heures et 30 minutes.

L'expression « température comprise de 35°C à 55°C » doit s'entendre comme toutes les températures comprises de 35°C à 55°C, à savoir 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, et 55°C.

Dans un aspect particulier du procédé de préparation tel que mentionné ci-dessus, la quantité appropriée d'enzyme pour hydrolyser une solution de protéines de 5 à 25% (p/p) est de 0,1% à 5% du poids de l'extrait protéique.

Dans un aspect particulier du procédé de préparation tel que mentionné ci-dessus, ledit procédé comprend une ou plusieurs étapes d'ajustement du pH en fonction de l'enzyme utilisée, notamment par ajout de d'HCl ou de KOH et/ou NaOH en quantité appropriée pour obtenir un pH compris entre 3,5 et 8.

Dans un aspect particulier du procédé de préparation tel que mentionné ci-dessus, ledit procédé comprend une ou plusieurs étapes d'inactivation de l'enzyme.

L'enzyme peut être inactivée en augmentant la température du milieu réactionnel à 95°C pendant 15 minutes afin de procéder à l'arrêt de la protéolyse par dénaturation thermique de l'enzyme.

Dans un aspect particulier du procédé de préparation tel que mentionné ci-dessus, ledit procédé comprend une étape de séparation dudit hydrolysat obtenu du reste du mélange réactionnel.

Dans un aspect particulier du procédé de préparation tel que mentionné ci-dessus, la séparation de l'hydrolysat d'au moins une protéine du reste du mélange réactionnel est réalisée par centrifugation à une vitesse comprise de entre 4000 et 7000 RPM, puis élimination du culot obtenu.

Dans un autre aspect particulier du procédé de préparation tel que mentionné ci-dessus, ledit procédé comprend également une étape de filtration préalablement à ladite étape de centrifugation. La filtration du milieu réactionnel permet d'éliminer les matières solides.

L'hydrolyse enzymatique est réalisée par une enzyme soigneusement sélectionnée pour permettre d'obtenir un hydrolysat d'au moins une protéine comportant ou étant constituée d'au moins 0,05% à <5% ou d'au moins 5% de motifs XAP.

L'hydrolyse enzymatique est réalisée par une préparation d'enzyme purifiée ou par un mélange non purifiée. La préparation enzymatique peut contenir des endo ou exo-peptidases, des protéases ou une mixture.

Dans un aspect particulier du procédé de préparation tel que mentionné ci-dessus, l'enzyme est choisie parmi l'alcalase, la Flavourzyme, la peptidase, la promod, la pepsine, la trypsine, la protéase N ou la Protamex.

Dans un aspect particulier du procédé de préparation tel que mentionné ci-dessus, la préparation enzymatique utilisée est Flavourzyme (mélange protéases/peptidases).

Dans un autre aspect particulier du procédé de préparation tel que mentionné ci-dessus, la préparation enzymatique utilisée est Protamex (mélange de protéases (l'alcalase de *Bacillus licheniformis* et la neutrase de *Bacillus amyloquefaciens*)).

Dans un autre aspect, l'hydrolyse est réalisée par une succession d'enzyme, à savoir la Protamex, puis la pepsine.

Par ailleurs, dans un aspect particulier de la description, l'hydrolysat d'au moins une protéine telle que mentionnée ci-dessus peut être utilisé seul ou en combinaison avec d'autres molécules.

Aux fins de la présente invention, lesdits peptides VAP et AP peuvent être des peptides de synthèse, des peptides d'origine végétale, d'origine marine ou d'origine animale, voire des peptides issus de protéines d'insectes.

Il en est de même des peptides contenus dans les hydrolysats précédemment définis.

Les peptides d'origine végétale peuvent ainsi être issus des protéines des légumineuse des protéines des céréales ; des protéines des graines oléagineuses ; ou des protéines des fruits oléagineux.

Les peptides d'origine marine peuvent ainsi être issus des protéines de poissons ou des protéines d'algues.

Les peptides d'origine animale peuvent ainsi être issus des protéines d'oeuf ou des protéines de lait.

Les peptides issus d'insectes peuvent ainsi être issus des protéines d'insectes comestibles.

Toujours aux fins de la présente description, la protéine comportant ou étant constituée d'au moins 0,05% à <5% ou d'au moins 5% de motifs XAP est également choisie parmi la protéine de poissons, la protéine d'algues, la protéine de lait, la protéine de légumineuses, la protéine de céréales, la protéine des graines oléagineuses, la protéine des fruits oléagineux et la protéine d'insectes comestibles.

Dans un aspect particulier de la description, ladite protéine de poisson est ainsi choisie parmi les protéines de carpe, de saumon, de sardine, de lieu, de cabillaud et d'aiglefin.

Dans un autre aspect particulier, ladite protéine d'algues est choisie parmi les protéines de *chondrus*, *palmaria*, *ulva*, *porphyra, laminaria*, *ascophyllum*, *undaria et himanthallia.*

Dans un autre aspect particulier, ladite protéine d'oeuf est choisie parmi l'ovomucine, le lysozyme et l'ovotransferrine.

Dans un autre aspect particulier, ladite protéine de lait est choisie parmi les protéines du petit lait et de la caséine. Le lait peut notamment provenir de vache, de jument ou de brebis. Plus particulièrement, lesdites protéines de lait peuvent être une bêta-lactoglobuline, une caséine, notamment une alpha-S1-caséine ou une Beta-caséine, une lactoferrine.

Dans un autre aspect particulier, ladite protéine des légumineuses est choisie parmi les protéines de lentilles, de haricots blancs et verts, de pois chiches, de fèves, de pois cassés et de soja. Plus particulièrement, ladite protéine peut-être une légumine, notamment une légumine A.

Dans un autre aspect particulier, ladite protéine des céréales est choisie parmi les protéines de maïs, de millet, d'orge, de seigle, de sarrasin, de quinoa et de riz.

Dans un autre aspect particulier, ladite protéine des graines oléagineuses est choisie parmi les protéines d'arachide, de citrouille, de lin, de courge.

Dans un autre aspect particulier, ladite protéine des protéines des fruits oléagineux est choisie parmi les protéines d'amandes, de noix, de cacahuètes, de noisettes, de pignons de pin, de pistaches, d'argan et d'olivier.

Dans un autre aspect particulier, ladite protéine des protéines d'insectes comestibles étant choisie parmi les protéines de grillons, de criquets, de sauterelles et de vers de farine.

La protéine selon la description peut également être une protéine fibreuse, notamment l'élastine, le collagène ou l'actine. L'actine peut notamment être d'origine marine (issue de saumon), l'élastine d'origine animale (issue de bovin), et le collagène d'origine animale ou marine (issu de bovin ou de saumon).

L'invention sera mieux illustrée par les exemples et les figures suivantes. Les exemples ci-après visent à éclaircir l'objet de l'invention et illustrer des modes de réalisation avantageux, mais en aucun cas vise à restreindre la portée de l'invention.

Légende des figures :

La **Figure 1** représente l'évolution du pourcentage d'inhibition de l'activité de l'alpha-glucosidase en fonction de la concentration en acarbose.
L'abscisse représente la concentration en acarbose (en mM), et l'ordonnée représente le pourcentage d'inhibition de l'alpha-glucosidase.
La **Figure 2** représente l'évolution du pourcentage d'inhibition de l'activité de l'alpha-glucosidase en fonction de la concentration en peptide AP.
L'abscisse représente la concentration en peptide AP (en mM), et l'ordonnée représente le pourcentage d'inhibition de l'alpha-glucosidase.
La **Figure 3** représente l'évolution du pourcentage d'inhibition de l'activité de l'alpha-glucosidase en fonction de la concentration en peptide VAP.
L'abscisse représente la concentration en peptide VAP (en mM), et l'ordonnée représente le pourcentage d'inhibition de l'alpha-glucosidase.

La **Figure 4** représente la linéarisation de Lineweavers-Burk de la réaction d'hydrolyse du p-NPG (le p-nitro phenyl glucopyranoside) en présence de différentes concentrations de l'inhibiteur peptidique LKP.

L'abscisse représente la concentration du substrat (en $1/[p\text{-}NPG]$ en $mole^{-1}$), et l'ordonnée représente la vitesse en ($1/Vi$ en $\mu mole^{-1}.min^{-1}$).

Les quatre droites représentent les concentrations en inhibiteurs suivantes :

- Cercle plein : pas d'inhibiteur
- Cercle vide : test en présence de 5 mM de LKP
- Triangle plein : test en présence de 6 mM de LKP
- Triangle vide : test en présence de 7,5 mM de LKP.

Cette linéarisation permet de déterminer les constantes de l'enzyme Km et Vmax par la représentation des inverses.

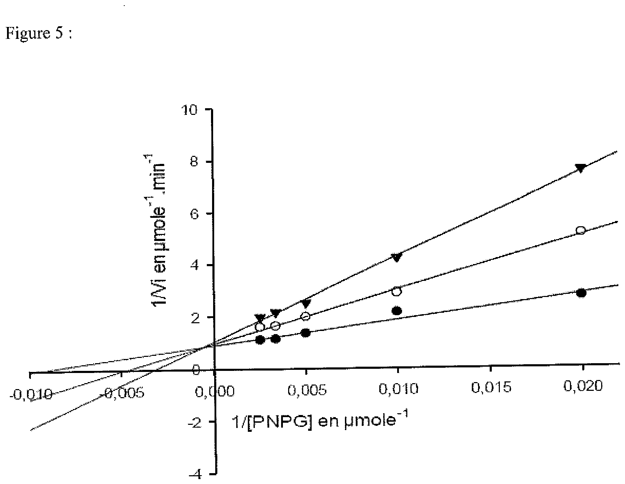

La **Figure 5** représente la linéarisation de Lineweavers-Burk de la réaction d'hydrolyse du p-NPG (le p-nitro phenyl glucopyranoside) en présence de différentes concentrations de l'inhibiteur peptidique AP.

L'abscisse représente la concentration du substrat (en $1/[p\text{-}NPG]$ en $\mu mole^{-1}$), et l'ordonnée représente la vitesse en ($1/Vi$ en $\mu mole^{-1}.min^{-1}$).

Les trois droites sont les suivantes :

- Cercle plein : sans enzyme
- Cercle vide : test en présence de 50 $\mu$M de AP
- Triangle plein : test en présence de 100 $\mu$M de AP

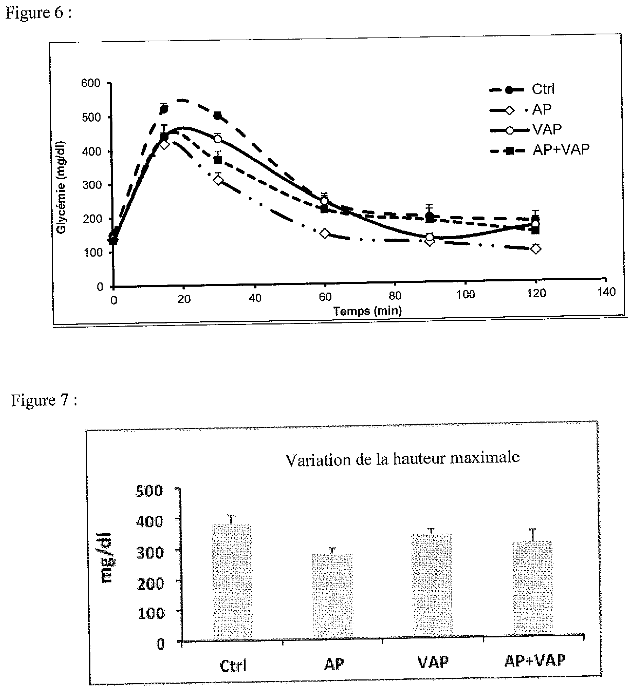

La **Figure 6** représente l'évolution de la glycémie (en valeurs brutes, c'est-à-dire la glycémie totale) au cours du test de tolérance au maltose.

L'abscisse représente le temps (en minutes) et l'ordonnée représente la glycémie (mg/dl).

La **Figure 7** représente les valeurs maximales de glycémie retranchées de la valeur au repos du test oral de tolérance au maltose.

La valeur au repos est de 150 mg/dl. La glycémie basale (au repos) a été mesurée 5 minutes avant la première administration par voie gastrique. En prenant comme référence la condition contrôle (solution saline administrée avant le gavage au maltose), la glycémie basale moyenne était de 143±34,2 mg/dl.

L'abscisse représente les groupes avec les différents peptides testés et l'ordonnée représente la glycémie (mg/dl).

Les groupes avec les différents peptides testés sont les suivants :

- Ctrl représente le groupe contrôle
- AP représente le groupe testé avec le peptide AP
- VAP représente le groupe testé avec le peptide VAP
- AP+VAP représente le groupe testé avec le peptide AP+VAP

La variation de la hauteur maximale représente le $\Delta_{Peak}$ c'est-à-dire la glycémie totale maximale moins la glycémie au repos. Plus précisément, cela représente la variation maximale de la glycémie liée à la condition expérimentale testée, c'est-à-dire la valeur de la glycémie la plus élevée moins la valeur basale mesurée 5 minutes avant la première administration par voie gastrique.

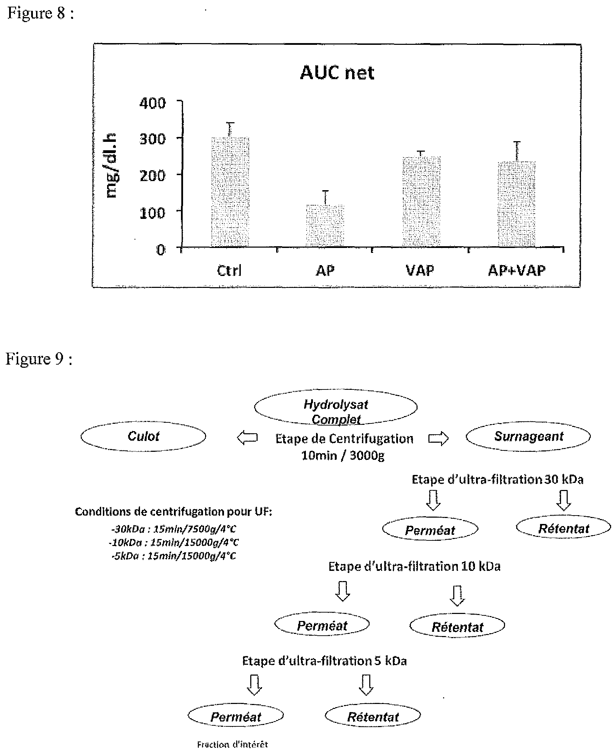

La **Figure 8** représente l'aire sous la courbe retranchée de la valeur de repos pour les résultats des quatre groupes testés précédemment mentionnés (Ctrl, AP, VAP, AP+VAP).

L'abscisse représente les groupes avec les différents peptides testés et l'ordonnée représente la valeur de l'aire (mg/dl.heure = mg/dl.h).

La **Figure 9** représente la méthodologie utilisée pour fractionner et concentrer les peptides de bas poids moléculaire issus de l'hydrolyse des protéines de lactosérum caprin.

UF signifie l'ultrafiltration.

30kDa : 15min/7500/4°C signifie une filtration sur filtre 30KDa pendant 15 minutes à 7500g à 4°C.

10 kDa : 15min/15000g/4°C signifie une filtration sur filtre 10KDa pendant 15 minutes à 15000g à 4°C.

5 kDa : 15min/15000g/4°C signifie une filtration sur filtre 5KDa pendant 15 minutes à 15000g à 4°C.

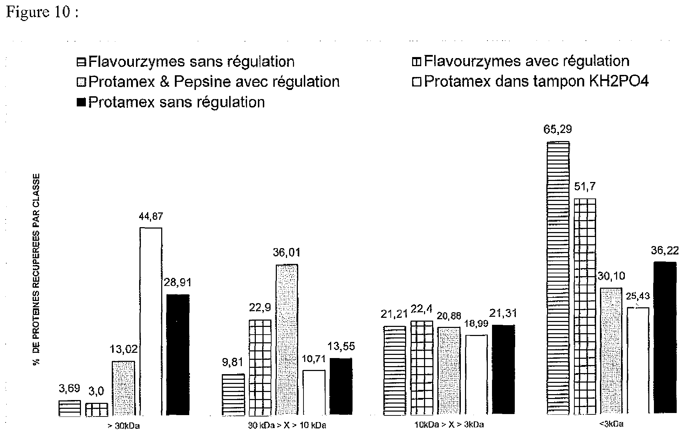

La **Figure 10** représente le profil de masse des hydrolysats de protéines réalisés par une enzyme (Flavourzymes ou Protamex), avec ou sans régulation du pH.

L'abscisse représente la taille des hydrolysats (plus précisément le poids moléculaire et l'ordonnée représente le pourcentage de protéines récupérées par classe (c'est-à-dire par fraction moléculaire). Les différentes fractions moléculaires sont les suivantes :

> 30kDa : protéines et peptides avec masse supérieure à 30 kDa,

30 kDa > X > 10 kDa : protéines et peptides avec masse comprise entre 30 et 10 kDa,

10 kDa > X > 3 kDa : protéines et peptides avec masse comprise entre 10 et 3 kDa,

< 3 kDa : protéines et peptides avec masse inférieure à 3 kDa,

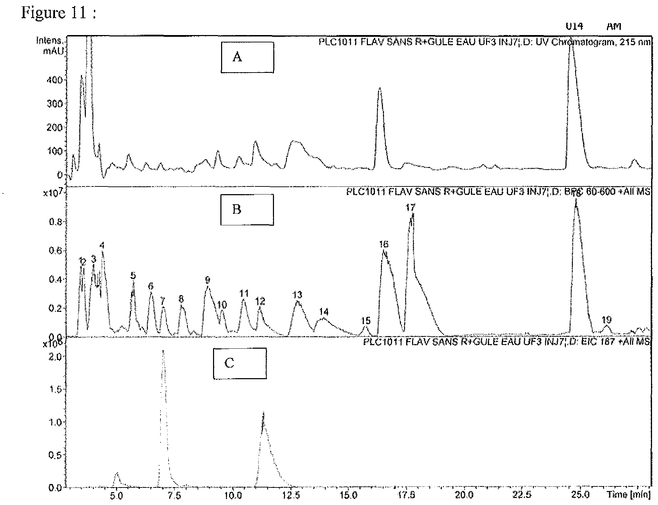

La **Figure 11** représente l'analyse LC-MS (HPLC-MS) sur colonne Waters BEH de l'hydrolysat de CPL par Flavourzymes dans de l'eau ultrapure.

L'abscisse représente le temps (en minutes), et l'ordonnée représente l'intensité (en mAU [AU = Unité arbitraire]).

Le cas A représente le spectre UV, unité arbitraires en mAU, pour l'UV à 215 nm ;

Le cas B représente le spectre de masse complet en nombres d'ions ;

Le cas C représente le spectre de masse de l'ion m/z 187.

## EXEMPLES :

**Exemple 1** : Test d'inhibition *in vitro* de l'activité de l'alpha-glucosidase en présence de différents peptides synthétiques

### Matériel et Méthodes

Le test d'inhibition de l'activité de l'alpha-glucosidase en présence de peptides synthétiques VW, VY, IY, KY, VY, KW, AP, LKP, GPL, VAP, et AKK a été réalisé selon le protocole suivant (d'après celui décrit dans Kang et al., Journal of Medicinal Plants Research 2012, 6:2850-2856). Les peptides synthétiques ont été fournis par GENOSPHERE Biotechnologies.

L'alpha-glucosidase qui a été utilisée est l'alpha-glucosidase recombinante de *Saccharomyces cerevisiae* qui est une maltase.

20 µL d'alpha-glucosidase dans du tampon 0,1 mol/L phosphate de sodium (pH 6,8) à une concentration finale de 0,2 U/mL ont été mélangés avec 8 µL de l'échantillon de peptide ou d'acarbose (commercialisé par Bayer AG sous le nom Glucor) à différentes concentrations (0,01 à 50 mmol/L). Les échantillons étaient préalablement solubilisés dans de l'eau déionisée contenant 10% de DMSO (diméthyl sulfoxide).

L'acarbose, inhibiteur synthétique commercial, considéré comme inhibiteur référant de l'alpha-glucosidase, est ici utilisé comme contrôle positif.

Après une incubation à 37°C pendant 20 minutes, 20 µL du substrat p-NPG (le p-nitro phenyl glucopyranoside) à 2,5 mM (préparé dans le même tampon précédemment cité) ont été ajoutés au mélange afin d'initier la réaction.

Le milieu réactionnel a été incubé pendant 30 minutes à 37°C puis il a été mis fin à la réaction par l'ajout de 80 µL d'une solution de carbonate de sodium ($Na_2CO_3$) à 0,3M.

La quantité de produit formé (le p-nitro phenyl (p-NP) de couleur jaune) a été mesurée par spectrophotométrie (absorbance à 410 nm, VersaMax™, Microplate Reader.

Le test a été réalisé en microplaque de 96 puits.

Tous les tests d'inhibition ont été réalisés en triplicata.

Le pourcentage d'inhibition a été calculé selon l'équation ci-dessous :

$$\% \, d'inhibition = \left[ 1 - \frac{(DO \, test \, echantillon - DO \, blanc \, test)}{(DO \, test \, controle - DO \, blanc \, controle)} \right] * 100$$

DO test échantillon correspond à la densité optique obtenue pour le mélange « échantillon + enzyme + substrat ».

DO blanc test correspond à la densité optique obtenue pour le mélange « échantillon + tampon ».

DO test contrôle correspond à la densité optique obtenue pour le mélange « tampon + enzyme + substrat »

DO blanc contrôle correspond à la densité optique obtenue pour le tampon.

Résultats

Chaque peptide VW, VY, IY, KY, VY, KW, AP, LKP, GPL, VAP, et AKK a été testé selon le protocole décrit ci-dessus dans la partie matériel & méthodes.

Différentes concentrations de ces peptides ont été utilisées pour déterminer leur IC$_{50}$, c'est-à-dire la concentration nécessaire pour inhiber 50% de l'activité de l'alpha-glucosidase.

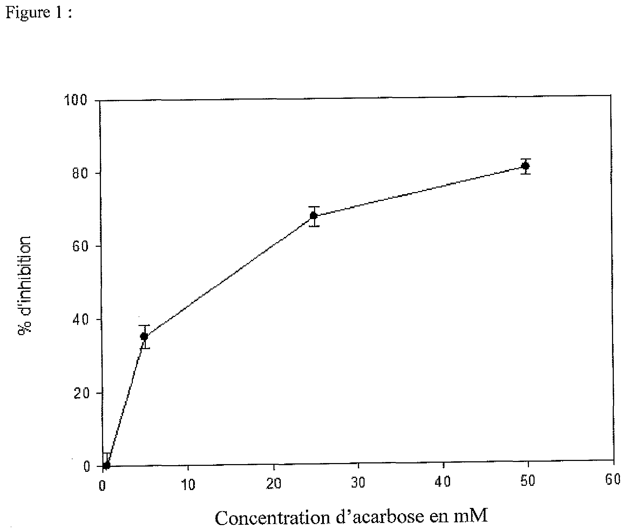

L'évolution du pourcentage d'inhibition en fonction de la concentration en acarbose (contrôle positif) est présentée en **Figure 1.**

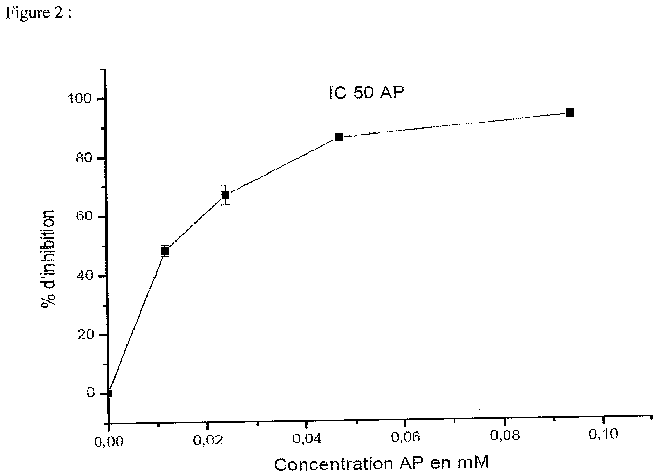

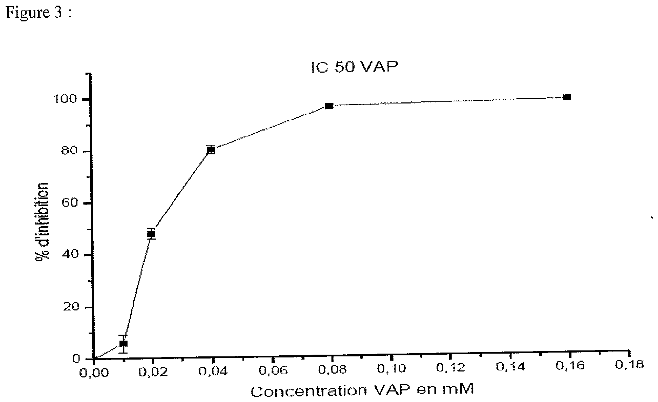

L'évolution du pourcentage d'inhibition en fonction de la concentration en peptide AP et en peptide VAP est présentée respectivement en **Figure 2** et en **Figure 3.**

Les résultats d'IC$_{50}$ des peptides ainsi que ceux de l'acarbose sont présentés dans le Tableau 1 ci-dessous :

| Séquence des peptides | Activité inhibitrice de l'$\alpha$-glucosidase (IC 50) en mM |
|---|---|
| Acarbose | 11,92 $\pm$ 1,44 |
| Miglitol | 39 $\pm$ 0,96 |
| LKP | 7,11 $\pm$ 0,20 |
| GPL | 4,82 $\pm$ 0,15 |
| VIY | Pas d'inhibition |
| AKK | Pas d'inhibition |
| VAP | 0,020 $\pm$ 0,0002 |
| AP | 0,0136 $\pm$ 0,001 |
| IY | 12,27 $\pm$ 0,25 |
| KY | 10,96 $\pm$ 0,1 |
| KW | 12,76 $\pm$ 0,98 |
| VW | Pas d'inhibition |
| VY | 2,7 $\pm$ 0,18 |
| IY | 12,26 $\pm$ 0,25 |

Certains peptides montrent donc une inhibition de l'activité de l'a glucosidase *in vitro*.

Parmi ces peptides, les peptides AP et VAP possèdent l'activité inhibitrice la plus élevée des peptides testés avec des IC$_{50}$ de 13,64 $\pm$0,92$\mu$M et 20,01$\pm$0,21 $\mu$M respectivement.

L'IC$_{50}$ du peptide AP est environ 874 fois plus faible que celle de l'acarbose, contrôle positif (IC$_{50}$=11920$\pm$1444 $\mu$M) alors que celle du peptide VAP est environ 595 fois plus faible.

Ces résultats confirment donc que les peptides VAP, AP, VY, LKP, IY, KY et KW et GPL largement présents dans les protéines de co-produits marins inhibent l'$\alpha$-glucosidase, et en particulier la maltase.

Néanmoins, les peptides LKP, GPL, IY, KY, KW et VY sont de faibles inhibiteurs de l'alpha-glucosidase, par rapport aux peptides AP et VAP. En effet, les peptides IY, KY et KW présentent des IC$_{50}$ proches de celle de l'acarbose, et les peptides LKP, GPL et VY présentent respectivement des IC$_{50}$ environ 355, 243 et 135 fois plus élevées par rapport à celles des peptides AP et VAP.

**Exemple 2 :** Détermination *in vitro* de l'inhibition de l'alpha-glucosidase par des peptides synthétiques - Test numéro 2

Matériel & Méthodes

20 $\mu$l d'$\alpha$-glucosidase dans du tampon 0,1M phosphate de potassium (pH 6,8) à 1,6 U/ml, sont mélangés avec 8 $\mu$l de l'échantillon peptide ou d'inhibiteur de référence à différentes concentrations.

Les échantillons sont préalablement solubilisés dans l'eau milliQ contenant 10 % DMSO (disponible chez Sigma sous la référence 472301).

Après une pré-incubation à 37°C pendant 15 min, 20 $\mu$l du substrat p-NPG à 20 mM dans du tampon 0,1 M phosphate de potassium (pH 6,8) sont ajoutés au mélange afin d'initier la réaction.

Le milieu réactionnel est incubé pendant 30 min à 37°C avant que la réaction ne soit stoppée par l'ajout de 80

μl d'une solution de carbonate de sodium (Na$_2$CO3) à 1M.

La quantité de produit formé (p-nitro phenyl) est mesurée par lecture de l'absorbance à 410 nm en utilisant le spectrophotomètre Fluostar Omega (BMG Labtech, Allemagne).

Tous les tests d'inhibition sont réalisés en triplicata.

L'enzyme utilisée est l'alpha-glucosidase recombinante de *S.cerevisiae* (il s'agit d'une maltase disponible chez Sigma, sous la référence G0660-750UN)

Le substrat utilisé est le 4-Nitrophenyl α-D-glucopyranoside (p-NPG) (disponible chez Sigma sous la référence N1377)

Les peptides ont été fournis par Génosphère et présentent une pureté > 95%

Les inhibiteurs utilisés sont l'Acarbose (disponible chez Sigma sous la référence A8980), le Miglitol (disponible chez Sigma sous la référence M1574), et le Voglibose (disponible chez Sigma sous la référence 50359)

Les résultats d'IC$_{50}$ des peptides testés et de l'acarbose, du miglitol et du voglibose sont présentés dans le Tableau 2 ci-dessous.

| Référence/Séquence des peptides | Activité inhibitrice de l'α-glucosidase | | |
|---|---|---|---|
| | PM (g/mol) | (IC 50) en mmol/l | (IC 50) en mg/ml |
| Acarbose | 645,6 | 11,92± 1,44 | 7,696 |
| Miglitol | 207,22 | 37,475±0,69 | 7,766 |
| Voglibose | 267,28 | 24.41±0.47 | 0,2673 |
| AP | 186 | 0,0136 ± 0,001⁻ | 0,0025 |
| VAP | 285 | 0,020± 0,0002 | 0,0057 |
| SAPLRVY (SEQ ID NO : 3) | 804 | 1.765±0.18 | 1,4191 |
| VAPFPEV (SEQ ID NO : 4) | 757 | 2.68±0.04 | 2,0288 |
| VY | 280 | 2,7 ±0,18 | 0,7560 |
| AVPYQR (SEQ ID NO : 5) | 732 | 3.19±0.18 | 2,3351 |
| VAPG (SEQ ID NO : 6) | 342 | 4.05±0,09 | 1,3851 |
| VGVAPG (SEQ ID NO : 7) | 498 | 4.59±0,06 | 2,2858 |
| GPL | 285 | 4,82 ± 0,15 | 1,3737 |
| LKP | 356 | 7,11 ±0,20 | 2,5312 |
| KY | 309 | 10,96±0,1 | 3,3866 |
| IY | 294 | 12,26±0,25 | 3,6044 |
| KW | 332 | 12,76 ± 0,98 | 4,2363 |
| Nb : PM signifie le Poids Moléculaire | | | |

Les résultats confirment que les peptides AP et VAP, inhibent fortement l'a-glucosidase, par rapport aux 3 références : l'acarbose, le miglitol et le voglibose, des inhibiteurs connus des glucosidases.

**Exemple 3 :** Détermination *in vitro* du type d'inhibition des peptides LKP et AP

A partir des vitesses initiales, les représentations de *Linerweaver-Burk* pour chaque concentration en inhibiteur LKP ont été représentées et le type d'inhibition a été déterminé. Le peptide synthétique LKP a été fourni par GENOSPHERE Biotechnologies.

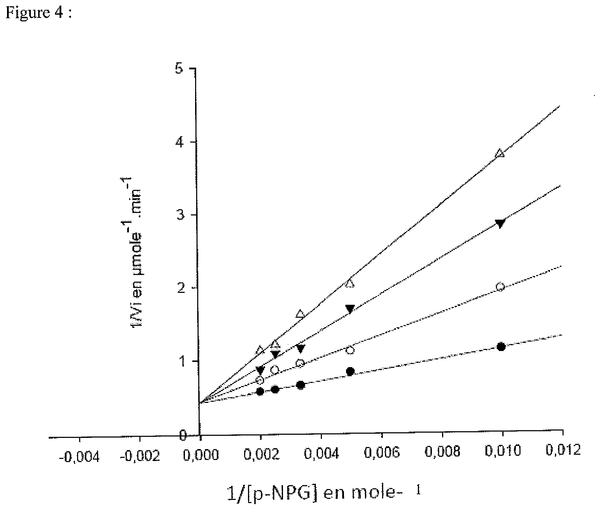

Les résultats sont présentés dans la **Figure 4.**

D'après les résultats, l'inhibition de la dégradation du p-NPG par le peptide LKP est de type compétitif : toutes les droites se coupent au point 1/Vmax.

Cette étude démontre que des peptides LKP ont une activité inhibitrice de l'a-glucosidase pancréatique.

Cette étude a également été réalisée avec le peptide AP qui a été fourni par GENOSPHERE Biotechnologies.

A partir des vitesses initiales, les représentations de *Linerweaver-Burk* pour chaque concentration en inhibiteur AP ont été représentées et le type d'inhibition a été déterminé.

Les résultats sont présentés dans la **Figure 5.**

D'après les résultats, l'inhibition de la dégradation du p-NPG par le peptide AP est de type compétitif : toutes les droites se coupent au point 1/Vmax.

Cette étude démontre que des peptides AP ont une forte activité inhibitrice de l'a-glucosidase pancréatique.

**Exemple 4 :** Test d'inhibition *in vivo* de l'activité de l'alpha-glucosidase en présence de différents peptides synthétiques

Matériel et Méthodes

L'activité inhibitrice des peptides AP et VAP et l'effet des peptides AP et VAP sur la réponse glycémique, peuvent être mesurés lors d'un test oral de tolérance au saccharose et au maltose *in vivo* chez la souris db/db.

Les souris sont âgées de 4 semaines et chaque souris est son propre témoin.

Cinq tests oraux de tolérance au saccharose et/ou maltose (4 g/kg) sont réalisés sur chaque souris avec un minimum de 72 h d'intervalle.

L'ordre est déterminé de façon à annihiler un effet potentiellement confondant d'une modification de la composition corporelle des souris durant les 3 semaines d'étude.

Les 5 tests sont les suivants :

- Un test contrôle (comprenant une solution saline à 0,9%) ;
- Un test avec le peptide AP (à une concentration de 500 mg/kg) ;
- Un test avec le peptide VAP (à une concentration de 500 mg/kg) ;
- Un test avec le peptide AP (à une concentration de 500 mg/kg) et avec le peptide VAP (à une concentration de 500 mg/kg) ;
- Un test avec l'acarbose (à une concentration de 10 mg/kg).

Le saccharose ou le maltose et les produits tests sont dilués dans une solution saline 0,9%, puis administrés directement par voie gastrique.

Cinq minutes après l'administration par voie gastrique, un premier prélèvement sanguin à la queue sera réalisé afin de déterminer la glycémie (t = 0) ; puis 6 autres prélèvements seront effectués après 15, 30, 45, 60, 90 et 120 minutes.

Le critère d'évaluation principal est la mesure de l'aire sous la courbe glycémique sur les 2 heures suivant l'administration du saccharose ou du maltose (AUC, aera under the curve, 0 - 120 minutes, en g*min/L).

L'alpha-glucosidase pouvant être utilisée aux fins des protocoles mentionnés ci-dessus peut par exemple être l'alpha-glucosidase recombinante de *Saccharomyces cerevisiae* qui est une maltase.

**Exemple 5 :** Test d'inhibition *in vivo* de l'activité de l'alpha-glucosidase en présence des peptides synthétiques AP et VAP

L'activité inhibitrice des peptides AP et VAP et l'effet des peptides AP et VAP sur la réponse glycémique, peuvent être mesurés lors d'un test oral de tolérance au maltose *in vivo* chez la souris db/db qui présente une intolérance au glucose d'origine génétique.

Les souris sont âgées de 4 semaines et chaque souris est son propre témoin.

Une prise de maltose (2g/kg) a été utilisée pour provoquer une hausse temporaire de la glycémie.

Le protocole expérimental

Quatre (4) tests oraux de tolérance au maltose (2 g/kg) ont été réalisés sur chaque souris avec un minimum de 48 h d'intervalle. L'ordre était déterminé de façon à annihiler un effet potentiellement confondant d'une modification de la composition corporelle des souris durant les 3 semaines d'étude.

Les 4 tests suivants ont été effectués dans un ordre aléatoire :

- Contrôle (solution saline 0,9%) ;
- Prise du peptide AP (500 mg/kg) ;
- Prise du peptide VAP (500 mg/kg) ;
- Prise des peptides AP (500 mg/kg) + VAP (500 mg/kg)

Le maltose et les produits tests ont été administrés directement par voie orale (gavage gastrique) et dilués dans une solution saline 0,9%.

Prélèvements expérimentaux

Cinq (5) minutes avant l'administration par voie gastrique, un premier prélèvement sanguin à la queue était réalisé afin de déterminer la glycémie (t = 0) ; puis 5 autres prélèvements ont été effectués après 15, 30, 60, 90 et 120 minutes.

Critères d'évaluation

- *Critère principal* : Le critère d'évaluation principal était la mesure de l'aire sous la courbe glycémique sur les 2 heures suivant l'administration du maltose (AUC, aera under the curve, 0 - 120 minutes, en mg*h/dl). Plus l'aire sous la courbe diminue, plus le peptide utilisé est efficace. L'aire sous la courbe est mieux représentative de la réponse glycémique à la charge orale en glucides, car elle prend en compte non seulement le niveau maximal de glycémie atteint, mais également la cinétique d'évolution de la glycémie sur les 2 heures qui suivent la prise de glucides.
- *Critères secondaires* : Les critères d'évaluation secondaires étaient : Cmax glycémie (valeur maximale mesurée au cours des 120 minutes), ΔCmax glycémie (ΔCmax glycémie = Cmax glycémie - valeur de la glycémie à t0 = 150), AUCnet (AUC calculé sur les valeurs de glycémie soustraites de la valeur à t0). L'AUC (aire sous la courbe) a été estimée selon la méthode des trapèzes. Pour l'AUCnet, la valeur de glycémie basale multipliée par le temps de mesure (2h) était retranchée à l'AUC calculée précédemment.

L'évolution de la glycémie au cours du test de tolérance au maltose est présentée dans la **Figure 6.** Elle représente les valeurs brutes (c'est-à-dire la valeur du dosage direct par opposition à la valeur "net" pour laquelle on retranche la valeur de repos). Le test a été effectué avec quatre groupes de 8 souris, et que la courbe a été effectuée avec la moyenne des résultats ?

Les peptides AP et VAP, utilisés seuls ou en combinaison permettent donc de diminuer la glycémie des souris testées.

Les valeurs maximales de glycémie retranchées de la valeur de repos au cours du test oral de tolérance au maltose sont présentées dans la **Figure 7.** Par exemple, la valeur pour AP est d'environ 280 mg/dl (430 - 150 = 280, c'est-à-dire la valeur maximale moins la valeur de repos mesurée 5 minutes avant le gavage. Cela permet de mettre en évidence plus directement l'impact de la condition expérimentale indépendamment de la valeur de glycémie basale qui peut présenter une certaine variabilité).

Ces valeurs démontrent un impact significatif du traitement avec le peptide AP sur la valeur maximale de glycémie obtenue au cours d'un test de tolérance au maltose.

L'impact significatif du peptide AP est confirmé par les résultats d'AUC qui sont présentés dans la **Figure 8.**

En effet, le traitement avec le peptide AP montre un impact bénéfique sur l'AUC glucose au cours du test oral de tolérance au maltose.

**Exemple 6 :** Réalisation d'un hydrolysat protéines de lactosérum de lait caprin avec régulation de pH

Source de Protéines

Concentré de protéines de lactosérum (CPL) isolé à partir de lait caprin brut (80% de protéines)

Enzymes utilisées

**Protamex (EC Number 3.4.21.14) :**

C'est un nom commercial de la marque Novozymes corp. L'enzyme est disponible chez Sigma sous la référence P0029.

Il s'agit d'un mélange de protéases (l'alcalase de *Bacillus licheniformis* et la neutrase de *Bacillus amyloquefaciens*)

L'activité de l'enzyme est de 1,5 U/g de solide.

Numéro de lot : 119K1454V.

**Flavourzymes** :

C'est un nom commercial de la marque Novozymes corp. L'enzyme est disponible chez Sigma sous la référence P6110.

Il s'agit d'un mélange protéases/peptidases *d'Aspergillus oryzae*

L'activité de l'enzyme est de 500 U/g

Numéro de lot : SLBJ3967U

**Endoprotéase Proline spécifique** :

L'enzyme est commercialisé par DSM sous la marque Brewers clarex (l'enzyme est isolée à partir d'*Aspergillus Niger*). L'enzyme est disponible chez Sigma sous la référence E1411

Il s'agit d'une proline spécifique des endopeptidases *de flavobacterium sp.* Plus particulièrement, l'enzyme hydrolyse spécifiquement les liaisons en C-terminal des prolines de la chaîne peptidique

L'activité de l'enzyme est de 5 U/mg.

Numéro de lot : SLBD9700V

**Pepsine :**

L'enzyme est disponible chez Sigma sous la référence P7000. Il s'agit d'une pepsine de muqueuse gastrique de porc.

L'activité de l'enzyme est de 250 U/mg de solide

Numéro de lot : SLBH3879V

Les hydrolyses sont réalisées sur une Station pH-Stat 718 Stat Titrino de Metrohm permettant de réguler la température et le pH, et d'obtenir un suivi des hydrolyses. Cette station est également équipée d'une cellule 728 Stirrer de Metrohm permettant une agitation de 200 à 1900 tour/min.

La solution de protéines de lactosérum de lait caprin correspond à 5% de matière sèche dans de l'eau (ce qui correspond dans l'exemple à 1500 mg dans 30 mL d'eau ultra pure).

Une étape de dénaturation par chauffage de la solution à 80°C pendant 10 minutes est appliquée avant hydrolyse.

Pour arrêter la réaction d'hydrolyse, la solution est portée à 90°C pendant 15 minutes afin de dénaturer les enzymes encore présentes dans le milieu.

L'hydrolysat est ensuite aliquoté puis conservé à -20°C avant purification et analyses.

*1. Cas d'une hydrolyse par une enzyme simple : Flavourzymes*

La solution est portée à une température de 50°C, le pH est fixé à 8,0 par ajout de NaOH 6M. L'hydrolyse commence lors de l'ajout de 750 μL de Flavourzymes (750 μL/30 mL/1500 mg de CPL, 5 % v/w). Le pH est maintenu à la valeur de 8 par ajout de NaOH 0,1M.

*2. Cas d'une double hydrolyse : Protamex et pepsine*

La solution est portée à une température de 50°C, le pH est fixé à 8,0 par ajout de NaOH 6M. L'hydrolyse commence lors de l'ajout de 60 mg de protamex (60 mg/30 mL/1500 mg de CPL, 4 % w/w). Le pH est maintenu à la valeur de 8 par ajout de NaOH 0,1M.

Lorsque l'hydrolyse est arrêtée, le pH est abaissé à une valeur de 2 avant d'ajouter 30 mg de pepsine (30 mg/30 mL/1500 mg de CPL, 2 % w/w). La régulation du pH est cette fois-ci effectuée par ajout de HCl 0,1M.

**Exemple 7 :** Réalisation d'un hydrolysat de protéines de lactosérum de lait caprin sans régulation de pH

Source de Protéines

Concentré de protéines de lactosérum (CPL) isolé à partir de lait caprin brut (80% de protéines)

Enzymes utilisées

Protamex (disponible chez Sigma sous la référence P0029) (60 mg/30 mL/1500 mg de CPL, 4 % w/w) ou Flavourzymes (disponible chez Sigma sous la référence P6110) (750 μL/30 mL/1500 mg de CPL, 5 % v/w).

Les hydrolyses sont réalisées soit dans de l'eau ultrapure soit dans un tampon phosphate de potassium, pH 8,0 et de molarité 50mM.

La solution de protéines de lactosérum de lait caprin correspond à 5% de matière sèche dans du tampon ou dans de l'eau ultrapure.

Une étape de dénaturation par chauffage de la solution à 80°C pendant 10 minutes est appliquée avant hydrolyse.

L'hydrolyse est ensuite réalisée sur des supports Radley Tech Carroussel 6 (il s'agit de réacteurs classiques de la marque Radley) contrôlant la température interne des milieux à 50°C et permettant une agitation de 600 rpm.

Pour arrêter la réaction d'hydrolyse, après 6h, la solution est portée à 90°C pendant 15 minutes afin de dénaturer les enzymes encore présentes dans le milieu.

L'hydrolysat est ensuite aliquoté puis conservé à -20°C avant fractionnement et analyses.

**Exemple 8** : Fractionnement des hydrolysats de protéines de lactosérum de lait caprin

Les hydrolysats précédemment réalisés (exemples 6 et 7) subissent une série de fractionnement permettant la concentration des peptides de bas poids moléculaire ciblés.

Une première étape de centrifugation vise à éliminer les protéines de hauts poids moléculaires non hydrolysés qui se trouvent dans le culot.

Une série d'ultrafiltrations successives de seuil de coupure de 30 à 3 kDa est ensuite appliquée au surnageant qui contient les protéines hydrolysées. Les ultrafiltrations sont réalisées avec des unités à centrifuger de référence Amicon Ultra de chez Millipore (2mL avec des seuils de coupure de 30 kDa, 10 et 3 kDa.). La centrifugeuse utilisée est un modèle Fisher Bioblock Scientific Sigma 3-18K - 3-16K.

La méthodologie utilisée est présentée sur la **Figure 9.**

A chaque stade intermédiaire, une partie des perméats, rétentats, culots et des surnageants est aliquotée puis lyophilisée.

Le résultat d'une centrifugation est l'obtention de deux fractions : le culot (solide, au fond du tube), et le surnageant (fraction liquide).

Le résultat d'une ultrafiltration est l'obtention d'un rétentat et d'un perméat.

Un rétentat est un terme utilisé pour les techniques de séparation avec des membranes décrivant les particules retenues lors d'une filtration. Le contraire du rétentat est le perméat.

Le rétentat est aussi appelé non-filtrat.

Un perméat est le liquide, où les peptides ont été enlevés à l'aide d'une membrane. Le perméat est aussi appelé filtrat.

Les échantillons sont ensuite conservés à -20°C avant analyses.

**Exemple 9** : Détermination du profil de masse des hydrolysats de protéines de lactosérum de lait caprin

A chaque stade de fractionnement, pour les hydrolysats précédemment réalisés (exemples 6 et 7), la quantité de protéines restante par la méthode BCA (BiCinchoninic acid Assay) a été déterminée. Cette quantification permet d'estimer le profil de masse des hydrolysats de protéines.

Réactifs

Les réactifs utilisés sont l'acide bicinchoninic (disponible chez Sigma sous la référence B9643), le sulfate de cuivre II (disponible chez Sigma sous la référence C2284), BSA (Bovine serum Albumine) (disponible chez Sigma sous la référence A7888)

Protocole

En microplaque 96 puits, ajouter 200 µL de réactif BCA, correspondant à un mélange Acide bicinchoninic : Sulfate de Cuivre (II) à un ratio 25 : 0,5 (V:V), à 25 µL de l'échantillon à doser.

Afin de déterminer la concentration, une gamme étalon de BSA est réalisée et testée dans des concentrations comprises entre 0 et 0,6 mg/mL. Une fois l'échantillon ajouté, la réaction est réalisée pendant 30 minutes à 37°C, avant que la lecture de DO (Densité Optique) ne soit faite à 526nm.

Les résultats sont présentés dans la **Figure 10.**

L'utilisation de Flavourzymes au profit de protamex permet de générer une partie plus importante de peptides inférieurs à 3kDa.

Plus particulièrement, l'utilisation de Flavourzymes sans régulation de pH, est le procédé d'hydrolyse qui permet d'obtenir, après fractionnement, la quantité la plus importante de peptides de poids moléculaires ciblée, inférieur à 3 kDa.

A l'inverse, l'utilisation d'un tampon et de l'enzyme Protamex ne permet de générer seulement 25% de peptides inférieurs à 3kDa. Ce protocole ne semble donc pas adapté pour la libération de peptides de bas poids moléculaire.

**Exemple 10** : Identification du peptide AP au sein des hydrolysats de protéines de lactosérum de lait caprin

*1. Caractéristiques du peptide AP synthétique par analyse par HPLC-MS*

L'identification est réalisée par une HPLC-analytique Agilent (1100 LC), à l'aide de la colonne C18 Prontosil (250x4mm, 2.0µm) ou d'une colonne Waters Xbridge BEH130 C18 (5µm, 4,6x250mm) et en utilisant une double détection : UV à 215 nm et spectrométrie de masse (MS-ion trap, avec ionisation de type électrospray en mode scan positif.).

Les conditions de MS sont : scan de 60 à 600 m/z ; target mass (m/z) de 187, température de la source 300°C avec un débit de 10L/min d'azote. Le gradient utilise deux solvants, le solvant A consistant en de l'eau milliQ avec 0,1% de TFA (acide trifluoroacétique) et le solvant B consistant en l'acétonitrile avec 0,1% de TFA, et commence à 1% de B pour atteindre 30% après 55min, puis 50% à 60min et enfin 100% à 65min, avant de retourner à 1% à 75min.

Le peptide AP de chez Genosphère (ayant une pureté supérieure à 95%) est analysé afin d'obtenir le spectre de masse de référence ainsi que le temps de rétention en UV et en masse.

Lorsque la séparation est effectuée sur une colonne C18 prontosil, le peptide pur AP possède un temps de rétention de 3.4 min et deux pics m/z caractéristiques sur son spectre de masse, 187 et 116. Le fragment 187 correspond à MH+, le 209 à MNa+ et le 116 à la fragmentation du peptide en proline coté C-terminal.

Lorsque la séparation est effectuée sur une colonne C18 Waters BEH, le peptide pur AP possède un temps de rétention de 10,5 min et deux pics m/z caractéristiques sur son spectre de masse, 187 et 116. Le fragment 187 correspond à MH+, le 209 à MNa+, le 373 à 2MH+, les 395 à 2MNa+ et le 116 à la fragmentation du peptide en proline coté C-terminal.

*2. Identification du peptide AP dans un hydrolysat obtenu par Flavourzymes*

Les hydrolysats des exemples 6.1 et 7 réalisés par l'enzyme Flavourzymes avec et sans régulation du pH sont analysés en HPLC-MS sur la colonne C18 Waters BEH, où le peptide AP possède un temps de rétention proche de 10,5 min. Sur la **Figure 11** sont présentés le spectre UV et masse ainsi que l'*extract ion chromatogram* pour une masse/charge cible de 187 (MH+ de AP) des hydrolysats.

A partir de l'EIC187 un pic majoritaire possédant un temps de rétention correspondant au peptide AP standard (c'est-à-dire la molécule d'AP pure de synthèse chimique) de l'ordre de 11 minutes, est retrouvé lors de l'hydrolyse par flavourzymes, et ce avec et sans régulation du pH. L'analyse du spectre de masse de ce pic fait apparaitre les marqueurs spécifiques du peptide AP (m/z=187 ; 116 ; 373) assurant de sa présence au sein de ces hydrolysats. Le second pic présent dans l'EIC, mais possédant un temps de rétention de l'ordre de 7 minutes, correspondant au peptide PA standard (c'est-à-dire la molécule de PA pur de synthèse chimique). L'analyse du spectre de masse de ce pic fait apparaitre les marqueurs spécifiques du peptide PA (m/z=187 ; 90 ; 373).

Deux peptides ayant une masse de 187, le peptide AP recherché mais également le peptide PA.

Ces analyses confirment que le protocole d'hydrolyse utilisant Flavourzymes avec ou sans régulation du pH, permet de libérer le peptide AP à partir de concentré de protéines de lactosérum caprin.

*3. Identification du peptide AP dans un hydrolysat obtenu par le couple protamex/pepsine*

L'hydrolysat de l'exemple 6.2 est ensuite analysé en HPLC-MS sur la colonne Prontosil, où le peptide AP possède un temps de retention de l'ordre de 3,5min. L'*extract ion chromatogram* pour une masse/charge cible de 187 (MH+ de AP) après la première étape d'hydrolyse par protamex puis après la deuxième phase hydrolytique par la pepsine est déterminé.

Après hydrolyse par protamex, le pic majoritaire comprenant une m/z de 187 est retrouvé pour un temps de rétention de l'ordre de 16 minutes. Ce temps de rétention correspond à un peptide de plus grande taille. Après l'action de la pepsine, le peptide AP de masse 187 apparaît dans l'hydrolysat à 3.5 min.

Ces analyses confirment que le protocole d'hydrolyse utilisant deux préparations enzymatique, Protamex puis pepsine, permet de libérer le peptide AP à partir de concentré de protéines de lactosérum caprin.

**Exemple 11** : Choix de protéines pour la réalisation d'un hydrolysat contenant le peptide AP

Toutes protéines possédant la séquence AP (comportant ou étant constituée d'au moins 0,05% à <5% ou d'au moins 5% de motifs AP) peuvent être utilisées pour l'invention, et notamment les protéines avec les séquences suivantes :

**1) Protéines de Lait (vache, jument, brebis)**

a/ Beta-lactoglobuline (Lait de vache)

Données Uniprot : P02754
1 AP, 178 AA, 19883 Da

MKCLLLALAL TCGAQALIVT QTMKGLDIQK VAGTWYSLAM AASDISLLDA

QS**AP**LRVYVE ELKPTPEGDL EILLQKWENG ECAQKKIIAE KTKIPAVFKI

DALNENKVLV LDTDYKKYLL FCMENSAEPE QSLACQCLVR TPEVDDEALE

KFDKALKALP MHIRLSFNPT QLEEQCHI (SEQ ID NO : 8)

b/ Alpha-S1-caseine (Lait de vache)

Données Uniprot : P02754
2 AP dont 1 VAP, 214 AA, 24529 Da

MKLLILTCLV AVALARPKHP IKHQGLPQEV LNENLLRFF**V AP**FPEVFGKE

KVNELSKDIG SESTEDQAME DIKQMEAESI SSSEEIVPNS VEQKHIQKED

VPSERYLGYL EQLLRLKKYK VPQLEIVPNS AEERLHSMKE GIHAQQKEPM

IGVNQELAYF YPELFRQFYQ LDAYPSGAWY YVPLGTQYTD **AP**SFSDIPNP

IGSENSEKTT MPLW (SEQ ID NO : 9)

c/ Beta-caseine (Lait de vache)

Données Uniprot : P02666
1 AP, 214 AA, 24529 Da

MKVLILACLV ALALARELEE LNVPGEIVES LSSSEESITR INKKIEKFQS

EEQQQTEDEL QDKIHPFAQT QSLVYPFPGP IPNSLPQNIP PLTQTPVVVP

PFLQPEVMGV SKVKEAM**AP**K HKEMPFPKYP VEPFTESQSL TLTDVENLHL

PLPLLQSWMH QPHQPLPPTV MFPPQSVLSL SQSKVLPVPQ KAVPYPQRDM

PIQAFLLYQE PVLGPVRGPF PIIV (SEQ ID NO : 10)

d/ Lactoferrine (Lait de vache)

Données Uniprot : P24627
5 AP dont 1VAP, 708 AA, 78056 Da

MKLFVPALLS LGALGLCLA**A P**RKNVRWCTI SQPEWFKCRR WQWRMKKLGA **P**SITCVRRAF ALECIRAIAE KKADAVTLDG GMVFEAGRDP YKLRPVAAEI YGTKESPQTH YYAVAVVKKG SNFQLDQLQG RKSCHTGLGR SAGWIIPMGI LRPYLSWTES LEPLQGAVAK FFSASCVPCI DRQAYPNLCQ LCKGEGENQC ACSSREPYFG YSGAFKCLQD GAGDVAFVKE TTVFENLPEK ADRDQYELLC LNNSR**AP**VDA FKECHLAQVP SHAVVARSVD GKEDLIWKLL SKAQEKFGKN

KSRSFQLFGS PPGQRDLLFK DSALGFLRIP SKVDSALYLG SRYLTTLKNL RETAEEVKAR YTRVVWCAVG PEEQKKCQQW SQQSGQNVTC ATASTTDDCI VLVLKGEADA LNLDGGYIYT AGKCGLVPVL AENRKSSKHS SLDCVLRPTE GYLAVAVVKK ANEGLTWNSL KDKKSCHTAV DRTAGWNIPM GLIVNQTGSC AFDEFFSQSC **AP**GADPKSRL CALCAGDDQG LDKCVPNSKE KYYGYTGAFR CLAEDVGDVA FVKNDTVWEN TNGESTADWA KNLNREDFRL LCLDGTRKPV TEAQSCHLA**V AP**NHAVVSRS DRAAHVKQVL LHQQALFGKN GKNCPDKFCL FKSETKNLLF NDNTECLAKL GGRPTYEEYL GTEYVTAIAN LKKCSTSPLL EACAFLTR (SEQ ID NO : 11)

**2) Les protéines fibreuses (élastine, collagène, actine)**

a/ Actine (Saumon atlantique)

Données Uniprot : B5XFZ3
4 AP dont 1VAP, 376 AA, 41584 Da

MVEDEVAALV IDNGSGMCKS GFAGDD**AP**RA VFPSIVGRPR HVGIMVGMGQ KDSYVGDEAQ SKRGILSLKY PIDHGIVTNW DDMEKIWHHT FYNELRV**AP**E EHPVLLTE**AP** LNPKNNREKM TQIMFETFNS PAMYVAIQAV LSLYASGRTT GIVLDSGDGV THTVPIYEGY ALPHAVLRLD LAGRDLTDYL MKVLTERGYS FTTTAEREIV RDVKEKLCYV ALDYTNELAV AGSSSSLEKS YELPDGQVIT IGSERFRCPE ALFQPALIGM EAVGIHETAY NSIMKCDVDI RKDLYANTVL SGGSTMFSGI ADRMQKEVSA L**AP**TTMKIKI ISPPERKYSV WIGGSILASL STFQQMWISK MEYDESGPAI VHRKCF (SEQ ID NO : 12)

b/ Collagène Alpha2 (I) (Oncorhynchus keta, Saumon kéta)

Données Uniprot : Q8UUJ4

8 AP, 1352 AA, 126443 Da

MLSFVDNRIL LLLAVTSLLA SCQSGPRGAK GPRGDRGPQG PNGRDGKAGL
PGVAGPPGPP GLGGNFAAQF DGGKGSDPGP GPMGLMGSRG PNGPPGSPGP
QGFTGHAGEP GEPGQTGSIG ARGPTGSAGK PGEDGNNGRP GKPGDRGGPG
TQGARGFPGT PGLPGMKGHR GYNGLDGRKG ESGTAGAKGE TGAHGANGTP
GPAGSRGLNG ERGRAGPAGP AGARGADGST GPAGPAGPLG AAGPPGFPG**A**
**P**GPKGEIGGA GSNGPSGPQG GRGEPGINGA VGPVGPVGNP GNNGINGAKG

AAGLPGVAGA **P**GFPGPRGGP GPQGPQGSTG ARGLGGDPGP SGQKGDSGAK
GEPGHSGVQG AAGPAGEEGK RGSTGEAGAT GPAGLRGARG GAGTRGLPGL
EGRGGPIGMP GARGATGPAG IRG**AP**GDAGR AGESGLTGAR GLPGNSGQGG
PPGKEGPSGA AGLDGRTGPP GPTGPRGQPG NIGFPGPKGP GGEAGKGGDK
GPTGATGLRG GPGADGNNG**A** **P**GPAGVVGNA GEKGEQGPSG **AP**GFQGLPGP
AGPAGEAGKA GNQGMPGDQG LPGPAGVKGE RGNSGPAGSA GSQGAIGARG
PAGTPGPDGG KGEPGSVGIV GAAGHQGPGG MPGERGAGGT PGPKGEKGEG
GHRGLEGNMG RDGARGAAGP SGPPGPSGAN GEKGESGSFG PAGPAGLRGP
SGERGEGGPA GPPGFAGPPG SDGQSGPRGE KGPAGGKGDV GPAGPAGPSG
QSGPSGASGP AGPPGGRGDA GPSGLTGFPG AAGRVGGPGP AGISGPPGSA
GPAGKDGPRG LRGDAGPGGP QGEQGVVGPA GIAGDKGPSG EGGPPG**AP**GT
AGPQGVLGPS GFVGLPGSRG DKGLPGGPGA VGEPGRLGPA GASGPRGPSG
NIGMPGMTGT QGEAGREGNS GNDGPPGRPG AAGFKGDRGE PGSPGALGSS
GQPGPNGPAG SAGRPGNRGE SGPTGNGGPV GAAGARGA**P**G PAGPRGEKGG
AGEKGDRGMK GLRGHGGLQG MPGPNGPSGE TGSAGITGPA GPRGPAGPHG
PPGKDGRAGG HGAIGPVGHR GPPGHLGPAG PPGSPGLPGP AGPAGGGYDQ
SGGYDEYRAD QPSLRAKDYE VDATIKSLNS QIENLLTPEG SKKNPARTCR
DIRLSHPEWS SGFYWIGPNQ GCIADAIKAY CDFSTGHTCI HPHPESIARK
NWYRSSENKK HVWFGETING GTEFAYNDET LSPQSMATQL AFMRLLANQA
TQNITYHCKN SVAYMDGENG NLKKAVLLQG SNDVELRAEG NSRFTFNVLE
DGCTRHTGQW SKTVIEYRTN KPSRLPILDI **AP**LDIGEADQ EFGLDIGPVC
FK (SEQ ID NO : 13)

c/ Collagène Alpha1 (II) chain (bovin)

Données Uniprot : P02459
21 AP, 1487 AA, 141828 Da

QMAGGFDEK AGGAQMGVMQ GPMGPMGPRG PPGPAGA**P**GP QGFQGNPGEP
GEPGVSGPMGPRGPPGPPGK PGDDGEAGKP GKSGERGPPG PQGARGFPGT
PGLPGVKGHR GYPGLDGAKGEAGA**P**GVKGE SGSPGENGSP GPMGPRGLPG
ERGRTGPAGA AGARGNDGQP GPAGPPGPVGPAGGPGFPGA **P**GAKGEAGPT
GARGPEGAQG PRGEPGTPGS PGPAGAAGNP GTDGIPGAKGSAGA**P**GIAGA
**P**GFPGPRGPP GPQGATGPLG PKGQTGEPGI AGFKGEQGPK GEPGPAGPQG

**AP**GPAGEEGK RGARGEPGGA GPAGPPGERG **AP**GNRGFPGQ DGLAGPKGA**P**
GERGPSGLAGPKGANGDPGR PGEPGLPGAR GLTGRPGDAG PQGKVGPSGA
**P**GEDGRPGPP GPQGARGQPGVMGFPGPKGA NGEPGKAGEK GLPGA**P**GLRG
LPGKDGETGA AGPPGPAGPA GERGEQGA**P**GPSGFGGLPGP PGPPGEGGKP
GDGGVPGEAG **AP**GLVGPRGE RGFPGERGSPGSQGLQGARGLPGTPGTDGP
KGAAGPAGPP GAQGPPGLQG MPGERGAAGI AGPKGDRGDV GEKGPEGA**P**G
KDGGRGLTGP IGPPGPAGAN GEKGEVGPPG PAGTAGARGA **P**GERGETGPP
GPAGFAGPPGADGQPGAKGE QGEAGQKGDA GA**P**GPQGPSG **AP**GPQGPTGV
TGPKGARGAQGPPGATGFPGAAGRVGPPGS NGNPGPPGPP GPSGKDGPKG
ARGDSGPPGR AGDPGLQGPA GPPGEKGEPGDDGPSGPDGP PGPQGLAGQR
GIVGLPGQRG ERGFPGLPGP SGEPGKQGA**P** GASGDRGPPGPVGPPGLTGP
AGEPGREGSP GADGPPGRDG AAGVKGDRGE TGAVGA**P**GA**P** GPPGSPGPAG
PIGKQGDRGE AGAQGPMGPA GPAGARGMPG PQGPRGDKGE TGEAGERGLK
GHRGFTGLQGLPGPPGPSGD QGASGPAGPS GPRGPPGPVG PSGKDGANGI
PGPIGPPGPR GRSGETGPAGPPGNPGPPGP PGPPGPGIDM SAFAGLGQRE
KGPDPLQYMR ADEAAGNLRQ HDAEVDATLKSLNNQIESLR SPEGSRKNPA
RTCRDLKLCH PEWKSGDYWI DPNQGCTLDA MKVFCNMETGETCVYPNPAS
VPKKNWWSSK SKDKKHIWFG ETINGGFHFS YGDDNLA**P**NT ANVQMTFLRL
LSTEGSQNIT YHCKNSIAYL DEAAGNLKKA LLIQGSNDVE IRAEGNSRFT
YTVLKDGCTKHTGKWGKTMI EYRSQKTSRL PIIDIA**P**MDI GGPEQEFGVD IGPVCFL
(SEQ ID NO : 14)

d/ Elastine (bovine)

Données Uniprot : F1NOH9

9 AP dont 2 VAP, 805 AA, 72317 Da

MAGLTAAARR PGVLLLLLCI LQPSQPGGVP GAVPGGVPGG VFFPGAGLGG LGVGALGPGV KPAKPGVGGL AGPGLGAGLG ALPGAFPGAL VPGGPAGAAA AYKAAAKAGA AGLGVGGIGG VGGLGVSTGA VVPQLGAGVG AGVKPGKVPG VGLPGVYPGG VLPGAGARFP GIGVLPGVPT GAGVKPK**AP**G GGGAFAGIPG VGPFGGQQPG VPLGYPIK**AP** KLPGGYGLPY STGKLPYGFG PGGVAGAAGK AGYPTGTGVG PQAAAAAAKA AAKLGAGGAG VLPGVGVGGA GIPG**AP**GAIP

GIGGIAGVG**A P**DAAAAAAAA AKAAKFGAAG GFPGVGVPGV GVPGVGVPGV GVPGVGVPGV GVPGVGVPGV GVPGVGVPGV GVPGVGVPGA VSPAAAAKAA AKAAKFGARG GVGVGGIPTF GVGPGGFPGI GDAAAAQAAA AAKAAKIGAG GVGALGGLVP G**AP**GAIPGVP GVGGVPGVGI PAAAAAKAAA KAAQFGLGPG VG**VAP**GVGVV PGVGVVPGVG **VAP**GIGLGPG GVIGAGVPAA AKSAAKAAAK AQFRAAAGLP AGVPGLGVGV GVPGLGVGVG VPGLGVGAGV PGLGAVPGTL AAAKAAKFGP GGVGALGGVG DLGGAGIPGG VAGVGPAAAA AAAKAAVQLV PKHRNPHAGL GHTISWPPWP PFPRPIAVPY VRRLPPPPYW EQPSCSCGIH PPICPSVRPS LSWFGRP**AP**L AGW**AP**PPSTW LTCHGSLGPA STPSHTPLRR GPEPLGVKSC TSWGRRNLRP NLDLPPRSTV SPSPPRATVL QSISPPPRPS LCVSL (SEQ ID NO : 15)

**3) Les protéines d'oeuf**

a/ Ovotransferrine (oeuf de poule)

Données Uniprot : P02789
5 AP, 705 AA, 75828 Da

MKLILCTVLS LGIAAVCFA**A P**PKSVIRWCT ISSPEEKKCN NLRDLTQQER

ISLTCVQKAT YLDCIKAIAN NEADAISLDG GQAFEAGL**AP** YKLKPIAAEV

YEHTEGSTTS YYAVAVVKKG TEFTVNDLQG KTSCHTGLGR SAGWNIPIGT

LLHRGAIEWE GIESGSVEQA VAKFFSASCV PGATIEQKLC RQCKGDPKTK

CARN**AP**YSGY SGAFHCLKDG KGDVAFVKHT TVNEN**AP**DQK DEYELLCLDG

SRQPVDNYKT CNWARVAAHA VVARDDNKVE DIWSFLSKAQ SDFGVDTKSD

FHLFGPPGKK DPVLKDLLFK DSAIMLKRVP SLMDSQLYLG FEYYSAIQSM

RKDQLTPSPR ENRIQWCAVG KDEKSKCDRW SVVSNGDVEC TVVDETKDCI

IKIMKGEADA VALDGGLVYT AGVCGLVPVM AERYDDESQC SKTDERPASY

FAVAVARKDS NVNWNNLKGK KSCHTAVGRT AGWVIPMGLI HNRTGTCNFD

EYFSEGC**AP**G SPPNSRLCQL CQGSGGIPPE KCVASSHEKY FGYTGALRCL

VEKGDVAFIQ HSTVEENTGG KNKADWAKNL QMDDFELLCT DGRRANVMDY

RECNLAEVPT HAVVVRPEKA NKIRDLLERQ EKRFGVNGSE KSKFMMFESQ

NKDLLFKDLT KCLFKVREGT TYKEFLGDKF YTVISSLKTC NPSDILQMCS

FLEGK (SEQ ID NO : 16)

**4) Les protéines végétales**

a/ Legumine A (Pisum sativum, protéines de pois)

Données Uniprot : P02857
1 AP, 517 AA, 58805 Da

MAKLLALSLS FCFLLLGGCF ALREQPQQNE CQLERLDALE PDNRIESEGG

LIETWNPNNK QFRCAGVALS RATLQRNALR RPYYSN**AP**QE IFIQQGNGYF

GMVFPGCPET FEEPQESEQG EGRRYRDRHQ KVNRFREGDI IAVPTGIVFW

MYNDQDTPVI AVSLTDIRSS NNQLDQMPRR FYLAGNHEQE FLQYQHQQGG

KQEQENEGNN IFSGFKRDYL EDAFNVRHI VDRLQGRNED EEKGAIVKVK

GGLSIISPPE KQARHQRGSR QEEDEDEEKQ PRHQRGSRQE EEEDEDEERQ

PRHQRRRGEE EEEDKKERGG SQKGKSRRQG DNGLEETVCT AKLRLNIGPS

SSPDIYNPEA GRIKTVTSLD LPVLRWLKLS AEHGSLHKNA MFVPHYNLNA

NSIIYALKGR ARLQVVNCNG NTVFDGELEA GRALTVPQNY AVAAKSLSDR

FSYVAFKTND RAGIARLAGT SSVINNLPLD VVAATFNLQR NEARQLKSNN

PFKFLVPARE SENRASA (SEQ ID NO : 17)

**Exemple 12** : Réalisation d'un hydrolysat de protéines de poids, de gélatine de poisson et de gélatine de boeuf par Flavourzymes

Sources de Protéines

Protéines de poids (disponible chez Nutralis de Roquette), Gélatine de poissons (disponible chez Sigma), Gélatine de boeuf (disponible chez Sigma).

Enzymes utilisées

Flavourzymes (disponible chez Sigma sous la référence P6110) (750 μL/30 mL/1500 mg de CPL, 5 % v/w)

Une étape de dénaturation par chauffage d'une solution de proteines (5% de matière sèche dans de l'eau ultrapure) à 80°C pendant 10 minutes est appliquée avant hydrolyse.

L'hydrolyse enzymatique par Flavourzymes, est ensuite réalisée sur des supports Radley Tech Carroussel 6 contrôlant la température interne des milieux à 50°C et permettant une agitation de 600 rpm.

Pour arrêter la réaction après 6h, la solution est portée à 90°C pendant 15 minutes afin de dénaturer les enzymes encore présentes dans le milieu. L'hydrolysat est ensuite aliquoté puis conservé à -20°C avant fractionnement et analyses.

Les hydrolysats réalisés par l'enzyme Flavourzymes sont analysés en HPLC-MS sur la colonne C18 Waters BEH.

La **Figure 11** présente le chromatogramme HPLC-MS obtenu avec une détection en UV à 215 nm (A), avec une détection en masse des ions totaux (B), avec une détection en masse sélective de l'ion m/z 187 caractéristique de AP et PA (C).

**Exemple 13 :** Action hypothétique d'une protéase type Thermolysine sur des protéines contenant des motifs AP et/ou VAP

1/ Action de la Thermolysine sur la Beta-lactoglobuline (Lait de vache) P02754

Les résultats de la recherche des peptides entre 150 et 250 Da sont présentés dans le Tableau 3 ci-dessous.

| m/z (mi) | Sequence | m/z (mi) | Sequence |
|---|---|---|---|
| 187.1077 | AP | 231.1703 | VL |
| 189.1234 | AV | 231.1703 | LV |
| 203.1390 | AL | 237.0904 | AM |
| 203.1390 | LA | 237.0904 | MA |
| 218.1135 | AQ | 245.1860 | LL |
| 219.1339 | IS | 245.1860 | LI |
| 219.1339 | LS | 245.1860 | IL |
| 221.0954 | AM | 245.1860 | II |
| 221.0954 | MA | 247.1288 | LD |
| 229.1547 | IP | 247.1288 | ID |
| 229.1547 | LP | 248.1241 | AGT |

2/ Action de la Thermolysine sur l'Alpha-Sl-caseine (Lait de vache) P02754

Les résultats de la recherche des peptides entre 150 et 250 Da sont présentés dans le Tableau 4 ci-dessous.

| m/z (mi) | Sequence | m/z (mi) | Sequence |
|---|---|---|---|
| 187.1077 | AP | 203.1390 | LA |
| 189.1234 | VA | 215.1390 | VP |

(suite)

| m/z (mi) | Sequence | m/z (mi) | Sequence |
|----------|----------|----------|----------|
| 189.1234 | AV | 231.1703 | LV |
| 189.1234 | LG | 245.1860 | LL |
| 189.1234 | IG | 245.1860 | LI |
| 189.1234 | MK | 247.1111 | MP |
| 203.1390 | AL | 247.1288 | LD |

3/ Action de la Thermolysine sur la Beta-caseine (Lait de vache) P02666

Les résultats de la recherche des peptides entre 150 et 250 Da sont présentés dans le Tableau 5 ci-dessous.

| m/z (mi) | Sequence | m/z (mi) | Sequence | m/z (mi) | Sequence |
|----------|----------|----------|----------|----------|----------|
| 189.1234 | VA | 219.1339 | LS | 237.0904 | AM |
| 189.1234 | MK | 221.0954 | AM | 237.1234 | AF |
| 193.0641 | AC | 223.0747 | MG | 245.1860 | LI |
| 203.1390 | AL | 229.1547 | LP | 245.1860 | IL |
| 203.1390 | LA | 231.1703 | VL | 245.1860 | LL |
| 207.0798 | MG | 231.1703 | LV | 245.1860 | II |
| 215.1390 | VP | 231.1703 | IV | 246.1448 | LN |
| 217.1547 | VV | 233.1496 | LT | 246.1812 | MKV |
| 247.1111 | MP | 249.1267 | VM | | |

4/ Action de la Thermolysine sur la Lactoferrine (Lait de vache) P24627

Les résultats de la recherche des peptides entre 150 et 250 Da sont présentés dans le Tableau 6 ci-dessous.

| m/z (mi) | Sequence | m/z (mi) | Sequence | m/z (mi) | Sequence |
|----------|----------|----------|----------|----------|----------|
| 187.1077 | AP | 229.1547 | IP | 245.1860 | LL |
| 189.1234 | VA | 231.1703 | VL | 246.1448 | LN |
| 189.1234 | AV | 231.1703 | IV | 246.1561 | AR |
| 189.1234 | LG | 231.1703 | LV | 246.1812 | VK |
| 189.1234 | MK | 233.1132 | VD | 249.1267 | MV |
| 191.1026 | AT | 233.1496 | LT | 223.0747 | MG |
| 193.0641 | AC | 235.1111 | LC | 223.1077 | FG |
| 203.1390 | AL | 237.1234 | AF | 227.1139 | AH |
| 203.1390 | AI | 237.1234 | FA | 207.0798 | MG |
| 203.1390 | LA | 239.1026 | YG | 215.1390 | VP |
| 204.0979 | AN | 244.1292 | APG | 217.1547 | VV |
| 205.0819 | AD | 245.1860 | LI | 227.1139 | AH |
| 218.1135 | AQ | 246.1812 | VK | 219.1339 | LS |
| 218.1499 | AK | 249.1267 | MV | 223.1077 | FG |

(suite)

| m/z (mi) | Sequence | m/z (mi) | Sequence | m/z (mi) | Sequence |
|----------|----------|----------|----------|----------|----------|
| 219.0975 | AE | 223.0747 | MG | 219.1339 | VT |

5/ Action de la Thermolysine sur l'Actine (Saumon atlantique) B5XFZ3

Les résultats de la recherche des peptides entre 150 et 250 Da sont présentés dans le Tableau 7 ci-dessous.

| m/z (mi) | Sequence | m/z (mi) | Sequence |
|----------|----------|----------|----------|
| 161.0921 | AA | 215.1390 | VP |
| 175.1077 | VG | 219.1339 | LS |
| 177.0870 | AS | 221.0954 | AM |
| 187.1077 | AP | 231.1703 | VL |
| 189.1234 | VA | 231.1703 | LV |
| 189.1234 | AV | 231.1703 | IV |
| 189.1234 | IG | 233.1132 | VD |
| 203.1390 | AL | 233.1496 | IT |
| 203.1390 | AI | 235.1111 | LC |
| 203.1390 | LA | 245.1860 | IL |
| 205.1183 | VS | 246.1448 | AVG |
| 215.1390 | VP | 247.1288 | LD |

6/ Action de la Thermolysine sur le Collagene Alpha2 (I) (Oncorhynchus keta, Saumon kéta) Q8UUJ4

Les résultats de la recherche des peptides entre 150 et 250 Da sont présentés dans le Tableau 8 ci-dessous.

| m/z (mi) | Sequence | m/z (mi) | Sequence |
|----------|----------|----------|----------|
| 175.1077 | VG | 223.1077 | FG |
| 187.1077 | AP | 229.1547 | LP |
| 189.1234 | VA | 231.1703 | VL |
| 189.1234 | AV | 233.1132 | VD |
| 189.1234 | IG | 233.1496 | IT |
| 189.1234 | LG | 234.1084 | AGS |
| 191.1026 | AT | 237.1234 | FA |
| 203.1390 | LA | 237.1234 | AF |
| 205.0819 | AD | 244.1292 | AGP |
| 207.0798 | MG | 244.1292 | APG |
| 218.1135 | AQ | 245.1860 | IL |
| 218.1135 | AAG | 245.1860 | LL |
| 219.1339 | MLS | 246.1448 | VAG |
| 219.1339 | LS | 246.1448 | AGV |
| 221.0954 | VC | 246.1448 | VGA |

(suite)

| m/z (mi) | Sequence | m/z (mi) | Sequence |
|----------|----------|----------|----------|
| 247.1288 | LD | 246.1448 | IGG |
| 248.1241 | ATG | 247.1288 | VE |

7/ Action de la Thermolysine sur le Collagen Alpha1 (II) chain (bovin) P02459

Les résultats de la recherche des peptides entre 150 et 250 Da sont présentés dans le Tableau 9 ci-dessous.

| m/z (mi) | Sequence | m/z (mi) | Sequence |
|----------|----------|----------|----------|
| 175.1077 | VG | 229.1547 | LP |
| 177.0870 | AS | 233.1132 | VD |
| 187.1077 | AP | 233.1496 | IT |
| 191.1026 | AT | 237.0904 | MS |
| 203.1390 | AL | 237.1234 | AF |
| 203.1390 | IA | 244.1292 | APG |
| 204.0979 | AGG | 244.1292 | AGP |
| 204.0979 | AN | 245.1860 | LL |
| 207.0798 | MG | 246.1448 | AVG |
| 218.1135 | AQ | 246.1448 | VQ |
| 218.1135 | AGA | 247.1288 | VE |
| 218.1135 | AAG | 247.1288 | ID |
| 218.1135 | AGA | 247.1288 | LD |
| 219.0975 | AE | 247.1288 | ID |
| 221.0954 | VC | 248.1241 | AGT |
| 223.0747 | MG | 248.1241 | ATG |
| 223.1077 | FG | | |

8/ Action de la Thermolysine sur l'Elastine (bovine) F1NOH9

Les résultats de la recherche des peptides entre 150 et 250 Da sont présentés dans le Tableau 10 ci-dessous.

| m/z (mi) | Sequence | m/z (mi) | Sequence |
|----------|----------|----------|----------|
| 161.0921 | AA | 223.1077 | FG |
| 175.1077 | VG | 229.1547 | LP |
| 187.1077 | AP | 229.1547 | IP |
| 189.0870 | MAG | 231.1703 | VL |
| 189.1234 | AV | 232.1292 | VGG |
| 189.1234 | LG | 233.1496 | LT |
| 189.1234 | IG | 235.1111 | LC |
| 191.1026 | AT | 237.1234 | AF |
| 203.1390 | AL | 239.1026 | YG |

(suite)

| m/z (mi) | Sequence | m/z (mi) | Sequence |
|----------|----------|----------|----------|
| 203.1390 | LA | 244.1292 | APG |
| 203.1390 | IA | 245.1860 | LL |
| 204.0979 | AGG | 246.1448 | VGA |
| 205.1183 | VS | 246.1448 | VAG |
| 215.1390 | VP | 246.1448 | AGV |
| 218.1135 | AGA | 246.1448 | VQ |
| 218.1135 | AAG | 246.1448 | LGG |
| 218.1135 | AQ | 246.1448 | IGG |
| 218.1499 | AK | 246.1561 | AR |
| 219.1339 | IS | 247.1288 | LD |
| 219.1339 | LS | | |

9/ Action de la Thermolysine sur l'Ovotransferrine (oeuf de poule) P02789

Les résultats de la recherche des peptides entre 150 et 250 Da sont présentés dans le Tableau 11 ci-dessous.

| m/z (mi) | Sequence | m/z (mi) | Sequence |
|----------|----------|----------|----------|
| 161.0921 | AA | 219.0975 | AE |
| 177.0870 | AS | 219.1339 | LS |
| 187.1077 | AP | 219.1339 | IS |
| 189.1234 | AV | 221.0954 | VC |
| 189.1234 | VA | 223.0747 | MG |
| 189.1234 | LG | 223.1077 | FG |
| 189.1234 | MK | 227.1139 | AH |
| 191.1026 | AT | 229.1547 | IP |
| 203.1390 | AI | 231.1703 | LV |
| 203.1390 | IA | 232.1292 | VN |
| 204.0979 | AN | 235.1111 | LC |
| 205.0819 | AD | 237.1234 | FA |
| 207.0798 | MG | 237.1234 | AF |
| 215.1390 | VP | 237.1234 | FA |
| 217.1547 | VV | 245.1860 | LI |
| 218.1499 | AK | 245.1860 | LL |
| 249.1267 | VM | 246.1561 | AR |
| 219.1339 | LS | 249.1267 | VM |

10/ Action de la Thermolysine sur la Legumine A (Pisum sativum, protéines de pois) P02857

Les résultats de la recherche des peptides entre 150 et 250 Da sont présentés dans le Tableau 12 ci-dessous.

| m/z (mi) | Sequence | m/z (mi) | Sequence |
|----------|----------|----------|----------|
| 177.0870 | AS | 229.1547 | LP |
| 189.1234 | VA | 231.1703 | VI |
| 189.1234 | AV | 231.1703 | IV |
| 191.1026 | AT | 233.1496 | LT |
| 203.1390 | AI | 237.0904 | AM |
| 203.1390 | LA | 237.1234 | FA |
| 203.1390 | IA | 245.1860 | LL |
| 205.1183 | VS | 245.1860 | II |
| 215.1390 | VP | 246.1448 | AGV |
| 217.1547 | VV | 246.1448 | LN |
| 218.1499 | MAK | 246.1561 | AR |
| 218.1499 | AK | 246.1812 | VK |
| 219.1339 | LS | 247.1288 | LD |
| 221.0954 | AM | 248.1241 | ASA |
| 223.1077 | FG | 249.1267 | MV |

**Exemple 14 :** Détermination *in vitro* de l'inhibition de la DPP-IV (DiPeptidyl Peptidase - IV) par les peptides synthétiques

L'activité inhibitrice de peptides synthétiques peut être mesurée *in vitro* selon le protocole suivant :

25 μl du substrat Gly-L-Pro-p-nitroanilide dans du tampon 0,1 M Tris-HCL (pH 8,0) à 1,6 mM, sont mélangés avec 25 μl de l'échantillon peptide ou d'inhibiteur de référence à différentes concentrations. Les échantillons sont préalablement solubilisés dans du tampon Tris HCL (pH8.0).

Après une pré-incubation à 37°C pendant 10 min, 50 μl de l'enzyme DPP-IV à une concentration de 0.01U/ml dans du tampon 0,1 M Tris-HCL (pH 8,0) sont ajoutés au mélange afin d'initier la réaction. Le milieu réactionnel est incubé pendant 60 min à 37°C avant que la réaction ne soit stoppée par l'ajout de 100 μl de tampon acétate de sodium (pH4) à 1M.

La quantité de produit formé (p-nitroanilide) est mesurée par lecture de l'absorbance à 385 nm en utilisant le spectrophotomètre Fluostar Omega (BMG Labtech, Allemagne).

Tous les tests d'inhibition sont réalisés en triplicata.

Le substrat utilisé est le Gly-L-Pro-p-nitroanilide hydrochloride (disponible chez Sigma sous la référence G0513).

L'enzyme utilisée est la DPP-IV du rein de porc (disponible chez Sigma sous la référence D7052)

L'inhibiteur de référence est le Diprotin A, correspondant au tripeptide Ile-Pro-Ile (disponible chez Sigma sous la référence I9759).

Les résultats d'$IC_{50}$ des peptides sur l'activité inhibitrice de la DPP-IV testée *in vitro* sont présentés dans le Tableau 13 ci-dessous.

| Peptides | (IC 50) en mmol/l |
|----------|-------------------|
| **Diprotin A (IPI)** | **0,0121±0,0002** |
| SAPLRVY (SEQ ID NO : 3) | 1.056±0.031 |
| VAPFPEV (SEQ ID NO : 4) | 1,184±0.008 |
| GPL | 1.853±0.298 |
| VAPG (SEQ ID NO : 6) | 2,271±0.131 |
| **AP** | **4,775±0,284** |
| **VAP** | **4,817±2,303** |

# EP 3 107 556 B1

(suite)

| Peptides | (IC 50) en mmol/l |
|---|---|
| AVPYQR (SEQ ID NO : 5) | 5,701±0,080 |
| VGVAPG (SEQ ID NO : 7) | 10,732±0,769 |
| KY | Pas inhibition |
| VW | Pas inhibition |
| VY | Pas inhibition |
| AVIPIPT | Pas inhibition |

Les résultats confirment que les peptides utilisés inhibent faiblement la DPP-IV par rapport à la référence qui est la Diprotin A, un inhibiteur connu de la DPP-IV. Notamment, les peptides AP et VAP inhibent très faiblement la DPP-IV par rapport à la référence la Diprotin A.

## Revendications

**1.** Composition comprenant au moins un peptide AP pour son utilisation dans la prévention et/ou le traitement du diabète de type 2.

**2.** Composition pour son utilisation selon la revendication 1, ladite composition comprenant au moins un peptide AP, notamment en association avec un peptide de type APX' dans lequel X' correspond à un acide aminé ou à un groupe d'acides aminés choisi parmi : Alanine ; Arginine ; Asparagine ; Aspartate ou Acide aspartique ; Cystéine ; Glutamate ou Acide glutamique ; Glutamine ; Glycine ; Histidine ; Isoleucine ; Leucine ; Lysine ; Méthionine ; Phénylalanine ; Proline ; Sérine ; Thréonine ; Tryptophane ; Tyrosine et Valine.

**3.** Composition pour son utilisation selon la revendication 2, ladite composition étant sous forme unitaire et comprenant une quantité de peptide AP de 5 mg à 3000 mg.

**4.** Composition pour son utilisation selon l'une quelconque des revendications 1 à 3, ladite composition comprenant une quantité de peptides VAP et AP de 5 mg à 3000 mg, et en particulier une quantité de 5 mg à 7,4 mg ou une quantité de 7,5 mg à 2250 mg ou une quantité supérieure à 2250 mg jusqu'à 3000 mg.

**5.** Composition pharmaceutique comprenant un peptide AP, ladite composition étant sous forme unitaire et comprenant une quantité de peptide AP de 5 mg à 7,4 mg, en association avec un véhicule pharmaceutiquement acceptable

**6.** Composition pharmaceutique selon la revendication 5, ladite composition comprenant une quantité de peptides VAP et AP de 5 mg à 7,4 mg.

**7.** Composition pharmaceutique selon l'une quelconque des revendications 5 ou 6, ladite composition comprenant un ou plusieurs inhibiteurs de l'amylase et/ou un ou plusieurs inhibiteurs de lipase.

**8.** Utilisation d'au moins un peptide AP pour la préparation d'une composition nutraceutique ou d'un complément alimentaire

**9.** Composition alimentaire ou nutraceutique pour inhiber l'alpha-glucosidase, ladite composition comprenant au moins un peptide AP, ladite composition étant sous forme unitaire et comprenant une quantité de peptide AP de 5 mg à 1000 mg

10. Composition alimentaire ou nutraceutique selon la revendication 9, ladite composition comprenant une quantité de peptides VAP et AP de 5 mg à 1000 mg.

**Patentansprüche**

1. Zusammensetzung, umfassend mindestens ein Peptid AP zur Verwendung bei der Vorbeugung und/oder Behandlung von Typ-2-Diabetes.

2. Zusammensetzung zur Verwendung nach Anspruch 1, wobei die Zusammensetzung mindestens ein Peptid AP umfasst, insbesondere in Verbindung mit einem Peptid von Typ APX', wobei X' einer Aminosäure oder eine Gruppe von Aminosäuren entspricht, ausgewählt aus: Alanin; Arginin; Asparagin; Aspartat oder Asparaginsäure; Cystein; Glutamat oder Glutaminsäure; Glutamin; Glycin; Histidin; Isoleucin; Leucin; Lysin; Methionin; Phenylalanin; Prolin; Serin; Threonin; Tryptophan; Tyrosin und Valin.

3. Zusammensetzung zur Verwendung nach Anspruch 2, wobei die Zusammensetzung in Einheitsform vorliegt und eine Menge an Peptid AP von 5 mg bis 3.000 mg umfasst.

4. Zusammensetzung nach einem der Ansprüche 1 bis 3, wobei die Zusammensetzung eine Menge der Peptide VAP und AP von 5 mg bis 3.000 mg und insbesondere eine Menge von 5 mg bis 7,4 mg oder eine Menge von 7,5 mg bis 2.250 mg oder eine Menge von mehr als 2.250 mg bis zu 3.000 mg umfasst.

5. Pharmazeutische Zusammensetzung, umfassend ein Peptid AP, wobei die Zusammensetzung in Einheitsform vorliegt und eine Menge an Peptid AP von 5 mg bis 7,4 mg in Verbindung mit einem pharmazeutisch verträglichen Vehikel umfasst.

6. Pharmazeutische Zusammensetzung nach Anspruch 5, wobei die Zusammensetzung eine Menge an Peptiden VAP und AP von 5 mg bis 7,4 mg umfasst.

7. Pharmazeutische Zusammensetzung nach einem der Ansprüche 5 oder 6, wobei die Zusammensetzung einen oder mehrere Amylaseinhibitoren und/oder einen oder mehrere Lipasseinhibitoren umfasst.

8. Verwendung mindestens eines Peptids AP zur Herstellung einer nutrazeutischen Zusammensetzung oder eines Nahrungsergänzungsmittels.

9. Nahrungsmittelzusammensetzung oder nutrazeutische Zusammensetzung zur Inhibition von alpha-Glucosidase, wobei die Zusammensetzung mindestens ein Peptid AP umfasst, wobei die Zusammensetzung in Einheitsform vorliegt und eine Menge an Peptid AP von 5 mg bis 1.000 mg umfasst.

10. Nahrungsmittelzusammensetzung oder nutrazeutische Zusammensetzung nach Anspruch 9, wobei die Zusammensetzung eine Menge an Peptiden VAP und AP von 5 mg bis 1.000 mg umfasst.

**Claims**

1. Composition comprising at least one AP peptide for use in the prevention and/or treatment of type 2 diabetes.

2. Composition for use according to claim 1, said composition comprising at least one AP peptide, in particular in combination with a peptide of type APX' in which X' corresponds to an amino acid or an amino acid group chosen from: Alanine; Arginine; Asparagine; Aspartate or Aspartic acid; Cysteine; Glutamate or Glutamic acid; Glutamine Glycine; Histidine; Isoleucine; Leucine; Lysine; Methionine; Phenylalanine; Proline; Serine; Threonine; Tryptophan; Tyrosine and Valine.

3. Composition for use according to claim 2, said composition being in unit form and comprising a quantity of AP peptide from 5 mg to 3000 mg.

4. Composition for use according to any one of claims 1 to 3, said composition comprising a quantity of VAP and AP peptides from 5 mg to 3000 mg, and in particular a quantity from 5 mg to 7.4 mg or a quantity from 7.5 mg to 2250 mg or a quantity greater than 2250 mg up to 3000 mg.

5. Pharmaceutical composition comprising an AP peptide, said composition being in unit form and comprising a quantity of AP peptide from 5 mg to 7.4 mg, in association with a pharmaceutically acceptable vehicle.

6. Pharmaceutical composition according to claim 5, said composition comprising a quantity of VAP and AP peptides from 5 mg to 7.4 mg.

7. Pharmaceutical composition according to any one of claims 5 or 6, said composition comprising one or more amylase inhibitors and / or one or more lipase inhibitors.

8. Use of at least one AP peptide for the preparation of a nutraceutical composition or a food supplement.

9. Food or nutraceutical composition for inhibiting alpha-glucosidase, said composition comprising at least one AP peptide, said composition being in unit form and comprising a quantity of AP peptide from 5 mg to 1000 mg.

10. Food or nutraceutical composition according to claim 9, said composition comprising a quantity of VAP and AP peptides from 5 mg to 1000 mg.

Figure 1 :

Figure 2 :

Figure 3 :

Figure 4 :

Figure 5 :

Figure 6 :

Figure 7 :

Figure 8 :

Figure 9 :

Figure 10 :

☐ Flavourzymes sans régulation
☐ Protamex & Pepsine avec régulation
■ Protamex sans régulation

☐ Flavourzymes avec régulation
☐ Protamex dans tampon KH2PO4

Figure 11 :

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006068480 A
- WO 2012085266 A2
- WO 2006084560 A
- WO 2005046672 A2
- WO 2006096769 A
- EP 0638317 B1
- FR 1451280

**Littérature non-brevet citée dans la description**

- **UDANI JK et al.** *Nutr J,* 2009, vol. 8, 52
- **MARUYAMA et al.** *Agric. Biol. Chem.,* 1987, vol. 51 (6), 1581-1586
- **CHEUNG et al.** *J. Biol. Chem.,* 1980, vol. 255, 401-407
- **KANG et al.** *Journal of Medicinal Plants Research,* 2012, vol. 6, 2850-2856